(19) Europäisches Patentamt
European Patent Office
Office européen des brevets

(11) EP 2 333 059 A1

(12) EUROPEAN PATENT APPLICATION
published in accordance with Art. 153(4) EPC

(43) Date of publication:
**15.06.2011 Bulletin 2011/24**

(21) Application number: **09803013.3**

(22) Date of filing: **30.07.2009**

(51) Int Cl.:
***C12N 15/09*** *(2006.01)* ***C12Q 1/68*** *(2006.01)*

(86) International application number:
**PCT/JP2009/063562**

(87) International publication number:
**WO 2010/013771 (04.02.2010 Gazette 2010/05)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **30.07.2008 JP 2008196513**
**15.06.2009 JP 2009142608**

(71) Applicant: **Nippon Steel Kankyo Engineering Co., Ltd.**
**Chiyoda-ku**
**Tokyo 101-0031 (JP)**

(72) Inventors:
- **SEKIGUCHI Yuji**
**Tsukuba-shi**
**Ibaraki 305-8566 (JP)**
- **NODA Naohiro**
**Tsukuba-shi**
**Ibaraki 305-8566 (JP)**
- **MIYATA Ryo**
**Tsukuba-shi**
**Ibaraki 305-8566 (JP)**
- **NAKAMURA Kazunori**
**Tokyo 101-0031 (JP)**
- **KURATA Shinya**
**Tokyo 101-0031 (JP)**
- **TSUNEDA Satoshi**
**Tokyo 169-8050 (JP)**
- **TANI Hidenori**
**Tokyo 169-8050 (JP)**

(74) Representative: **Hartz, Nikolai**
**Wächtershäuser & Hartz**
**Patentanwaltspartnerschaft**
**Weinstrasse 8**
**80333 München (DE)**

# (54) UNIVERSAL NUCLEIC ACID PROBE SET AND METHOD FOR UTILIZATION THEREOF

(57) A nucleic acid probe set includes (A) a fluorescent probe and (B) a binding probe. The fluorescent probe (A) is formed of an oligonucleotide, which includes (a) a nucleotide unit labeled with (d) a fluorescent substance. The binding probe (B) is formed of an oligonucleotide having (b1) a fluorescent probe binding region, which can hybridize to the fluorescent probe (A), and (b2) a target nucleic acid binding region, which can hybridize to a target nucleic acid sequence (C). The fluorescent substance (d) is a fluorescent substance which changes in fluorescent character upon interaction with guanine. At least one of nucleotide units which constitute the fluorescent probe (A) is an artificial nucleotide unit having a function to raise a dissociation temperature between the probe (A) and the fluorescent probe binding region (b1). The nucleic acid probe is provided with an improved fluorescence quenching efficiency.

Fig. 6

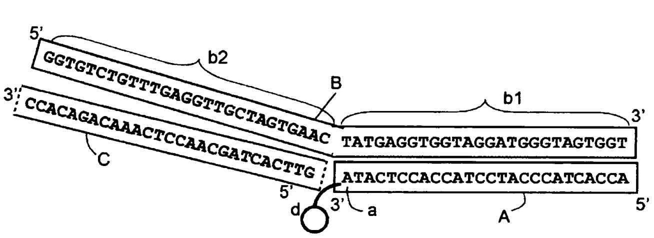


EP 2 333 059 A1

SEQUENCE LISTING

<110> National Institute of Advanced Industrial Science and Technology
J-Bio 21 Corporation
Nippon Steel Kankyo Engineering Co.,Ltd.
<120> Universal nucleic acid probe set and use thereof
<130> PCT-83-ENJBS
<150> JP2008-196513
<151> 2008-07-30
<150> JP2009-142608
<151> 2009-06-15
<160> 38

<210> 1
<211> 21
<212> DNA
<213> Artificial Sequence
<220>
<223> forward primer
<400> 1
ggttggccaa tctactccag g 21

<210> 2
<211> 22
<212> DNA
<213> Artificial Sequence
<220>
<223> reverse primer
<400> 2
tggtctcctt aaacctgtct tg 22

<210> 3
<211> 27
<212> DNA
<213> Homo sapiens
<400> 3
gttcactagc aacctcaaac agacacc 27

<210> 4
<211> 53
<212> DNA
<213> Artificial Sequence
<220>
<221> modified_base
<222> 53
<223> phosphorylated
<400> 4
ggtgtctgtt tgaggttgct agtgaactat gaggtggtag gatgggtagt ggt 53

<210> 5
<400> 5
000

<210> 6
<211> 21
<212> DNA
<213> Artificial Sequence
<220>
<223> forward primer
<400> 6
catgtacgtt gctatccagg c 21

<210> 7
<211> 21
<212> DNA
<213> Artificial Sequence
<220>
<223> reverse primer
<400> 7
ctccttaatg tcacgcacga t 21

<210> 8
<211> 29
<212> DNA
<213> Homo sapiens
<400> 8
gtgaggatct tcatgaggta gtcagtcag 29

<210> 9
<211> 55
<212> DNA
<213> Artificial Sequence
<220>
<221> modified_base
<222> 55
<223> phosphorylated
<400> 9
ctgactgact acctcatgaa gatcctcact atgaggtggt aggatgggta gtggt 55

<210> 10
<400> 10
000

<210> 11
<400> 11
000

<210> 12
<211> 16
<212> DNA
<213> Artificial Sequence
<220>
<223> complementary sequence
<400> 12
gggttggggg aggggg 16

<210> 13
<211> 21
<212> DNA
<213> Artificial Sequence
<220>
<223> forward primer
<400> 13
ggttggccaa tctactccag g 21

<210> 14
<211> 22
<212> DNA
<213> Artificial Sequence
<220>
<223> reverse primer
<400> 14

tggtctcctt aaacctgtct tg 22

<210> 15
<211> 27
<212> DNA
<213> Homo sapiens
<400> 15
gttcactagc aacctcaaac agacacc 27

<210> 16
<211> 53
<212> DNA
<213> Artificial Sequence
<220>
<221> modified_base
<222> 53
<223> phosphorylated
<400> 16
ggtgtctgtt tgaggttgct agtgaactat gaggtggtag gatgggtagt ggt 53

<210> 17
<211> 26
<212> DNA
<213> Artificial Sequence
<220>
<221> modified_base
<222> 26
<223> labeled with BODIPY-FL
<400> 17
accactaccc atcctaccac ctcata 26

<210> 18
<211> 50
<212> DNA
<213> Artificial Sequence
<220>
<221> modified_base
<222> 50
<223> phosphorylated
<400> 18
cgatgcagcg agtggcggcg atgggtgtct gtttgaggtt gctagtgaac 50

<210> 19
<211> 23
<212> DNA
<213> Artificial Sequence
<220>
<221> modified_base
<222> 1
<223> labeled with BODIPY-FL
<400> 19
catcgccgcc actcgctgca tcg 23

<210> 20
<400> 20
000

<210> 21
<211> 16
<212> DNA
<213> Artificial Sequence

<220>
<223> complementary sequence
<400> 21
gggaaggggg aggggg 16

<210> 22
<211> 47
<212> DNA
<213> Artificial Sequence
<220>
<223> binding probe
<400> 22
ggtgtctgtt tgaggttgct agtgaactat gaggaagggg gaggggg 47

<210> 23
<211> 21
<212> DNA
<213> Artificial Sequence
<220>
<223> forward primer
<400> 23
catgtacgtt gctatccagg c 21

<210> 24
<211> 21
<212> DNA
<213> Artificial Sequence
<220>
<223> reverse primer
<400> 24
ctccttaatg tcacgcacga t 21

<210> 25
<211> 17
<212> DNA
<213> Artificial Sequence
<220>
<223> forward primer
<400> 25
cgctgaatga ggcttcc 17

<210> 26
<211> 18
<212> DNA
<213> Artificial Sequence
<220>
<223> reverse primer
<400> 26
cagcacattg ccaaacac 18

<210> 27
<211> 31
<212> DNA
<213> Artificial Sequence
<220>
<223> binding probe
<400> 27
cttccattgg gtgccagctt gggggagggg g 31

<210> 28
<400> 28

000

<210> 29
<211> 17
<212> DNA
<213> Artificial Sequence
<220>
<223> forward primer
<400> 29
agctctcttg ccccatg 17

<210> 30
<211> 17
<212> DNA
<213> Artificial Sequence
<220>
<223> reverse primer
<400> 30
gccagcgaag tcacgaa 17

<210> 31
<211> 17
<212> DNA
<213> Artificial Sequence
<220>
<223> forward primer
<400> 31
tggcctcacg agaacag 17

<210> 32
<211> 18
<212> DNA
<213> Artificial Sequence
<220>
<223> reverse primer
<400> 32
gagtcccatc accaggtc 18

<210> 33
<211> 36
<212> DNA
<213> Artificial Sequence
<220>
<223> binding probe
<400> 33
ccatcgcccg gactccgaga ctcttggggg aggggg 36

<210> 34
<211> 20
<212> DNA
<213> Artificial Sequence
<220>
<223> forward primer
<400> 34
agtggtggct aatgagagaa 20

<210> 35
<211> 17
<212> DNA
<213> Artificial Sequence
<220>

<223> reverse primer
<400> 35
aaggagtggc agcaagt 17

<210> 36
<211> 26
<212> DNA
<213> Artificial Sequence
<220>
<223> forward primer
<400> 36
ttcttctgtc atttgcacat ttatct 26

<210> 37
<211> 21
<212> DNA
<213> Artificial Sequence
<220>
<223> reverse primer
<400> 37
aactgaccct ttatgacgta g 21

<210> 38
<211> 33
<212> DNA
<213> Artificial Sequence
<220>
<223> binding probe
<400> 38
cactcgatca aactgtggtc ttgggggagg ggg 33

SEQUENCE LISTING

<110> National Institute of Advanced Industrial Science and Technology
J-Bio 21 Corporation
Nippon Steel Kankyo Engineering Co.,Ltd.
<120> Universal nucleic acid probe set and use thereof
<130> PCT-83-ENJBS
<150> JP2008-196513
<151> 2008-07-30
<150> JP2009-142608
<151> 2009-06-15
<160> 38

<210> 1
<211> 21
<212> DNA
<213> Artificial Sequence
<220>
<223> forward primer
<400> 1
ggttggccaa tctactccag g 21

<210> 2
<211> 22
<212> DNA
<213> Artificial Sequence
<220>
<223> reverse primer
<400> 2
tggtctcctt aaacctgtct tg 22

<210> 3
<211> 27
<212> DNA
<213> Homo sapiens
<400> 3
gttcactagc aacctcaaac agacacc 27

<210> 4
<211> 53
<212> DNA
<213> Artificial Sequence
<220>
<221> modified_base
<222> 53
<223> phosphorylated
<400> 4
ggtgtctgtt tgaggttgct agtgaactat gaggtggtag gatgggtagt ggt 53

<210> 5
<400> 5
000

<210> 6
<211> 21
<212> DNA
<213> Artificial Sequence
<220>
<223> forward primer
<400> 6
catgtacgtt gctatccagg c 21

<210> 7
<211> 21
<212> DNA
<213> Artificial Sequence
<220>
<223> reverse primer

<400> 7
ctccttaatg tcacgcacga t 21

<210> 8
<211> 29
<212> DNA
<213> Homo sapiens
<400> 8
gtgaggatct tcatgaggta gtcagtcag 29

<210> 9
<211> 55
<212> DNA
<213> Artificial Sequence
<220>
<221> modified_base
<222> 55
<223> phosphorylated
<400> 9
ctgactgact acctcatgaa gatcctcact atgaggtggt aggatgggta gtggt 55

<210> 10
<400> 10
000

<210> 11
<400> 11
000

<210> 12
<211> 16
<212> DNA
<213> Artificial Sequence
<220>
<223> complementary sequence
<400> 12
gggttggggg aggggg 16

<210> 13
<211> 21
<212> DNA
<213> Artificial Sequence
<220>
<223> forward primer
<400> 13
ggttggccaa tctactccag g 21

<210> 14
<211> 22
<212> DNA
<213> Artificial Sequence
<220>
<223> reverse primer
<400> 14
tggtctcctt aaacctgtct tg 22

<210> 15
<211> 27
<212> DNA
<213> Homo sapiens
<400> 15
gttcactagc aacctcaaac agacacc 27

<210> 16
<211> 53
<212> DNA
<213> Artificial Sequence
<220>

<221> modified_base
<222> 53
<223> phosphorylated
<400> 16
ggtgtctgtt tgaggttgct agtgaactat gaggtggtag gatgggtagt ggt 53

<210> 17
<211> 26
<212> DNA
<213> Artificial Sequence
<220>
<221> modified_base
<222> 26
<223> labeled with BODIPY-FL
<400> 17
accactaccc atcctaccac ctcata 26

<210> 18
<211> 50
<212> DNA
<213> Artificial Sequence
<220>
<221> modified_base
<222> 50
<223> phosphorylated
<400> 18
cgatgcagcg agtggcggcg atgggtgtct gtttgaggtt gctagtgaac 50

<210> 19
<211> 23
<212> DNA
<213> Artificial Sequence
<220>
<221> modified_base
<222> 1
<223> labeled with BODIPY-FL
<400> 19
catcgccgcc actcgctgca tcg 23

<210> 20
<400> 20
000

<210> 21
<211> 16
<212> DNA
<213> Artificial Sequence
<220>
<223> complementary sequence
<400> 21
gggaaggggg aggggg 16

<210> 22
<211> 47
<212> DNA
<213> Artificial Sequence
<220>
<223> binding probe
<400> 22
ggtgtctgtt tgaggttgct agtgaactat gaggaagggg gaggggg 47

<210> 23
<211> 21
<212> DNA
<213> Artificial Sequence
<220>
<223> forward primer
<400> 23

catgtacgtt gctatccagg c 21

<210> 24
<211> 21
<212> DNA
<213> Artificial Sequence
<220>
<223> reverse primer
<400> 24
ctccttaatg tcacgcacga t 21

<210> 25
<211> 17
<212> DNA
<213> Artificial Sequence
<220>
<223> forward primer
<400> 25
cgctgaatga ggcttcc 17

<210> 26
<211> 18
<212> DNA
<213> Artificial Sequence
<220>
<223> reverse primer
<400> 26
cagcacattg ccaaacac 18

<210> 27
<211> 31
<212> DNA
<213> Artificial Sequence
<220>
<223> binding probe
<400> 27
cttccattgg gtgccagctt gggggagggg g 31

<210> 28
<400> 28
000

<210> 29
<211> 17
<212> DNA
<213> Artificial Sequence
<220>
<223> forward primer
<400> 29
agctctcttg ccccatg 17

<210> 30
<211> 17
<212> DNA
<213> Artificial Sequence
<220>
<223> reverse primer
<400> 30
gccagcgaag tcacgaa 17

<210> 31
<211> 17
<212> DNA
<213> Artificial Sequence
<220>
<223> forward primer
<400> 31
tggcctcacg agaacag 17

<210> 32
<211> 18
<212> DNA
<213> Artificial Sequence
<220>
<223> reverse primer
<400> 32
gagtcccatc accaggtc 18

<210> 33
<211> 36
<212> DNA
<213> Artificial Sequence
<220>
<223> binding probe
<400> 33
ccatcgcccg gactccgaga ctcttggggg aggggg 36

<210> 34
<211> 20
<212> DNA
<213> Artificial Sequence
<220>
<223> forward primer
<400> 34
agtggtggct aatgagagaa 20

<210> 35
<211> 17
<212> DNA
<213> Artificial Sequence
<220>
<223> reverse primer
<400> 35
aaggagtggc agcaagt 17

<210> 36
<211> 26
<212> DNA
<213> Artificial Sequence
<220>
<223> forward primer
<400> 36
ttcttctgtc atttgcacat ttatct 26

<210> 37
<211> 21
<212> DNA
<213> Artificial Sequence
<220>
<223> reverse primer
<400> 37
aactgaccct ttatgacgta g 21

<210> 38
<211> 33
<212> DNA
<213> Artificial Sequence
<220>
<223> binding probe
<400> 38
cactcgatca aactgtggtc ttgggggagg ggg 33

## Description

### Technical Field

This relates to the field of genetic engineering, and more specifically, to a nucleic acid probe set useful for analyzing a nucleic acid and a method for using the same.

### Background Art

In detection methods of target nucleic acids, single-stranded nucleic acid probes are widely used. These single-stranded nucleic acid probes have base sequences designed to hybridize specifically to the target nucleic acids, and are labeled with fluorescent substances. As an example of these single-stranded nucleic acid probes, there is a Q-probe (Quenching Probe) that uses, as a probe-labeling fluorescent substance, a fluorescent substance the fluorescence emission of which is reduced under the action of guanine when the fluorescent substance is located near guanine compared with when it is in a normal state. By adding the Q-probe to a solution to be measured and conducting a measurement of fluorescence, SNP genotyping or quantification of a gene can be performed simply and conveniently. The Q-probe has excellent advantages in that the structure of the probe is simple, no trial-and-error approach is needed for the designing of the probe, and highly-accurate measurement results can be obtained (see, for example, Patent Document 1 and Patent Document 2).
As such a conventional, single-stranded nucleic acid probe, however, a fluorescently-labeled nucleic acid probe having a different base sequence has to be prepared specifically for every target nucleic acid to be detected. Such a fluorescently-labeled nucleic acid probe is accompanied by problems that it is relatively costly and its synthesis requires a long time, and therefore, involves problems that an experiment making use of it is costly and requires a lot of time in preparation.
With the foregoing in view, the present inventors proposed to design a Q-Probe as a complex formed of plural nucleic acids. Described specifically, the present inventors designed nucleic acid probe sets (universal Q-probe sets) each of which comprises (A) a fluorescent probe and (B) a binding probe having (b1) a fluorescent probe binding region complementary to the fluorescent probe and (b2) a sequence complementary to a target nucleic acid sequence (C), and have been working toward their practical use.

### Prior Art Documents

### Patent Documents

Patent Document 1: JP-B-3437816
Patent Document 2: JP-B-3963422

### Disclosure of the Invention

### Problem to Be Solved by the Invention

The use of a universal Q-probe set in a real-time PCR experiment can drastically reduce the cost required for the preparation of a probe. However, the fluorescence quenching efficiency available from the use of a universal Q-probe set is limited to as low as a half to one-third or so of that available from the use of a conventional, single-stranded Q-probe, and accordingly, there is an outstanding desire for an improvement in fluorescence quenching efficiency for practical use.
Therefore, a first object of the present invention is to provide a universal Q-probe set with a fluorescence quenching efficiency improved to a similar level as those of conventional, single-stranded Q-probes and also a method for designing such a universal Q-probe set, and further to provide a method for its use.
A second object of the present invention is to provide an oligonucleotide that can form a stable complex which does not dissociate in a water system, and further to provide a method for its use.
A third object of the present invention is to provide a universal Q-probe set with an improved fluorescence quenching efficiency and also a method for designing the universal Q-probe set, and further to provide a method for its use.

## Means for Solving the Problem

The above-described first object can be achieved by the present invention to be described hereinafter. Described specifically, the present invention provides, in a first thereof, a nucleic acid probe set comprising (A) a fluorescent probe, which is formed of an oligonucleotide including (a) a nucleotide unit labeled with (d) a fluorescent substance, and (B) a binding probe formed of an oligonucleotide having (b1) a fluorescent probe binding region, which can hybridize to the fluorescent probe (A), and (b2) a target nucleic acid binding region, which can hybridize to a target nucleic acid sequence (C), wherein the fluorescent substance (d) is a fluorescent substance which changes in fluorescent character upon interaction with guanine, and at least one of nucleotide units which constitute the fluorescent probe (A) is an artificial nucleotide unit having a function to raise a dissociation temperature between the probe (A) and the fluorescent probe binding region (b1).

In the nucleic acid probe set according to the first aspect of the present invention, the artificial nucleotide unit having the function to raise the dissociation temperature may preferably be at least one artificial nucleotide unit selected from the group consisting of LNA, PNA, ENA, 2' 4' -BNA$^{NC}$ and 2' , 4' -BNA$^{COC}$ units.

In the nucleic acid probe set according to the first aspect of the present invention, preferably at least one-third, more preferably at least 80% of the nucleotide units which constitute the fluorescent probe (A) may be artificial nucleotide units.

In the nucleic acid probe set according to the first aspect of the present invention, the fluorescent substance (d) may preferably be any one selected from the group consisting of fluorescein, fluorescein-4-isothiocyanate, tetrachlorofluorescein, hexachlorofluorescein, tetrabromosulfonefluorescein, EDANS (5-(2-aminoethylamino)-1-naphthalensulfonic acid), 6-carboxy-4',5'-dichloro-2',7'-dimethoxyfluorescein (6-JOE), 3,6-diamino-9-[2,4-bis(lithiooxycarbonyl)phenyl]-4-[lithioxysulfonyl)-5-sulfonatoxanthylium/3,6-diamin o-9-[2,5-bis(lithiooxycarbonyl)phenyl]-4-(lithooxys ulfonyl)-5-sulfonatoxanthylium, [2,3,3,7,7,8-hexamethyl-5-[4-[5-(2,5-dioxo-3-pyrrol in-1-yl)pentylcarbamoyl]phenyl]-2,3,7,8-tetrahydro-9-azonia-1H-pyrano[3,2-f:5,6-f']diindole-10,12-disu lfonic acid 12-sodium] anion salt, 2-oxo-6,8-difluoro-7-hydroxy-2H-1-benzopyran-3-carb oxylic acid, rhodamine 6G, carboxyrhodamine 6G, tetramethylrhodamine, carboxytetramethylrhodamineand 4,4-difluoro-5,7-dimethyl-4-bora-3a,4a-diaza-s-inda cene-3-propionic acid.

In the nucleic acid probe set according to the first aspect of the present invention, the target nucleic acid binding region (b2) may be located preferably on a side of a 5' -end of the binding probe (B), and the nucleotide unit (a) labeled with the fluorescent substance (d) is a 3'-terminal nucleotide unit of the fluorescent probe (A).

The present invention also provides, in the first aspect thereof, a method for detecting a target nucleic acid, which comprises the following steps (1) to (4):

(1) hybridizing the nucleic acid probe set according to the first aspect of the present invention and the target nucleic acid with each other,
(2) then measuring the fluorescence intensity of a hybridized complex of the nucleic acid probe set and target nucleic acid,
(3) conducting the steps (1) and (2) by changing a ratio of the nucleic acid probe set to the target nucleic acid, and
(4) comparing the fluorescence intensities obtained from the steps (2) and (3).

The present invention further provides, in the first aspect thereof, a method for analyzing a nucleic acid for a base sequence polymorphism, which comprises the following steps (1) to (4):

(1) hybridizing the nucleic acid probe set according to the first aspect of the present invention and a target nucleic acid with each other,
(2) then measuring a temperature dependence of fluorescence intensity with respect to a hybridized complex of the nucleic acid probe set and target nucleic acid,
(3) conducting the steps (1) and (2) by using another nucleic acid in place of the target nucleic acid, and
(4) comparing the temperature dependences of fluorescence intensity as obtained from the steps (2) and (3).

The present invention still further provides, in the first aspect thereof, a method, which comprises conducting a melting curve analysis on a complex of the nucleic acid probe set according to the first aspect of the present invention and a target nucleic acid.

The present invention provides, in a second aspect thereof, an oligonucleotide probe comprising nucleotide units including (a') a nucleotide unit labeled with (h) a labeling substance, a part or all of said nucleotide units being an artificial nucleotide unit or units having a function to raise a dissociation temperature of the oligonucleotide probe from a complementary strand, said dissociation temperature of the oligonucleotide probe from the complementary strand being 100°C or higher under normal pressure conditions.

In the second aspect of the present invention, the artificial nucleotide unit or units having the function to raise the dissociation temperature from the complementary strand may preferably be one or more artificial nucleotide units each selected from the group consisting of LNA, PNA, ENA, 2', 4' -$BNA^{NC}$ and 2' 4' -$BNA^{COC}$ units; and the labeling substance (h) may preferably be a fluorescent substance, quencher substance, protein or functional group.

The present invention also provides, in the second aspect thereof, a method, which comprises hybridizing the oligonucleotide probe according to the second aspect of the present invention with (E) an oligonucleotide having a complementary base sequence to label the oligonucleotide (E) with the labeling substance (h); and the nucleic acid probe set according to the first aspect of the present invention, wherein the oligonucleotide probe according to the second aspect of the present invention, in which the labeling substance (h) is a fluorescent substance which changes in fluorescent character upon interaction with guanine, is used as a fluorescent probe (A).

The present invention provides, in a third aspect thereof, a nucleic acid probe set comprising (A) one fluorescent probe, which is formed of an oligonucleotide including (a) a nucleotide unit labeled with (d) a fluorescent substance, and (B) one binding probe formed of an oligonucleotide having (b1) one fluorescent probe binding region, which can hybridize to the fluorescent probe (A), and (b2) one target nucleic acid binding region, which can hybridize to a target nucleic acid sequence (C), wherein the fluorescent substance (d) is a fluorescent substance which changes in fluorescent character upon interaction with guanine, the nucleotide unit (a) is a 3'-terminal nucleotide unit of the fluorescent probe (A), and the target nucleic acid binding region (b2) is located on a side of a 5' -end of the binding probe (B).

**Advantageous Effects of the Invention**

According to the first aspect of the present invention, there is provided a universal Q-probe set with a fluorescence quenching efficiency improved to a similar level as those of conventional, single-stranded Q-probes. The use of the universal Q-probe set according to the first aspect of the present invention in place of a single-stranded Q-probe makes it possible to significantly reduce the cost required for a real-time PCR experiment.

According to the second aspect of the present invention, there is provided an oligonucleotide capable of forming a stable complex that does not dissociate in a water system.

According to the third aspect of the present invention, there is provided a universal Q-probe set with an improved fluorescence quenching efficiency. The use of the universal Q-probe set according to the third aspect of the present invention in place of a conventional single-stranded Q-probe makes it possible to significantly reduce the cost required for a real-time PCR experiment.

**Brief Description of the Drawings**

[FIG. 1] FIG. 1 is a schematic diagram showing a nucleic acid probe complex in the first aspect of the present invention before hybridization with a target nucleic acid (C).

[FIG. 2] FIG. 2 is a schematic diagram showing on an enlarged scale an area near a fluorescent substance (d) of the nucleic acid probe complex in the first aspect of the present invention.

[FIG. 3] FIG. 3 is a schematic diagram illustrating a nucleic acid probe complex in the first aspect of the present invention, which has a binding probe with a target nucleic acid binding region thereof being located on a side of a 5'-end.

[FIG. 4] FIG. 4 is a schematic diagram illustrating another nucleic acid probe complex in the first aspect of the present invention, which has a binding probe with a target nucleic acid binding region thereof being located on a side of a 3'-end.

[FIG. 5] FIG. 5 is a schematic diagram showing another nucleic acid probe complex in the first aspect of the present invention, which comprises a binding probe, said binding probe having two fluorescent probe binding regions, and two fluorescent probes.

[FIG. 6] FIG. 6 is a schematic diagram showing a nucleic acid probe set according to the first aspect of the present invention as used in Example 1 and a target nucleic acid sequence upon hybridization thereof.

[FIG. 7] FIG. 7 is a graph obtained by conducting real-time PCR amplification with the nucleic acid probe set according to the first aspect of the present invention while using a β-globin gene as a target nucleic acid, and plotting fluorescence quenching efficiencies.

[FIG. 8] FIG. 8 is a graph obtained by conducting real-time PCR amplification with a nucleic acid probe set, which had a fluorescent probe constituted of DNA units alone, while using the β-globin gene as a target nucleic acid, and plotting fluorescence quenching efficiencies.

[FIG. 9] FIG. 9 is a graph obtained by conducting real-time PCR amplification with a nucleic acid probe set according to the first aspect of the present invention while using a portion of a β-actin gene as a target nucleic acid sequence, and plotting fluorescence quenching efficiencies.

[FIG. 10] FIG. 10 is a graph obtained by conducting real-time PCR amplification with a nucleic acid probe set, which had a fluorescent probe constituted of DNA units alone, while using the portion of the β-actin gene as a target nucleic acid sequence, and plotting fluorescence quenching efficiencies.

[FIG. 11] FIG. 11 is a graph showing the results of a melting curve analysis.

[FIG. 12] FIG. 12 is a schematic diagram showing a nucleic acid probe complex in the third aspect of the present invention before hybridization with a target nucleic acid (C).

[FIG. 13] FIG. 13 is a schematic diagram showing on an enlarged scale an area near a fluorescent substance (d) of the nucleic acid probe complex in the third aspect of the present invention.

[FIG. 14] FIG. 14 is a schematic diagram illustrating a nucleic acid probe complex in the third aspect of the present invention, which has a binding probe (B) with a fluorescent probe binding region (b1) thereof being located on a side of a 3'-end.

[FIG. 15] FIG. 15 is a schematic diagram illustrating a nucleic acid probe complex in the third aspect of the present invention, which has a binding probe (B) with a fluorescent probe binding region (b1) thereof being located on a side of a 5'-end.

[FIG. 16] FIG. 16 is a schematic diagram showing a nucleic acid probe set according to the third aspect of the present invention as used in Example 5 and a target nucleic acid sequence upon hybridization thereof.

[FIG. 17] FIG. 17 is a graph obtained by conducting real-time PCR amplification with a nucleic acid probe set according to the third aspect of the present invention while using the β-globin gene as a target nucleic acid, and plotting fluorescence quenching efficiencies.

[FIG. 18] FIG. 18 is a graph obtained by conducting real-time PCR amplification with a nucleic acid probe set of Comparative Example 3 while using the β-globin gene as a target nucleic acid, and plotting fluorescence quenching efficiencies.

[FIG. 19] FIG. 19 is a graph showing the results of a melting curve analysis.

[FIG. 20] FIG. 20 is a schematic diagram illustrating an outline of Application Example 1.

[FIG. 21] FIG. 21 is a schematic diagram illustrating a fluorescent probe and binding probe in Application Example 2.

[FIG. 22] FIG. 22 is a schematic diagram illustrating a hybridized state of the fluorescent probe and binding probe in Application Example 2.

[FIG. 23] FIG. 23 is a schematic diagram illustrating a nucleic acid probe set of Application Example 2 in a state that the nucleic acid probe set had bound to a target nucleic acid.

[FIG. 24] FIG. 24 is a schematic diagram illustrating a fluorescent probe and binding probe in Application Example 3.

[FIG. 25] FIG. 25 is a schematic diagram illustrating a hybridized state of the fluorescent probe and binding probe in Application Example 3.

[FIG. 26] FIG. 26 is a schematic diagram illustrating a nucleic acid probe set of Application Example 3 in a state that the nucleic acid probe set had bound to a target nucleic acid.

[FIG. 27] FIG. 27 is a schematic diagram illustrating a fluorescent probe and binding probe in Application Example 4.

[FIG. 28] FIG. 28 is a schematic diagram illustrating a hybridized state of the fluorescent probe and binding probe in Application Example 4.

[FIG. 29] FIG. 29 is a schematic diagram illustrating a nucleic acid probe set of Application Example 4 in a state that the nucleic acid probe set had bound to a target nucleic acid.

[FIG. 30] FIG. 30 is a schematic diagram illustrating a fluorescent probe and binding probe in Application Example 5.

[FIG. 31] FIG. 31 is a schematic diagram illustrating a hybridized state of the fluorescent probe and binding probe in Application Example 5.

[FIG. 32] FIG. 32 is a schematic diagram illustrating a nucleic acid probe set of Application Example 5 in a state that the nucleic acid probe set had bound to a target nucleic acid.

[FIG. 33] FIG. 33 is a graph showing the results of a melting curve analysis of ADRB2 gene polymorphisms.

[FIG. 34] FIG. 34 is a graph showing the results of a melting curve analysis of ADRB3 gene polymorphisms.

[FIG. 35] FIG. 35 is a graph showing the results of a melting curve analysis of UCP1 gene polymorphisms.

## Best Modes for Carrying out the Invention

Best modes for carrying out the present invention will next be described with reference to drawings. It is to be noted that in the present invention, the hybridized complex of the fluorescent probe (A) and binding probe (B) may be called "the nucleic acid probe complex".

Further, the term "nucleotide" as used herein is not limited to deoxyribonucleotides as basic units of DNA and ribonucleotides as basic units of RNA, but shall be construed to also include artificially-synthesized monomers such as LNAs (Locked Nucleic Acids) and peptide nucleic acids (PNAs). The term "oligonucleotide" as used herein means an oligomer formed from a nucleotide monomer. This oligomer may be formed from only deoxyribonucleotide, ribonucleotide, LNA or PNA units, or may be a chimeric molecule thereof.

The term "target nucleic acid" as used herein means a nucleic acid to be subjected to quantification, analysis or the like, and shall be construed to also include a portion or portions of one or more of various nucleic acids or genes in some instances. Monomers that constitute target nucleic acids can be of any type, and deoxyribonucleotides, ribonucleotides, LNAs, PNAs, artificially-modified nucleic acids and the like can be mentioned.

The term "target nucleic acid sequence (C) " as used herein means a base sequence region, which is located in a target nucleic acid and specifically hybridizes to a target nucleic acid binding region (b2) in a binding probe (B) that constitutes a nucleic acid probe set according to the present invention. Further, the term "normal pressure" as used herein means one(1) atmospheric pressure.

<First Aspect of the Present Invention>

Examples of the nucleic acid probe set according to the first aspect of the present invention are shown in FIGS. 1, and 3 to 6. In these figures, there are shown fluorescent probes (A), binding probes (B), target nucleic acid sequences (C), and fluorescent substances (d).

The nucleic acid probe set according to the first aspect of the present invention comprises the fluorescent probe (A) and the binding probe (B). The binding probe (B) has a fluorescent probe binding region (b1), which has a base sequence complementary to the fluorescent probe (A), and a target nucleic acid binding region (b2), which has a base sequence complementary to the target nucleic acid sequence (C).

The fluorescent probe (A), which constitutes the nucleic acid probe set according to the first aspect of the present invention, is an oligonucleotide including a nucleotide unit (a) labeled with the fluorescent substance (d). No particular limitation is imposed on the base sequence of the fluorescent probe (A) insofar as the fluorescent probe (A) can hybridize with the fluorescent probe binding region (b1) in the binding probe (B). The base sequence of the fluorescent probe (A), therefore, does not depend on the base sequence of a target nucleic acid to be detected or analyzed. Accordingly, the fluorescent probe (A) that constitutes the nucleic acid probe set according to the first aspect of the present invention is not required to have a base sequence corresponding to the specific target nucleic acid, and the fluorescent probe (A) of the same base sequence can be commonly used for different target nucleic acids. The nucleic probe set according to the first aspect of the present invention is, therefore, called "a universal nucleic probe set" by the present inventors. The use of the nucleic acid probe set according to the first aspect of the present invention for the analysis of a target nucleic acid has an advantage in that it is no longer needed to prepare a fluorescent probe, which has a costly fluorescent substance, specifically for the target nucleic acid to be detected or analyzed and the production cost of the fluorescent probe can be minimized.

The fluorescent probe (A) includes, as at least one of nucleotide units as the basic units of the probe, an artificial nucleotide unit or units having a function to raise the dissociation temperature between the probe (A) and the fluorescent probe binding region (b1). Owing to the inclusion of the artificial nucleotide unit or units in the fluorescent probe (A), Tm between the fluorescent probe (A) and the fluorescent probe binding region (b1) becomes higher. By increasing the proportion of the artificial nucleotide unit or units in the fluorescent probe (A), the Tm between the fluorescent probe (A) and the binding probe (B) can be easily made higher than the Tm between the target nucleic acid sequence (C) and the target nucleic acid binding region (b2), thereby making it possible to provide the nucleic acid probe set according to the first aspect of the present invention with higher stability at elevated temperatures, and hence, with improved reliability as a fluorescent probe.

By increasing the proportion of the artificial nucleotide unit or units in the fluorescent probe (A), The Tm between the fluorescent probe (A) and the binding probe (B) can be made higher than the thermal denaturation temperature (for example, 95°C) of PCR, so that the fluorescentprobe (A) and the binding probe (B) canalways remain as a stable nucleic acid probe complex during PCR cycles.

As examples of the artificial nucleotide unit or units having the function to raise the dissociation temperature between the fluorescent probe (A) and the fluorescent probe binding region (b1) as described above, LNA, PNA, ENA, 2' ,4' -BNA$^{NC}$ and 2' ,4' -BNA$^{COC}$ units can be mentioned.

An LNA monomer is a nucleotide having two ring structures that the 2'-oxygen and 4'-carbon atoms of ribose are connected together via a methylene unit. Due to the inclusion of these two ring structures, the LNA monomer has low structural freedom, and compared with DNA or RNA monomer, strongly hybridizes with a complementary strand. It is, therefore, known that by substituting one or more mononucleotide units (DNA monomers), which make up an oligonucleotide formed of DNA units, to a like number of LNA units, the Tm between the oligonucleotide and a complementary strand rises.

PNA is an abbreviation of peptide nucleic acid, and has a structure that a structure composed of N-(2-aminoethyl) glycine units linked together via amide bonds is contained as a backbone and base moieties (purine rings or pyrimidine rings) are connected to nitrogen atoms in the backbone via $-COCH_2-$. Different from DNA or RNA monomer, PNA monomer does not produce strong electrostatic repulsion against a complementary strand as no charge exists on its phosphate moieties. The dissociation temperature from the complementary strand, therefore, rises when one or more DNA units are substituted to a like number of PNA units.

ENA is an abbreviation of 2'-O,4'-C-ethylene-bridged nucleic acid, and has a structure that the 2-O and 4-C atoms of a furanose ring are bridged together via an ethylene unit. It is known that by substituting one or more of mononucleotide units (DNA units), which make up an oligonucleotide formed of the DNA units, to a like number of ENA units, the Tm between the oligonucleotide and a complementary strand rises.

BNA is an abbreviation of bridged nucleic acid. 2' ,4' -$BNA^{NC}$ has a structure that in a furanose ring, the 2-O atom is bridged to the 4-C atom via -$NRCH_2$- (R: methyl group), while 2' ,4' -$BNA^{COC}$ has a structure that the 2-O and 4-C atoms of a furanose ring are bridged together via -$CH_2OCH_2$-. Each of these artificial nucleotide units is also known to raise the Tm between an oligonucleotide formed of DNA units and a complementary strand when one or more of nucleotide units (DNAmonomers) making up the oligonucleotide are substituted to a like number of such BNA units.

The proportion of the artificial nucleotide unit or units in the fluorescent probe (A), said proportion being required to make the Tm between the fluorescent probe (A) and the binding probe (B) higher than the thermal denaturation temperature of PCR, also depends on the base number and base sequence of the fluorescent probe (A), and cannot be specified. Preferably, however, the proportion of the artificial nucleotide unit or units may be at least one third of the entire nucleotide units, with at least 80% thereof being more preferred.

By increasing the proportion of the artificial nucleotide unit or units in the fluorescent probe (A), the interaction between the fluorescent probe (A) and the fluorescent probe binding region (b1) is strengthened. The base numbers of the fluorescent probe (A) and fluorescent probe binding region (b1) can, therefore, be decreased compared with the case that the fluorescent probe (A) is formed of DNA units alone. Upon synthesis of a binding probe (B) of a large base number, an error occurs. However, the use of a fluorescent probe binding region (b1) of a decreased base number can reduce the error, and can increase the synthesis yield of a binding probe (B). This leads to a reduction in the production cost for the probe set according to the first aspect of the present invention.

Usable as the fluorescent substance (d) with which the fluorescent probe (A) is labeled in the first aspect of the present invention is a fluorescent substance (d) which changes in fluorescent character upon interaction with guanine. In the present invention, the term "fluorescent character" means fluorescence intensity, the expression "guanine and the fluorescent substance interact with each other to change the fluorescent character of the fluorescent substance" means that the fluorescence intensity of the fluorescent substance in a state that guanine and the fluorescent substance are not interacting with each other is different from its fluorescence intensity in a state that they are interacting with each other, and on the extent of this difference, no limitation shall be imposed. Further, the term "quenched or quenching" of fluorescence means that upon interaction of a fluorescent substance with guanine, the fluorescence intensity decreases compared with the fluorescence intensity when the fluorescent substance is not interacting with guanine, and on the extent of this decrease, no limitation shall be imposed.

Examples of fluorescent substances, which can be suitably used in the nucleic acid probe set according to the first aspect of the present invention, include fluorescein and its derivatives [e.g., fluorescein-4-isothiocyanate (FITC), tetrachlorofluorescein, hexachlorofluorescein, tetrabromosulfonefluorescein (TBSF), and derivatives thereof], EDANS (5-(2-aminoethylamino)-1-naphthalenesulfonic acid), 6-carboxy-4' ,5'-dichloro-2' ,7'-dimethoxyfluorescein (6-JOE), 3,6-diamino-9-[2,4-bis(lithiooxycarbonyl)phenyl]-4-(lithioxysulfonyl)-5-sulfonatoxanthylium/3,6-diamin o-9-[2,5-bis(lithiooxycarbonyl)phenyl]-4-(lithooxys ulfonyl)-5-sulfonatoxanthylium (available as "Alexa Fluor 488" from Invitrogen Corp.), [2,3,3,7,7,8-hexamethyl-5-[4-[5-(2,5-dioxo-3-pyrrol in-1-yl)pentylcarbamoyl]phenyl]-2,3,7,8-tetrahydro-9-azonia-1H-pyrano[3,2-f:5,6-f']diindole-10,12-disu lfonic acid 12-sodium] anion salt (available as "Alexa Fluor 532" from Invitrogen Corp.), Cy3 (GE Healthcare Bioscience), Cy5 (GE Healthcare Bioscience), 2-oxo-6,8-difluoro-7-hydroxy-2H-1-benzopyran-3-carb oxylic acid (available as "Pacific Blue" from Invitrogen Corp.), rhodamine 6G (R6G) and its derivatives (for example, carboxyrhodamine 6G (CR6G), tetramethylrhodamine (TMR), tetramethylrhodamine isothiocyanate (TMRITC), x-rhodamine, carboxytetramethylrhodamine (TAMRA)), Texas red (Invitrogen Corp.), 4,4-difluoro-5,7-dimethyl-4-bora-3a,4a-diaza-s-inda cene-3-propionic acid (available as "BODIPY-FL" from Invitrogen Corp.), BODIPY-FL/C3 (Invitrogen Corp.), BODIPY-FL/C6 (Invitrogen Corp.), BODIPY-5-FAM (Invitrogen Corp.), BODIPY-TMR (Invitrogen Corp.), BODIPY-TR (Invitrogen Corp.), BODIPY-R6G (Invitrogen Corp.), BODIPY564 (Invitrogen Corp.), and BODIPY581 (Invitrogen Corp.).

Of these, the use of fluorescein, fluorescein-4-isothiocyanate, tetrachlorofluorescein, hexachlorofluorescein, tetrabromosulfonefluorescein, EDANS, 6-JOE, Alexa Fluor 488, Alexa Fluor 532, Pacific Blue, rhodamine 6G, carboxyrhodamine 6G, tetramethylrhodamine, carboxytetramethylrhodamine or BODIPY-FL is more preferred, and the use of Pacific Blue, carboxyrhodamine 6G or BODIPY-FL is most preferred.

The target nucleic acid binding region (b2) of the binding probe (B) is designed such that, when the nucleic acid probe complex in the present invention has hybridized to the target nucleic acid sequence (C), the fluorescent substance (d) and a guanine base in a target nucleic acid can be brought into contact with each other. As a consequence, upon hybridization of the nucleic acid probe complex in the present invention with the target nucleic acid sequence (C), the fluorescence of the fluorescent substance (d) is quenched by the guanine base, and by detecting this quenching phenomenon, the target nucleic acid can be quantified.

The guanine, which can interact with the fluorescent substance (d) to give the fluorescence quenching effect, may exist either in the base sequence of the target nucleic acid sequence (C) or in a base sequence outside the target nucleic acid sequence (C), insofar as it exists in the target nucleic acid. When the guanine exists in the target nucleic acid sequence (C) and forms a base pair with cytosine in the hybridized binding probe (B), the interaction between the fluorescent substance (d) and the guanine is somewhat weaker although no particular problem arises. When the guanine forms no base pair for such a reason that the guanine exists outside the base sequence region of the target nucleic acid, on the other hand, the interaction between the fluorescent substance (d) and the guanine is facilitated. Accordingly, the latter situation is more preferred.

Referring next to FIG. 2, a description will be made about conditions under which the fluorescent substance (d) and a desired nucleotide unit (hereinafter called as "the nucleotide unit δ"), which has the guanine base in the target nucleic acid, can interact with each other when the nucleic acid probe complex in the first aspect of the present invention and the target nucleic acid sequence (C) have hybridized with each other. It is to be noted that the nucleotide unit, which has the guanine base at the 5'-end of the target nucleic acid sequence (C), is the nucleotide unit δ in the case of the description to be made hereinafter.

FIG. 2 shows, on an enlarged scale, an area around the fluorescent substance (d) in FIG. 1. The conditions under which the fluorescent substance (d) and the nucleotide unit δ can interact with each other depend *inter alia* on the length of a below-described spacer that connects the fluorescent substance (d) and the nucleotide unit (a) labeled with the fluorescent substance (d) each other, and cannot be specified. Generalizing these conditions, however, they can be defined as will be described hereinafter

The fluorescent probe (A) is now assumed to have hybridized with the binding probe (B). Base pairs are formed between the fluorescent probe binding region (b1) and the fluorescent probe (A). A nucleotide unit, which exists in the fluorescent probe binding region (b1) and is closest to the target nucleic acid binding region (b2), will hereinafter be called "the nucleotide unit α". The distance between this nucleotide unit α and a base, which exists in the binding probe (B) and forms a base pair with the nucleotide unit (a), will be designated as "X" expressed in terms of the number of base (s). It is to be noted that an adjacent nucleotide unit is counted as "X=1" and a nucleotide unit located adjacent with one base interposed therebetween is counted as "X=2".
In FIG. 2, the nucleotide unit α and the nucleotide unit, which exists in the binding probe (B) and forms a base pair with the nucleotide unit (a), are commonly a nucleotide unit having a thymine base located at the 5'-end in the fluorescent probe binding region (b1). Therefore, X is 0 (X=0).

On the other hand, base pairs are formed between the target nucleic acid sequence (C) and the target nucleic acid binding region (b2). A nucleotide unit, which exists in the target nucleic acid binding region (b2) and is closest to the nucleotide unit α, will be designated as "the nucleotide unit β". A nucleotide unit, which exists in the target nucleic acid sequence (C) and forms a base pair with the nucleotide unit β, will be designated as "the nucleotide unit γ". The distance between the nucleotide unit γ and the nucleotide unit δ will be designated as "Y" expressed in terms of the number of base (s). The counting method of Y is the same as X. In FIG. 2, the nucleotide unit γ and the nucleotide unit δ are commonly a nucleotide unit having a guanine base located at the 5' -end in the target nucleic acid sequence (C). Therefore, Y is 0 (Y=0).

As conditions for permitting interaction between the fluorescent substance (d) and the guanine in the nucleotide unit δ when the nucleic acid probe complex in the present invention and the target nucleic acid sequence (C) have hybridized with each other, the sum of X and Y may preferably be 5 or smaller. The sum of X and Y may be more preferably 3 or smaller, with 0 being most preferred, although it also depends on the length of the spacer connecting the fluorescent substance (d) and the nucleotide unit (a) labeled with the fluorescent substance (d).

Concerning the fluorescent probe (A) for use in the nucleic acid probe set according to the first aspect of the present invention, its production can rely upon a custom oligonucleotide synthesis service company (for example, Tsukuba Oligo Service Co., Ltd., Ibaraki, Japan) or the like. No particular limitation is imposed on the method for labeling the fluorescent substance on the oligonucleotide, and a conventionally-known labeling method can be used (Nature Biotechnology, 14, 303-308, 1996; Applied and Environmental Microbiology, 63, 1143-1147, 1997; Nucleic Acids Research, 24, 4532-4535, 1996).

When desired to couple a fluorescent substance, for example, to the 5'-terminal nucleotide unit, it is necessary to first introduce, for example, $-(CH_2)_n-SH$ as a spacer to a 5' -terminal phosphate group in a manner known *per se* in the art. As such a spacer, a commercial spacer can be used (for example, Midland Certified Reagent Company, U.S.A). In this case, n may stand for 3 to 8, with 6 being preferred. By coupling a fluorescent substance having SH reactivity or its derivative to the spacer, a fluorescently-labeled oligonucleotide can be obtained. The fluorescently-labeled oligonucleotide can be purified by reverse phase chromatography or the like to provide the fluorescent probe (A) for use in the present invention.

As an alternative, a fluorescent substance can also be coupled to the 3'-terminal nucleotide unit of the oligonucleotide. In this case, it is necessary to introduce, for example, $-(CH_2)_n-NH_2$ as a spacer to the OH group on the 3' -C atom of ribose or deoxyribose. As such a spacer, a commercial spacer can also be used (for example, Midland Certified Reagent Company, U.S.A). As an alternative method, it is also possible to introduce a phosphate group to the OH group on the 3' -C atom of ribose or deoxyribose and then to introduce, for example, $-(CH_2)_n-SH$ as a spacer to the OH group in the phosphate group. In this case, n may stand for 3 to 8, with 4 to 7 being preferred.

By coupling a fluorescent substance, which has reactivity to an amino group or SH group, or a derivative thereof to the above-described spacer, an oligonucleotide labeled with the fluorescent substance can be synthesized. The oligonucleotide can be purified by reverse phase chromatography or the like to provide the fluorescent probe (A) for use in the first aspect of the present invention. When desired to introduce $-(CH_2)_n-NH_2$ as a spacer, it is convenient to use a kit reagent (for example, Uni-link aminomodifier, Clonetech Laboratories, Inc.). The fluorescent substance can then be coupled to the oligonucleotide in a manner known *per se* in the art.

The nucleotide unit (a) in the fluorescent probe (A), said nucleotide unit (a) being labeled with the fluorescent substance, is not limited to one of both terminal nucleotide units in the oligonucleotide, and a nucleotide unit other than both the terminal nucleotide units can be labeled with the fluorescent substance (ANALYTICAL BIOCHEMISTRY, 225, 32-38, 1998).

Upon designing a nucleic acid probe set from one fluorescent probe (A) and one binding probe (B), two ways can be considered, one being to design a target nucleic acid binding region (b2) on the side of the 5' -end of the binding probe (B) as illustrated in FIG. 3, and the other to design it on the side of the 3' -end of the binding probe (B) as illustrated in FIG. 4. For enabling a fluorescent substance (d) and a guanine base in a target nucleic acid to interact with each other in these cases, it is necessary to conduct the labeling such that a nucleotide unit (a) labeled with the fluorescent substance (d) is located on the side of the 3'-end of the fluorescent probe (A) when the target nucleic acid binding region (b2) is located on the side of the 5' -end of the binding probe (B). When the target nucleic acid binding region (b2) is located on the side of the 3' -end of the binding probe (B), on the other hand, it is necessary to conduct the labeling such that the nucleotide unit (a) labeled with the fluorescent substance (d) is located on the side of the 5' -end of the fluorescent probe (A).

As a result of conducting many experiments and scrutinizing the fluorescence quenching efficiencies of such two types of nucleic acid probe sets as described above, the present inventors have found that the nucleic acid probe set, which is designed such that the target nucleic acid binding region (b2) is located on the side of the 5' -end of the binding probe (B) and the nucleotide unit (a) labeled with the fluorescent substance is located on the side of the 3'-end of the fluorescent probe (A), exhibits a higher quenching efficiency than the nucleic acid probe set, which is designed such that the target nucleic acid binding region (b2) is located on the side of the 3' -end of the binding probe (B) and the nucleotide unit (a) labeled with the fluorescent substance is located on the side of the 5' -end of the fluorescent probe (A). By designing the nucleic acid probe set according to the first aspect of the present invention such that the target nucleic acid binding region (b2) is located on the side of the 5' -end of the binding probe (B) and the nucleotide unit (a) labeled with the fluorescent substance is located on the side of the 3' -end of the fluorescent probe (A) and by using the nucleic acid probe set in a real-time PCR measurement or the like, a more accurate measurement can hence be performed than the use of the nucleic acid probe set designed such that the target nucleic acid binding region (b2) is located on the side of the 3' -end of the binding probe (B) and the nucleotide unit (a) labeled with the fluorescent substance is located on the side of the 5' -end of the fluorescent probe (A). The former design is preferred accordingly.

Although it is unknown for what reason such a difference in fluorescence quenching efficiency as described above arises by the difference in the position of the target nucleic acid binding region (b2), the present inventors presume that, when the target nucleic acid binding region (b2) is located on the side of the 3' -end of the binding probe (B), the fluorescent substance (d) labeled on the side of the 5' -end of the fluorescent probe (A) interacts, in an extension reaction of PCR, with DNA polymerase moved from the side of the 3' -end of the target nucleic acid and the quenching of the fluorescent substance (d) is interfered by the interaction.

The fluorescent probe (A) that constitutes the nucleic acid probe set according to the first aspect of the present invention is only needed to have a base sequence which can hybridize with the fluorescent probe binding region (b1) in the binding probe (B), and no particular limitation is imposed on its base length. However, a length of 4 bases or less may not be preferred in that it may lead to a lower hybridization efficiency, and a length of 51 bases or more may not be preferred either in that it tends to form non-specific hybrids when used in a real-time PCR measurement or the like. Therefore, the fluorescent probe (A) may be preferably 5 to 50 bases long, more preferably 10 to 35 bases long, especially preferably 10 to 20 bases long.

The base sequence of the fluorescent probe (A) may include one or more nucleotide units which are not complementary to the corresponding one or ones in the fluorescent probe binging region (b1), insofar as the fluorescent probe (A) can hybridize with the fluorescent probe binging region (b1) in the binding probe (B). Similarly, the base sequence of the fluorescent probe binding region (b1) in the binding probe (B) is not particularly limited insofar as the fluorescent probe binding region (b1) can hybridize with the fluorescent probe (A), and its base length depends on the base length of the fluorescent probe (A).

The target nucleic acid binding region (b2) in the binding probe (B) is needed to have a base sequence which can hybridize with the target nucleic acid (C). The base length of the target nucleic acid binding region (b2) depends on the base length of the target nucleic acid sequence (C). However, a length of 4 bases or less may not be preferred in that it may lead to a lower efficiency of hybridization with the target nucleic acid sequence (C), and a length of 61 bases or more may not be preferred either in that it leads to a reduction in yield upon synthesis of the binding probe (B) and also tends to form non-specific hybrids when used in a real-time PCR measurement or the like. Therefore, the target nucleic acid binding region (b2) may be preferably 5 to 60 bases long, more preferably 15 to 30 bases long. The target nucleic acid binding region (b2) may include a base sequence that forms no base pair with the target nucleic acid sequence (C), insofar as it can hybridize with the target nucleic acid sequence (C).

The nucleic acid probe set according to the first aspect of the present invention can be used in various analysis methods of nucleic acids. A description will hereinafter be made of an illustrative detection method of a target nucleic acid, which uses the nucleic acid probe set according to the present invention to determine whether or not the target nucleic acid exists in a solution.

A solution, which is to be detected for the target nucleic acid and will hereinafter be called "the detection sample", is first serially diluted to prepare several kinds of solutions. The nucleic acid probe set according to the first aspect of the present invention, in other words, the fluorescent probe (A) and binding probe (B) are added in constant amounts, respectively, to these serially-diluted detection samples. After the solutions are adjusted in temperature such that the thus-added nucleic acid probe complex in the first aspect of the present invention and the target nucleic acid can hybridize with each other, the solutions are measured for fluorescence intensity. The temperature, at which the probe complex in the present invention and the target nucleic acid are subjected to hybridization with each other, varies depending on the melting temperature (hereinafter called "Tm1") of the hybridized complex of the nucleic acid probe complex in the present invention and the target nucleic acid and other solution conditions. However, the hybridization temperature may be preferably in a temperature range where sequence-specific hybridization takes place between the nucleic acid probe complex and the target nucleic acid but non-specific hybridization does not occur between them, more preferably Tm1 to (Tm1 - 40)°C, still more preferably Tm1 to (Tm1 - 20) °C, even still more preferably Tm1 to (Tm1 - 10)°C. As one example of such a preferred temperature, about 60°C can be mentioned.

The melting temperature (hereinafter called "Tm2") of the complex of the fluorescent probe (A) and binding probe (B), which constitute the nucleic acid probe set according to the first aspect of the present invention, may be preferably higher than Tm1, with (Tm2 - Tm1) of 5°C or greater being more preferred, to assure the measurement of the fluorescence intensity. Compared with a case that the nucleotide units constituting the fluorescent probe (A) are all DNA units, the substitution of at least one nucleotide unit to a like number of LNA unit or units can raise the Tm2 by 2 to 6°C although this temperature rise also depends on the base length and base sequence. When the nucleic acid probe set according to the first aspect of the present invention is used in PCR, the adjustment of the proportion of LNA unit (s) in the oligonucleotide, which constitutes the fluorescent probe (A), such that the Tm2 becomes 95°C or higher can always bring the nucleic acid probe set into the form of a complex, and can use the nucleic acid probe set by considering it practically as a single-stranded nucleic acid probe. It is, therefore, possible to design the fluorescent probe (A) and target nucleic acid binding region (b2) without giving consideration to the above-described (Tm2 - Tm1).

When the target nucleic acid does not exist in the detection sample, a similar fluorescence intensity is observed from each of the serially-diluted detection samples. When the target nucleic acid exists in the detection sample, on the other hand, fluorescence from the fluorescent substance in the nucleic acid probe set according to the first aspect of the present invention is quenched by guanine in the nucleic acid which includes the target nucleic acid. The degree of this quenching is varied by changing the ratio of the nucleic acid probe set to the target nucleic acid in the solution. By adding the nucleic acid probe set according to the first aspect of the present invention to detection samples, which have been serially diluted as mentioned above, and measuring their fluorescence intensities, it is, therefore, possible to determine the existence/non-existence of the target nucleic acid from the occurrence/non-occurrence of a fluorescence quenching and also to quantify the existing amounts of the target nucleic acid from the magnitudes of the fluorescence quenching.

The nucleic acid probe set according to the first aspect of the present invention can also be used in a real-time PCR method. When quantification of an amplification product is desired by using the nucleic acid probe set according to the present invention in the real-time PCR method, a base sequence to be amplified by PCR or a portion thereof is chosen as a target nucleic acid, and the base sequence of the target nucleic acid bindingregion (b2) in the binding probe (B) isdetermined such that the target nucleic acid binding region (b2) can hybridize with the target nucleic acid.

The nucleic acid probe set according to the first aspect of the present invention, which has been prepared as described above, is added to a PCR reaction solution, a PCR reaction is conducted, and the fluorescence intensity is measured in each cycle of PCR. When the target nucleic acid in the reaction solution is amplified through the PCR reaction, the fluorescence from the fluorescent substance in the nucleic acid probe set according to the present invention is quenched by guanine in the target nucleic acid. The amplification product by PCR can, therefore, be quantified from the fluorescence intensity and the degree of the fluorescence quenching.

The nucleic acid probe set according to the first aspect of the present invention can also be used in an analysis of a nucleic acid for a base sequence polymorphism. Examples of analyzable base sequence polymorphisms include a single nucleotide polymorphism, base substitution, base deletion, base insertion and the like with respect to a base sequence as a reference. One example of such an analysis method will be described hereinafter.

In this analysis method, the target nucleic acid sequence (C) is used as a reference base sequence. A solution containing the target nucleic acid and another solution containing a nucleic acid to be analyzed are first prepared. After the nucleic acid probe set according to the first aspect of the present invention, that is, the binding probe (B), which has the target nucleic acid binding region (b2) designed to hybridize with the target nucleic acid sequence (C), and the fluorescent probe (A) are added to the respective solutions, the added nucleic acid probe complex in the first aspect of the present invention is subjected to hybridization with the target nucleic acid and the nucleic acid to be analyzed in the respective solutions, and the temperature dependences of fluorescence intensities are then measured. Described specifically, while changing the temperature of each solution from a low temperature to a high temperature, the fluorescence intensity is measured at each temperature.

A plot of the measurement results against temperature is called a "melting curve". By differentiating the melting curve of the solution, which contains the target nucleic acid, with respect to temperature, the Tm1 of the hybridized complex of the nucleic acid probe complex in the first aspect of the present invention and the target nucleic acid can be easily determined as a temperature that indicates an extreme value. Such a melting curve analysis can be performed by using a commercial program known well to those skilled in the art.

The fluorescence intensity of the solution, which contains the target nucleic acid, is reduced at a low temperature by the fluorescence quenching effect of guanine in the target nucleic acid. When the solution temperature is raised to around Tm1, however, the target nucleic acid dissociates from the nucleic acid probe complex in the first aspect of the present invention, the degree of fluorescence quenching decreases, and therefore, the fluorescence intensity suddenly increases. When there is, in the base sequence of the nucleic acid to be analyzed, a base sequence polymorphism, for example, a single nucleotide polymorphism, base substitution, base deletion, base insertion or the like with respect to the base sequence of the target nucleic acid, the Tm1 of the hybridized complex of the nucleic acid to be analyzed and the nucleic acid probe complex in the present invention indicates a value lower than the Tm1 of the hybridized complex of the target nucleic acid sequence and the nucleic acid probe complex in the present invention. By comparing the temperature dependence of the fluorescence intensity of the hybridized complex of the target nucleic acid and the nucleic acid probe complex in the first aspect of the present invention with the temperature dependence of the fluorescence intensity of the hybridized complex of the nucleic acid as the analysis target and the nucleic acid probe complex in the present invention, the nucleic acid as the analysis target can, therefore, be analyzed for a base sequence polymorphism with respect to the target nucleic acid sequence (C). As such an analytical procedure, their melting curves may be compared with each other. However, the existence or non-existence of a mutation can be readily determined by differentiating the respective melting curves with respect to temperature, determining the Tm1s as temperatures that give extreme values, and then comparing the Tm1s.

When a nucleotide unit having a guanine base that applies the quenching effect to the fluorescent substance in the fluorescent probe has mutated in the base sequence of the nucleic acid as an analysis target, no decrease occurs in fluorescence intensity by the fluorescence quenching effect at any temperature so that the mutation can be specified from the melting curve.

In a melting curve analysis, it has heretofore been needed to prepare fluorescently-labeled, costly nucleic acid probes of different base sequences specifically for individual target nucleic acids, and therefore, a substantial time has been needed for their synthesis. The use of the nucleic acid probe set according to the first aspect of the present invention in a melting curve analysis can obviate the preparation of fluorescently-labeled, costly nucleic acid probes specifically for individual target nucleic acids, and therefore, can reduce the preparation time for the melting curve analysis and can more economically perform the melting curve analysis.

The above-described nucleic acid probe set according to the first aspect of the present invention is consisted of the binding probe (B), which has the one fluorescent probe binding region (b1), and the one fluorescent probe (A). As an alternative, the nucleic acid probe set according to the first aspect of the present invention may have two fluorescent probe binding regions (b1) as shown in FIG. 5. These fluorescent probe binding regions (b1-1,b1-2) may have the same base sequence or different base sequences, although the different base sequences are preferred. In this case, the fluorescent probe may preferably have two fluorescent probes (A1,A2) of different base sequences.

The nucleic acid probe set according to the first aspect of the present invention, in which the binding probe (B) has the two fluorescent probe binding regions of different base sequences, can be suitably used as a replacement for a conventionally-used AB probe in an ABC-PCR (Alternately Binding Probe Competitive PCR; see Tani et al., Analytical Chemistry, Preprint) method.

As conventional, fluorescently-labeled AB probes for use in the above-described ABC-PCR method, different types of probes have to be used specifically for individual base sequences to be amplified. The use of nucleic acid probe set according to the first aspect of the present invention in place of the above-described AB probes can obviate the need to prepare different types of fluorescently-labeled, costly fluorescent probes specifically for individual base sequences to be amplified, and therefore, can perform the ABC-PCR method more economically.

Upon using the nucleic acid probe set according to the first aspect of the present invention, which has the two fluorescent probe binding regions (b1), as a replacement for an AB probe in the ABC-PCR method, the fluorescent substances that label the two fluorescent probes (A1,A2) may preferably be different kinds of fluorescent substances (d1,d2) which are different in both excitation wavelength and fluorescence wavelength. The combination of BODIPY-FL and TAMRA can be mentioned as a preferred example of the combination of the fluorescent substances (d1,d2).

<Second Aspect of the Present Invention>

The second aspect of the present invention relates to an oligonucleotide probe comprising nucleotide units including (a') a nucleotide unit labeled with (h) a labeling substance, a part or all of said nucleotide units being an artificial nucleotide unit or units having a function to raise a dissociation temperature of the oligonucleotide probe from a complementary strand, said dissociation temperature of the oligonucleotide probe from the complementary strand being 100°C or higher under normal pressure conditions.

As the artificial nucleotide unit or units having the function to raise the dissociation temperature, one or more artificial nucleotide units each selected from the group consisting of LNA, PNA, ENA, 2' ,4' -BNA$^{NC}$ and 2' ,4' -BNA$^{COC}$ units can be mentioned.

As the labeling substance (h) that labels the above-described oligonucleotide probe according to the present invention, a fluorescent substance, quencher substance, protein, functional group or the like can be mentioned, and depending on the analysis method, a desired labeling substance can be chosen by one skilled in the art. It is to be noted that the term "quencher substance" means a substance which has a function to weaken the fluorescence to be emitted by the fluorescence substance when the quencher substance is located near the fluorescence substance.

The oligonucleotide probe according to the present invention can always form a stable complex with a nucleotide (E), which has a sequence complementary to the probe, under normal pressure conditions in a water system, and therefore, can specifically hybridize with the nucleotide (E) to practically label the nucleotide with the labeling substance (h). Considering the complex of the probe and complementary strand as a single molecule, the complex can also be used in various analyses such as gene analyses.

By using, as the labeling substance (h), a fluorescent substance which changes in fluorescent character upon interaction with guanine, the probe according to the second aspect of the present invention can be used as the fluorescent probe (A) in the first aspect of the present invention.

<Third Aspect of the Present Invention>

Examples of the nucleic acid probe set according to the third aspect of the present invention are shown in FIGS. 12 to 14 and 16. In these figures, there are shown fluorescent probes (A), binding probes (B), target nucleic acid sequences (C), and a fluorescent substance (d).

The nucleic acid probe set according to the third aspect of the present invention comprises the one fluorescent probe (A) and the one binding probe (B). The binding probe (B) has one fluorescent probe binding region (b1), which has a base sequence complementary to the fluorescent probe (A), and one target nucleic acid binding region (b2), which has a base sequence complementary to the target nucleic acid sequence (C).

The fluorescent probe (A), which constitutes the nucleic acid probe set according to the third aspect of the present invention, is an oligonucleotide including a nucleotide unit (a) labeled with the fluorescent substance (d). No particular limitation is imposed on the base sequence of the fluorescent probe (A) insofar as it can hybridize with the fluorescent probe binding region (b1) in the binding probe (B). The base sequence of the fluorescent probe (A), therefore, does not depend on the base sequence of a target nucleic acid to be detected or analyzed. Accordingly, the fluorescent probe (A) that constitutes the nucleic acid probe set according to the third aspect of the present invention is not required to have a base sequence corresponding to the specific target nucleic acid, and the fluorescent probe (A) of the same base sequence can be commonly used for different target nucleic acids. The nucleic probe set according to the third aspect of the present invention is, therefore, called "a universal nucleic probe set" by the present inventors. The use of the nucleic acid probe set according to the third aspect of the present invention for the analysis of a target nucleic acid has an advantage in that it is no longer needed to prepare a fluorescent probe, which has a costly fluorescent substance, specifically for the target nucleic acid to be detected or analyzed and the production cost of the fluorescent probe can be minimized.

The nucleotide units as basic units of the fluorescent probe (A) are not limited to deoxyribonucleotides as basic units of DNA or ribonucleotides as basic units of RNA, and artificial nucleotide units, which have the above-described function to raise the dissociation temperature between the fluorescent probe (A) and the binding probe (B), can be also used. As examples of such artificial nucleotide units, LNA, PNA, ENA, 2' ,4'-BNA$^{NC}$ and 2' ,4' -BNA$^{COC}$ units can be mentioned.

An LNA monomer is a nucleotide having two ring structures that the 2'-oxygen and 4'-carbon atoms of ribose are connected together via a methylene unit. Due to the inclusion of these two ring structures, the LNA monomer has low structural freedom, and compared with DNA or RNA monomer, strongly hybridizes with a complementary strand. By increasing the proportion of the LNA monomer in the fluorescent probe (A), the Tm between the fluorescent probe (A) and the binding probe (B) can be easily made higher than the Tm between the target nucleic acid sequence (C) and the target nucleic acid binding region (b2), thereby making it possible to provide the nucleic acid probe set according to the third aspect of the present invention with increased stability at elevated temperatures and hence to provide it with improved reliability as a fluorescent probe.

By increasing the proportion of the artificial nucleotide unit or units in the fluorescent probe (A), the Tm between the fluorescent probe (A) and the binding probe (B) can be made higher than the thermal denaturation temperature (for example, 95°C) of PCR, so that the fluorescent probe (A) and the binding probe (B) can always remain as a stable nucleic acid probe complex during PCR cycles. The proportion of the artificial nucleotide unit or units in the fluorescent probe (A), said proportion being required to make the Tm between the fluorescent probe (A) and the binding probe (B) higher than the thermal denaturation temperature of PCR, also depends on the base number and base sequence of the fluorescent probe (A) and cannot be specified. Preferably, however, the proportion of the artificial nucleotide unit or units may be at least one third of the entire nucleotide units, with at least 80% thereof being more preferred.

By increasing the proportion of the artificial nucleotide unit or units in the fluorescent probe (A), the interaction between the fluorescent probe (A) and the fluorescent probe binding region (b1) is strengthened. The base numbers of the fluorescent probe (A) and fluorescent probe binding region (b1) can, therefore, be decreased compared with the case that the fluorescent probe (A) is formed of DNAunits alone. Upon synthesis of a binding probe (B) of a large base number, an error occurs. However, the use of a fluorescent probe binding region (b1) of a smaller base number can reduce the error, and can increase the synthesis yield of a binding probe (B). This leads to a reduction in the production cost for the probe set according to the third aspect of the present invention.

Usable as the fluorescent substance (d) with which the fluorescent probe (A) is labeled in the third aspect of the present invention is a fluorescent substance (d) which changes in fluorescent character upon interaction with guanine. In the third aspect of the present invention, the term "fluorescent character" means fluorescence intensity, the expression "guanine and the fluorescent substance interact with each other to change the fluorescent character of the fluorescent substance" means that the fluorescence intensity of the fluorescent substance in a state that guanine and the fluorescent substance are not interacting with each other is different from its fluorescence intensity in a state that they are interacting with each other, and on the extent of this difference, no limitation shall be imposed. Further, the term "quenched or quenching" of fluorescence means that upon interaction of a fluorescent substance with guanine, the fluorescence intensity decreases compared with the fluorescence intensity when the fluorescent substance is not interacting with guanine, and on the extent of this decrease, no limitation shall be imposed.

Examples of fluorescent substances, which can be suitably used in the nucleic acid probe set according to the third aspect of the present invention, include fluorescein and its derivatives [e.g., fluorescein-4-isothiocyanate (FITC), tetrachlorofluorescein, hexachlorofluorescein, tetrabromosulfonefluorescein (TBSF), and derivatives thereof], EDANS (5-(2-aminoethylamino)-1-naphthalenesulfonic acid), 6-JOE, Alexa Fluor 488 (Invitrogen Corp.), Alexa Fluor 532 (Invitrogen Corp.), Cy3 (GE Healthcare Bioscience), Cy5 (GE Healthcare Bioscience), Pacific Blue (Invitrogen Corp.), rhodamine 6G (R6G) and its derivatives (for example, carboxyrhodamine 6G (CR6G), tetramethylrhodamine (TMR), tetramethylrhodamine isothiocyanate (TMRITC), x-rhodamine, carboxytetramethylrhodamine (TAMRA)), Texas red (Invitrogen Corp.), BODIPY-FL (Invitrogen Corp.), BODIPY-FL/C3 (Invitrogen Corp.), BODIPY-FL/C6 (Invitrogen Corp.), BODIPY-5-FAM (Invitrogen Corp.), BODIPY-TMR (Invitrogen Corp.), BODIPY-TR (Invitrogen Corp.), BODIPY-R6G (Invitrogen Corp.), BODIPY564 (Invitrogen Corp.), and BODIPY581 (Invitrogen Corp.).

Of these, the use of fluorescein, fluorescein-4-isothiocyanate, tetrachlorofluorescein, hexachlorofluorescein, tetrabromosulfonefluorescein, EDANS, 6-JOE, Alexa Fluor 488, Alexa Fluor 532, Pacific Blue, rhodamine 6G, carboxyrhodamine 6G, tetramethylrhodamine, carboxytetramethylrhodamine or BODIPY-FL is more preferred, and the use of BODIPY-FL is most preferred.

The target nucleic acid binding region (b2) of the binding probe (B) is designed such that, when the nucleic acid probe complex in the third aspect of the present invention has hybridized to the target nucleic acid sequence (C), the fluorescent substance (d) and a guanine base in a target nucleic acid can be brought into contact with each other. As a consequence, upon hybridization of the nucleic acid probe complex in the third aspect of the present invention with the target nucleic acid sequence (C), the fluorescence of the fluorescent substance (d) is quenched by the guanine base, and by detecting this quenching phenomenon, the target nucleic acid can be quantified.

The guanine, which can interact with the fluorescent substance (d) to give fluorescence quenching effect, may exist either in the base sequence of the target nucleic acid sequence (C) or in a base sequence outside the target nucleic acid sequence (C), insofar as it exists in the target nucleic acid. When the guanine exists in the target nucleic acid sequence (C) and forms a base pair with cytosine in the hybridized binding probe (B), the interaction between the fluorescent substance (d) and the guanine is somewhat weaker although no particular problem arises. When the guanine forms no base pair for such a reason that the guanine exists outside the base sequence region of the target nucleic acid, on the other hand, the interaction between the fluorescent substance (d) and the guanine is facilitated. Accordingly, the latter situation is more preferred.

Referring next to FIG. 13, a description will be made about conditions under which the fluorescent substance (d) and a desired nucleotide unit (hereinafter called as "the nucleotide unit δ"), which has the guanine base in the target nucleic acid, can interact with each other when the nucleic acid probe complex in the third aspect of the present invention and the target nucleic acid sequence (C) have hybridized with each other. It is to be noted that the nucleotide unit, which has the guanine base at the 5' -end of the target nucleic acid sequence (C), is the nucleotide unit δ in the case of the description to be made hereinafter.

FIG. 13 shows, on an enlarged scale, an area around the fluorescent substance (d) in FIG. 12. The conditions under which the fluorescent substance (d) and the nucleotide unit δ can interact with each other depend *inter alia* on the length of a below-described spacer that connects the fluorescent substance (d) and the nucleotide unit (a) labeled with the fluorescent substance (d) each other, and cannot be specified. Generalizing these conditions, however, they can be defined as will be described hereinafter.

The fluorescent probe (A) is now assumed to have hybridized with the binding probe (B). Base pairs are formed between the fluorescent probe binding region (b1) and the fluorescent probe (A). A nucleotide unit, which exists in the fluorescent probe binding region (b1) and is closest to the target nucleic acid binding region (b2), will hereinafter be called "the nucleotide unit α". The distance between this nucleotide unit α and a base, which exists in the binding probe (B) and forms a base pair with the nucleotide unit (a), will be designated as "X" expressed in terms of the number of base (s) . It is to be noted that an adjacent nucleotide unit is counted as "X=1" and a nucleotide unit located adjacent with one base interposed therebetween is counted as "X=2".

In FIG. 13, the nucleotide unit a and the nucleotide unit, which exists in the binding probe (B) and forms a base pair with the nucleotide unit (a), are commonly a nucleotide unit having a thymine base located at the 5'-end in the fluorescent probe binding region (b1). Therefore, X is 0 (X=0).

On the other hand, base pairs are formed between the target nucleic acid sequence (C) and the target nucleic acid binding region (b2). A nucleotide unit, which exists in the target nucleic acid binding region (b2) and is closest to the nucleotide unit α, will be designated as "the nucleotide unit β". A nucleotide unit, which exists in the target nucleic acid sequence (C) and forms a base pair with the nucleotide unit β, will be designated as "the nucleotide unit γ". The distance between the nucleotide unit γ and the nucleotide unit δ will be designated as "Y" expressed in terms of the number of base (s). The counting method of Y is the same as X. In FIG. 13, the nucleotide unit γ and the nucleotide unit δ are commonly a nucleotide unit having a guanine base located at the 5' -end in the target nucleic acid sequence (C). Therefore, Y is 0.

As conditions for permitting interaction between the fluorescent substance (d) and the guanine in the nucleotide unit δ when the nucleic acid probe complex in the third aspect of the present invention and the target nucleic acid sequence (C) have hybridized with each other, the sum of X and Y may preferably be 5 or smaller. The sum of X and Y may be more preferably 3 or smaller, with 0 being most preferred, although it also depends on the length of the spacer connecting the fluorescent substance (d) and the nucleotide unit (a) labeled with the fluorescent substance (d).

Concerning the fluorescent probe (A) for use in the nucleic acid probe set according to the third aspect of the present invention, its production can rely upon a custom oligonucleotide synthesis service company (for example, Tsukuba Oligo Service Co., Ltd., Ibaraki, Japan) or the like. No particular limitation is imposed on the method for labeling the fluorescent substance on the oligonucleotide, and a conventionally-known labeling method can be used (Nature Biotechnology, 14, 303-308, 1996; Applied and Environmental Microbiology, 63, 1143-1147, 1997; Nucleic Acids Research, 24, 4532-4535, 1996).

When desired to couple a fluorescent substance, for example, to the 5'-terminal nucleotide unit, it is necessary to first introduce, for example, $-(CH_2)_n-SH$ as a spacer to a 5' -terminal phosphate group in a manner known per se in the art. As such a spacer, a commercial spacer can be used (for example, Midland Certified Reagent Company, U.S.A). In this case, n may stand for 3 to 8, with 6 being preferred. By coupling a fluorescent substance having SH reactivity or its derivative to the spacer, a fluorescently-labeled oligonucleotide can be obtained. The fluorescently-labeled oligonucleotide can be purified by reverse phase chromatography or the like to provide the fluorescent probe (A) for use in the present invention.

As an alternative, a fluorescent substance can also be coupled to the 3'-terminal nucleotide unit of the oligonucleotide. In this case, it is necessary to introduce, for example, $-(CH_2)_n-NH_2$ as a spacer to the OH group on the 3' -C atom of ribose or deoxyribose. As such a spacer, a commercial spacer can also be used (for example, Midland Certified Reagent Company, U.S.A). As an alternative method, it is also possible to introduce a phosphate group to the OH group on the 3'-C atom of ribose or deoxyribose and then to introduce, for example, $-(CH_2)_n-SH$ as a spacer to the OH group in the phosphate group. In this case, n may stand for 3 to 8, with 4 to 7 being preferred.

By coupling a fluorescent substance, which has reactivity to an amino group or SH group, or a derivative thereof to the above-described spacer, an oligonucleotide labeled with the fluorescent substance can be synthesized. The oligonucleotide can be purified by reverse phase chromatography or the like to provide the fluorescent probe (A) for use in the third aspect of the present invention. When desired to introduce $-(CH_2)_n-NH_2$ as a spacer, it is convenient to use a kit reagent (for example, Uni-link amino modifier,Clonetech Laboratories, Inc.). The fluorescent substance can then be coupled to the oligonucleotide in a manner known per se in the art.

The nucleotide unit (a) in the fluorescent probe (A), said nucleotide unit (a) being labeled with the fluorescent substance, is not limited to one of both terminal nucleotide units in the oligonucleotide, and a nucleotide unit other than both the terminal nucleotide units can be labeled with the fluorescent substance (ANALYTICAL BIOCHEMISTRY, 225, 32-38, 1998).

In the nucleic acid probe set according to the third aspect of the present invention, the target nucleic acid binding region (b2) of the binding probe (B) is designed such that it is located on the side of the 5'-end of the binding probe (B).

Upon designing a nucleic acid probe set from one fluorescent probe (A) and one binding probe (B), two ways can be considered, one being to design a target nucleic acid binding region (b2) on the side of the 5' -end of the binding probe (B) as illustrated in FIG. 14, and the other to design it on the side of the 3' -end of the binding probe (B) as illustrated in FIG. 15. For enabling a fluorescent substance (d) and a guanine base in a target nucleic acid to interact with each other in these cases, it is necessary to conduct the labeling such that a nucleotide unit (a) labeled with the fluorescent substance (d) is located on the side of the 3'-end of the fluorescent probe (A) when the target nucleic acid binding region (b2) is located on the side of the 5' -end of the binding probe (B) . When the target nucleic acid binding region (b2) is located on the side of the 3'-end of the binding probe (B), on the other hand, it is necessary to conduct the labeling such that the nucleotide unit (a) labeled with the fluorescent substance (d) is located on the side of the 5'-end of the fluorescent probe (A).

As a result of conducting many experiments and scrutinizing the fluorescence quenching efficiencies of such two types of nucleic acid probe sets as described above, the present inventors have found that a higher quenching efficiency is exhibited when designed such that the target nucleic acid binding region (b2) is located on the side of the 5' -end of the binding probe (B) and the nucleotide unit (a) labeled with the fluorescent substance is located on the side of the 3' -end of the fluorescent probe (A) than when designed such that the target nucleic acid binding region (b2) is located on the side of the 3' -end of the binding probe (B) and the nucleotide unit (a) labeled with the fluorescent substance is located on the side of the 5' -end of the fluorescent probe (A). When the nucleic acid probe set according to the third aspect of the present invention is used in real-time PCR, a more accurate measurement can be performed than the use of the nucleic acid probe set designed such that the target nucleic acid binding region (b2) is located on the side of the 3' -end of the binding probe (B) and the nucleotide unit (a) labeled with the fluorescent substance is located on the side of the 5' -end of the fluorescent probe (A) .

Although it is unknown for what reason such a difference in fluorescence quenching efficiency as described above arises by the difference in the position of the target nucleic acid binding region (b2), the present inventors presume that, when the target nucleic acid binding region (b2) is located on the side of the 3'-end of the binding probe (B), the fluorescent substance (d) labeled on the side of the 5'-end of the fluorescent probe (A) interacts, in an extension reaction of PCR, with DNA polymerase moved from the side of the 3' -end of the target nucleic acid and the quenching of the fluorescent substance (d) is interfered by the interaction.

The fluorescent probe (A) that constitutes the nucleic acid probe set according to the third aspect of the present invention is only needed to have a base sequence which can hybridize with the fluorescent probe binding region (b1) in the binding probe (B), and no particular limitation is imposed on its base length. However, a length of 4 bases or less may not be preferred in that it may lead to a lower hybridization efficiency, and a length of 51 bases or more may not be preferred either in that it tends to form non-specific hybrids when used in a real-time PCR measurement or the like. Therefore, the fluorescent probe (A) may be preferably 5 to 50 bases long, more preferably 10 to 35 bases long, especially preferably 10 to 20 bases long.

The base sequence of the fluorescent probe (A) may include one or more nucleotide units which are not complementary to the corresponding one or ones in the fluorescent probe binging region (b1), insofar as the fluorescent probe (A) can hybridize with the fluorescent probe binging region (b1) in the binding probe (B). Similarly, the base sequence of the fluorescent probe binding region (b1) in the binding probe (B) is not particularly limited insofar as the fluorescent probe binding region (b1) can hybridize with the fluorescent probe (A), and its base length depends on the base length of the fluorescent probe (A).

The target nucleic acid binding region (b2) in the binding probe (B) is needed to have a base sequence which can hybridize with the target nucleic acid (C). The base length of the target nucleic acid binding region (b2) depends on the base length of the target nucleic acid sequence (C) . However, a length of 4 bases or less may not be preferred in that it may lead to a lower efficiency of hybridization with the target nucleic acid sequence (C), and a length of 61 bases or more may not be preferred either in that it leads to a reduction in yield upon synthesis of the binding probe (B) and also tends to form non-specific hybrids when used in a real-time PCR measurement or the like. Therefore, the target nucleic acid binding region (b2) may be preferably 5 to 60 bases long, more preferably 15 to 30 bases long. The target nucleic acid binding region (b2) may include a base sequence that forms no base pair with the target nucleic acid sequence (C), insofar as it can hybridize with the target nucleic acid sequence (C).

The nucleic acid probe set according to the third aspect of the present invention can be used in various analysis methods of nucleic acids. A description will hereinafter be made of an illustrative detection method of a target nucleic acid, which uses the nucleic acid probe set according to the third aspect of the present invention to determine whether or not the target nucleic acid exists in a solution.

A solution, which is to be detected for the target nucleic acid and will hereinafter be called "the detection sample", is first serially diluted to prepare several kinds of solutions. The nucleic acid probe set according to the third aspect of the present invention, in other words, the fluorescent probe (A) and binding probe (B) are added in constant amounts, respectively, to these serially-diluted detection samples. After the solutions are adjusted in temperature such that the thus-added nucleic acid probe complex in the present invention and the target nucleic acid can hybridize with each other, the solutions are measured for fluorescence intensity. The temperature, at which the probe complex in the present invention and the target nucleic acid are subjected to hybridization with each other, varies depending on the melting temperature (hereinafter called "Tm1") of the hybridized complex of the nucleic acid probe complex in the third aspect of the present invention and the target nucleic acid and other solution conditions. However, the hybridization temperature may be preferably in a temperature range where sequence-specific hybridization takes place between the nucleic acid probe complex and the target nucleic acid but non-specific hybridization does not occur between them, more preferably Tm1 to (Tm1- 40)°C, still more preferably Tm1 to (Tm1-20) °C, even still more preferably Tm1 to (Tm1 - 10 ) °C. As one example of such a preferred temperature, about 60°C can be mentioned.

The melting temperature (hereinafter called "Tm2") of the complex of the fluorescent probe (A) and binding probe (B), which constitute the nucleic acid probe set according to the present invention, may be preferably higher than Tm1, with (Tm2 - Tm1) of 5°C or greater being more preferred, to assure the measurement of the fluorescence intensity. Compared with a case that the nucleotide units constituting the fluorescent probe (A) are all DNA units, the substitution of at least one nucleotide unit to a like number of LNA unit or units can raise the Tm2 by 2 to 6°C although this temperature rise also depends on the base length and base sequence. When the nucleic acid probe set according to the third aspect of the present invention is used in PCR, the adjustment of the proportion of LNA unit(s) in the oligonucleotide, which constitutes the fluorescent probe (A), such that the Tm2 becomes 95°C or higher can always bring the nucleic acid probe set into the form of a complex, and can use the nucleic acid probe set by considering it practically as a single-stranded nucleic acid probe. It is, therefore, possible to design the fluorescent probe (A) and target nucleic acid binding region (b2) without giving consideration to the above-described (Tm2 - Tm1).

When the target nucleic acid does not exist in the detection sample, a similar fluorescence intensity is observed from each of the serially-diluted detection samples. When the target nucleic acid exists in the detection sample, on the other hand, fluorescence from the fluorescent substance in the nucleic acid probe set according to the present invention is quenched by guanine in the nucleic acid which includes the target nucleic acid. The degree of this quenching is varied by changing the ratio of the nucleic acid probe set to the target nucleic acid in the solution. By adding the nucleic acid probe set according to the present invention to detection samples, which have been serially diluted as mentioned above, and measuring their fluorescence intensities, it is, therefore, possible to determine the existence/non-existence of the target nucleic acid from the occurrence/non-occurrence of a fluorescence quenching and also to quantify the existing amounts of the target nucleic acid from the magnitudes of the fluorescence quenching.

The nucleic acid probe set according to the third aspect of the present invention can also be used in a real-time PCR method. When quantification of an amplification product is desired by using the nucleic acid probe set according to the present invention in the real-time PCR method, a base sequence to be amplified by PCR or a portion thereof is chosen as a target nucleic acid, and the base sequence of the target nucleic acid binding region (b2) in the binding probe (B) is determined such that the target nucleic acid binding region (b2) can hybridize with the target nucleic acid.

The nucleic acid probe set according to the third aspect of the present invention, which has been prepared as described above, is added to a PCR reaction solution, a PCR reaction is conducted, and the fluorescence intensity is measured in each cycle of PCR. When the target nucleic acid in the reaction solution is amplified through the PCR reaction, the fluorescence from the fluorescent substance in the nucleic acid probe set according to the present invention is quenched by guanine in the target nucleic acid. The amplification product by PCR can, therefore, be quantified from the fluorescence intensity and the degree of the fluorescence quenching.

The nucleic acid probe set according to the third aspect of the present invention can also be used in an analysis of a nucleic acid for a base sequence polymorphism. Examples of analyzable base sequence polymorphisms include a single nucleotide polymorphism, base substitution, base deletion, base insertion and the like with respect to a base sequence as a reference. One example of such an analysis method will be described hereinafter.

In this analysis method, the target nucleic acid sequence (C) is used as a reference base sequence. A solution containing the target nucleic acid and another solution containing a nucleic acid to be analyzed are first prepared. After the nucleic acid probe set according to the third aspect of the present invention, that is, the binding probe (B), which has the target nucleic acid binding region (b2) designed to hybridize with the target nucleic acid sequence (C), and the fluorescent probe (A) are added to the respective solutions, the added nucleic acid probe complex in the present invention is subjected to hybridization with the target nucleic acid and the nucleic acid to be analyzed in the respective solutions, and the temperature dependences of fluorescence intensities are then measured. Described specifically, while changing the temperature of each solution from a low temperature to a high temperature, the fluorescence intensity is measured at each temperature.

A plot of the measurement results against temperature is called a "melting curve". By differentiating the melting curve of the solution, which contains the target nucleic acid, with respect to temperature, the Tm1 of the hybridized complex of the nucleic acid probe complex in the present invention and the target nucleic acid can be easily determined as a temperature that indicates an extreme value. Such a melting curve analysis can be performed by using a commercial program known well to those skilled in the art.

The fluorescence intensity of the solution, which contains the target nucleic acid, is reduced at a low temperature by the fluorescence quenching effect of guanine in the target nucleic acid. When the solution temperature is raised to around Tm1, however, the target nucleic acid dissociates from the nucleic acid probe complex in the third aspect of the present invention, the degree of fluorescence quenching decreases, and therefore, the fluorescence intensity suddenly increases. When there is, in the base sequence of the nucleic acid to be analyzed, a base sequence polymorphism, for example, a single nucleotide polymorphism, base substitution, base deletion, base insertion or the like with respect to the base sequence of the target nucleic acid, the Tm1 of the hybridized complex of the nucleic acid to be analyzed and the nucleic acid probe complex in the third aspect of the present invention indicates a value lower than the Tm1 of the hybridized complex of the target nucleic acid sequence and the nucleic acid probe complex in the third aspect of the present invention. By comparing the temperature dependence of the fluorescence intensity of the hybridized complex of the target nucleic acid and the nucleic acid probe complex in the present invention with the temperature dependence of the fluorescence intensity of the hybridized complex of the nucleic acid as the analysis target and the nucleic acid probe complex in the present invention, the nucleic acid as the analysis target can, therefore, be analyzed for a base sequence polymorphism with respect to the target nucleic acid sequence (C) . As such an analytical procedure, their melting curves may be compared with each other. However, the existence or non-existence of a mutation can be readily determined by differentiating the respective melting curves with respect to temperature, determining the Tm1s as temperatures that give extreme values, and then comparing the Tm1s.

When a nucleotide unit having a guanine base that applies the quenching effect to the fluorescent substance in the fluorescent probe has mutated in the base sequence of the nucleic acid as an analysis target, no decrease occurs in fluorescence intensity by the fluorescence quenching effect at any temperature so that the mutation can be specified from the melting curve.

In a melting curve analysis, it has heretofore been needed to prepare fluorescently-labeled, costly nucleic acid probes of different base sequences specifically for individual target nucleic acids, and therefore, a substantial time has been needed for their synthesis. The use of the nucleic acid probe set according to the third aspect of the present invention in a melting curve analysis can obviate the preparation of fluorescently-labeled, costly nucleic acid probes specifically for individual target nucleic acids, and therefore, can reduce the preparation time for the melting curve analysis and can more economically perform the melting curve analysis.

## Examples

The present invention will next be described more specifically based on examples. However, the following examples are merely illustrative of the present invention, and are not intended to be limiting the present invention.

<First Aspect of the Present Invention>

Example 1

Using a nucleic acid probe set according to the present invention for a portion of the human β-globin gene as a target nucleic acid sequence, a real-time PCR experiment was performed, and the effectiveness of the nucleic acid probe set according to the present invention was evaluated.

As reaction solutions for real-time PCR, a PCR reaction solution, which was free of a human genomic DNA sample (Human Genomic DNA; Novagen Inc.), and PCR reaction solutions, which contained $10^2$, $10^3$, $10^4$, $10^5$, $10^6$ and $10^7$ copies of the human genomic DNA sample, respectively, were prepared. Each reaction solution contained TITANIUM Taq DNA polymerase (Clonetech Laboratories, Inc.) as DNA polymerase, four types of dNTPs (final concentration: 0.2 mM, each), a forward primer (SEQ ID NO: 1, final concentration: 1 μM), a reverse primer (SEQ ID NO: 2, final concentration: 0.3 μM), a predetermined amount of TITANIUM Taq PCR buffer (Clonetech Laboratories, Inc.), and a nucleic acid probe set according to the present invention. By the PCR reaction, a nucleic acid containing the above-described target nucleic acid sequence was to be amplified.
SEQ ID NO:1 ggttggccaatctactccagg

SEQ ID NO:2 tggtctccttaaacctgtcttg

The nucleic acid probe set according to the present invention was for the portion of the human β-glonbin gene as a target nucleic acid sequence (SEQ ID NO:3), and was consisted of a binding probe (SEQ ID NO:4; final concentration: 100 nM) and a fluorescent probe (final concentration: 50 nM). The binding probe had, on the side of a 3'-end thereof, a fluorescent probe binding region of a base sequence complementary to the fluorescent probe, and on the side of a 5'-end thereof, a target nucleic acid binding region of a base sequence complementary to the target nucleic acid sequence. The fluorescent probe had a base sequence (SEQ ID NO:5), and was labeled at a 3' -terminal nucleotide unit thereof with BODIPY-FL (Invitrogen Corp.). It is to be noted that as all the nucleotide units making up the fluorescent probe, units of LNA (ThermoElectron Measurement Systems, Inc.) were used.

SEQ ID NO:3 gttcactagcaacctcaaacagacacc

SEQ ID NO:4 ggtgtctgtttgaggttgctagtgaactatgaggtggtaggatgggtagtg gt

SEQ ID NO:5 accactacccatcctaccacctcata-BODIPY-FL

FIG. 6 shows a schematic diagram of the above-described nucleic acid probe complex in the present invention upon hybridization with the target nucleic acid sequence (SEQ ID NO: 3). The fluorescent substance coupled to the fluorescent probe is considered to receive the fluorescence quenching effect from the guanine at the 5' -end of the target nucleic acid sequence.

In the above-described nucleic acid probe set according to the present invention, the binding probe had a phosphate group at the 3'-end thereof. It is to be noted that, when an LNA-containing fluorescent probe is used as in this example, the above-described phosphorylation at the 3' -end is not essential because the fluorescent probe does not dissociate from its associated binding probe under normal reaction conditions and the binding probe does not function as a primer. Synthesis of the nucleic acid probe was relied upon Tsukuba Oligo Service Co., Ltd. (Tsukuba, Japan), and syntheses of the forward primer and reverse primer were relied upon Nihon Gene Research Laboratories, Inc. (Sendai, Japan).

Using a real-time PCR system (LightCycler® 1.5, Roche Diagnostics K.K.), the reaction solutions were subjected to the following PCR reaction.

(1) Thermal denaturation step: 95°C, 120 seconds
(2) Thermal denaturation step: 95°C, 30 seconds
(3) Annealing step: 55°C, 30 seconds
(4) Extension step: 72°C, 30 seconds

After the thermal reaction step (1), the steps (2) to (4) were repeated 50 cycles. In each of the thermal denaturation step (2) and annealing step (3), the fluorescence intensity was measured. It is to be noted that the excitation wavelength was set at 450 to 495 nm and the detection wavelength was set at 505 to 537 nm.

The resulting fluorescence intensities were introduced into the following equation (1) to determine the fluorescence quenching efficiencies with respect to the six kinds of reaction solutions that contained the target nucleic acid.

$$\text{Fluorescence quenching efficiency} = [(G_{U,55}/G_{U,95}) - (G_{55}/G_{95})]/[(G_{U,55}/G_{U,95})] \qquad (1)$$

where,

$G_{U,55}$: Fluorescence intensity of a reaction solution in a given cycle before occurrence of a nucleic acid amplification in the annealing step (3).

$G_{U,95}$: Fluorescence intensity of the reaction solution in the given cycle before occurrence of a nucleic acid amplification in the thermal denaturation step (2).

$G_{55}$ : Fluorescence intensity of the reaction solution in the annealing step (3).

$G_{95}$ : Fluorescence intensity of the reaction solution in the thermal denaturation step (2).

FIG. 7 shows a graph obtained by plotting the thus-determined fluorescence quenching efficiencies on a graph sheet on which PCR cycles were plotted along the abscissa. It is understood from the graph that a target nucleic acid can be accurately quantified by conducting real-time PCR while using the nucleic acid probe set according to the present invention. It is to be noted that the average of maximum values of fluorescence quenching efficiency with respect to the above-described six kinds of PCR samples was about 37%.

Example 2

An experiment was performed in a similar manner as in Example 1 except that the five nucleotide units on the side of the 5'-end of the fluorescent probe were changed to a like number of DNA units. In this example, the average of maximum values of fluorescence quenching efficiency was about 35%. It is to be noted that the proportion of the LNA units in the fluorescent probe used in this example was about 81% of the entire units.

Comparative Example 1

A real-time PCR experiment was performed in a similar manner as in Example 1 except that DNA units were used as all the nucleotide units making up the fluorescent probe.
FIG. 8 shows a graph obtained by plotting the thus-determined fluorescence quenching efficiencies on a graph sheet on which PCR cycles were plotted along the abscissa. The average of maximum values of fluorescence quenching efficiency with respect to the six kinds of PCR samples was about 25%, and, compared with Example 1 in which all the nucleotide units making up the fluorescent probe were LNA units, the fluorescence quenching efficiency was about two thirds. In other words, the fluorescence quenching efficiency was improved by about 1.5 times by changing the nucleotide units, which constituted the fluorescent probe, from the DNA units to the LNA units.

Example 3

Using a nucleic acid probe set according to the present invention for a portion of the human β-actin gene as a target nucleic acid sequence (SEQ ID NO:8), a real-time PCR experiment was performed, and the effectiveness of the nucleic acid probe set according to the present invention was evaluated.

With respect to 7 kinds of samples which contained $10^2$, $10^3$, $10^4$, $10^5$, $10^6$, $10^7$ and $10^8$ copies, respectively, of an mRNA (Beta actin mRNA, Human; product of Nippon Gene Co., Ltd.) having the full-length sequence of the human β-actin gene, a cDNA was prepared with a reverse transcriptase (SuperScript III RT: Invitrogen Corp.) in a manner known *per se* in the art.

As reaction solutions for real-time PCR, reaction solutions, which contained the cDNA of the human β-actin gene as obtained from the seven kinds of samples, respectively, and a PCR reaction solution, which was free of the cDNA, were prepared. Each reaction solution was prepared following a manual provided in a kit (TITANIUM Taq PCR Kit, product of Takara Bio Inc.) except for the inclusion of a forward primer (SEQ ID NO: 6, final concentration: 0.3 μM) and a reverse primer (SEQ ID NO: 7, final concentration: 1.0 μM). By the PCR reaction, a 262-bp nucleic acid containing the above-described target nucleic acid sequence was to be amplified.
SEQ ID NO:6 catgtacgttgctatccaggc
SEQ ID NO:7 ctccttaatgtcacgcacgat

The nucleic acid probe set according to the present invention was for a portion of the human β-actin gene as a target nucleic acid (SEQ ID NO: 8), and was consisted of a binding probe (SEQ ID NO: 9; final concentration: 100 nM) and a fluorescent probe (final concentration: 50 nM). The binding probe had, on the side of a 3' -end thereof, a fluorescent probe binding region of a base sequence complementary to the fluorescent probe, and on the side of a 5' -end thereof, a target nucleic acid binding region of a base sequence complementary to the target nucleic acid. The fluorescent probe had a base sequence (SEQ ID NO: 10), and was labeled at a 3' -terminal nucleotide unit thereof with BODIPY-FL (Invitrogen Corp.). It is to be noted that as all the nucleotide units making up the fluorescent probe, units of LNA (ThermoElectron Measurement Systems, Inc.) were used.
SEQ ID NO:8 gtgaggatcttcatgaggtagtcagtcag
SEQ ID NO:9 ctgactgactacctcatgaagatcctcactatgaggtggtaggatgggtag tggt
SEQ ID NO:10 accactacccatcctaccacctcata-BODIPY-FL

In the above-described nucleic acid probe set according to the present invention, the binding probe had a phosphate group at the 3' -end thereof. Synthesis of the nucleic acid probe was relied upon Tsukuba Oligo Service Co., Ltd. (Tsukuba, Japan), and syntheses of the forward primer and reverse primer were relied upon Nihon Gene Research Laboratories, Inc. (Sendai, Japan).

Using a real-time PCR system (LightCycler® 1.5, Roche Diagnostics K.K.), the reaction solutions were subjected to the following PCR reaction.

(1) Thermal denaturation step: 95°C, 120 seconds
(2) Thermal denaturation step: 95°C, 30 seconds
(3) Annealing step: 55°C, 30 seconds
(4) Extension step: 72°C, 30 seconds

After the thermal reaction step (1), the steps (2) to (4) were conducted 50 cycles. In each of the thermal denaturation step (2) and annealing step (3), the fluorescence intensity was measured. It is to be noted that the excitation wavelength was set at 450 to 495 nm and the detection wavelength was set at 505 to 537 nm.

The resulting fluorescence intensities were introduced into the above-described equation (1) to determine the fluorescence intensities with respect to the seven kinds of reaction solutions that contained the target nucleic acid. FIG. 9 shows a graph obtained by plotting the thus-determined fluorescence quenching efficiencies on a graph sheet on which PCR cycles were plotted along the abscissa. It is understood from the graph that a target nucleic acid can be accurately quantified by conducting real-time PCR while using the nucleic acid probe set according to the present invention. It is to be noted that the average of maximum values of fluorescence quenching efficiency with respect to the above-described seven kinds of PCR samples was about 32%.

Comparative Example 2

A real-time PCR experiment was performed in a similar manner as in Example 2 except that DNA units were used as all the nucleotide units making up the fluorescent probe.
FIG. 10 shows a graph obtained by plotting the thus-determined fluorescence quenching efficiencies on a graph sheet on which PCR cycles were plotted along the abscissa. The average of maximum values of fluorescence quenching efficiency with respect to the seven kinds of PCR samples was about 25%, and, compared with Example 2 in which all the nucleotide units making up the fluorescent probe were LNA units, the fluorescence quenching efficiency was about three quarters. In other words, the fluorescence quenching efficiency was improved by about 1.3 times by changing the nucleotide units, which constituted the fluorescent probe, from the DNA units to the LNA units.

<Second Aspect of the Present Invention>

Example 4

A solution was prepared by adding an oligonucleotide (SEQ ID NO: 11, final concentration: 50 nM), another oligonucleotide (SEQ ID NO:12, final concentration: 400 nM), KCl (final concentration: 50 mM), Tris-HCl (final concentration: 10 mM), and $MgCl_2$ (final concentration: 1.5 mM). The former oligonucleotide was formed of LNA units only and was labeled at a 3'-terminal nucleotide with BODIPY-FL (Invitrogen Corp.), while the latter was formed of only DNA units only. The solution was brought to a volume of 20 μL, and its pH was adjusted to 8.7 at room temperature.
SEQ ID NO:11 ccccctcccccaa-BODIPY-FL
SEQ ID NO:12 gggttgggggaggggg

The above-described reaction solution was subjected to a real-time PCR system (LightCycler®, Roche Diagnostics K.K.), and a melting curve analysis was performed. The results are shown in FIG. 11.

As no dissociation peak was observed in FIG. 11, it has become evident that, once the oligonucleotides (SEQ ID NO: 11 and SEQ ID NO:12) hybridize with each other, they do not dissociate even at 97°C and always form a stable complex in a water system under normal pressure.

<Third Aspect of the Present Invention>

Example 5

Using a nucleic acid probe set according to the present invention for a portion of the human β-globin gene as a target nucleic acid sequence, a real-time PCR experiment was performed, and the effectiveness of the nucleic acid probe set according to the present invention was evaluated.

As reaction solutions for real-time PCR, a PCR reaction solution, which was free of a human genomic DNA sample (Human Genomic DNA; Novagen Inc.), and PCR reaction solutions, which contained $10^2$, $10^3$, $10^4$, $10^5$, $10^6$ and $10^7$ copies of the human genomic DNA sample, respectively, were prepared. Each reaction solution contained TITANIUM Taq DNA polymerase (Clonetech Laboratories, Inc.) as DNA polymerase, four types of dNTPs (final concentration: 0.2 mM, each), a forward primer (SEQ ID NO: 13, final concentration: 1 μM), a reverse primer (SEQ ID NO: 14, final concentration: 0.3 μM), a predetermined amount of TITANIUM Taq PCR buffer (Clonetech Laboratories, Inc.), and a nucleic acid probe set according to the present invention. By the PCR reaction, a nucleic acid containing the above-described target nucleic acid sequence was to be amplified.
SEQ ID NO:13 ggttggccaatctactccagg
SEQ ID NO:14 tggtctccttaaacctgtcttg

The nucleic acid probe set according to the present invention was for the portion of the human β-glonbin gene as a target nucleic acid sequence (SEQ ID NO:15), and was consisted of a binding probe (SEQ ID NO:16; final concentration: 100 nM) and a fluorescent probe (final concentration: 50 nM) . The binding probe had, on the side of a 3' -end thereof, a fluorescent probe binding region of a base sequence complementary to the fluorescent probe, and on the side of a 5' -end thereof, a target nucleic acid binding region of a base sequence complementary to the target nucleic acid sequence. The fluorescent probe had a base sequence (SEQ ID NO:17), and was labeled at a 3' -terminal nucleotide unit thereof with BODIPY-FL (Invitrogen Corp.). It is to be noted that the nucleotide units which made up the fluorescent probe were all DNA units.

SEQ ID NO:15 gttcactagcaacctcaaacagacacc

SEQ ID NO:16 ggtgtctgtttgaggttgctagtgaactatgaggtggtaggatgggtagtg gt

SEQ ID NO:17 accactacccatcctaccacctcata-BODIPY-FL

FIG. 16 shows a schematic diagram of the above-described nucleic acid probe complex in the present invention upon hybridization with the target nucleic acid sequence (SEQ ID NO:15). The fluorescent substance coupled on the fluorescent probe is considered to receive the fluorescence quenching effect from the guanine at the 5' -end of the target nucleic acid sequence.

In the above-described nucleic acid probe set according to the present invention, the binding probe had a phosphate group at the 3' -end thereof. Synthesis of the nucleic acid probe was relied upon Tsukuba Oligo Service Co., Ltd. (Tsukuba, Japan), and syntheses of the forward primer and reverse primer were relied upon Nihon Gene Research Laboratories, Inc. (Sendai, Japan).

Using a real-time PCR system (LightCycler® 480, Roche Diagnostics K.K.), the reaction solutions were subjected to the following PCR reaction.

(1) Thermal denaturation step: 95°C, 120 seconds
(2) Thermal denaturation step: 95°C, 30 seconds
(3) Annealing step: 55°C, 30 seconds
(4) Extension step: 72°C, 30 seconds

After the thermal reaction step (1), the steps (2) to (4) were conducted 50 cycles. In each of the thermal denaturation step (2) and annealing step (3), the fluorescence intensity was measured. It is to be noted that the excitation wavelength was set at 450 to 495 nm and the detection wavelength was set at 505 to 537 nm.

The resulting fluorescence intensities were introduced into the following equation (2) to determine the fluorescence quenching efficiencies with respect to the six kinds of reaction solutions that contained the target nucleic acid.

$$\text{Fluorescence quenching efficiency} = [(G_{U,55}/G_{U,95}) - (G_{55}/G_{95})]/[(G_{U,55}/G_{U,95})] \qquad (2)$$

where,

$G_{U,55}$: Fluorescence intensity of a reaction solution in a given cycle before occurrence of a nucleic acid amplification in the annealing step (3).

$G_{U,95}$: Fluorescence intensity of the reaction solution in the given cycle before occurrence of a nucleic acid amplification in the thermal denaturation step (2).

$G_{55}$: Fluorescence intensity of the reaction solution in the annealing step (3).

$G_{95}$: Fluorescence intensity of the reaction solution in the thermal denaturation step (2).

FIG. 17 shows a graph obtained by plotting the thus-determined fluorescence quenching efficiencies on a graph sheet on which PCR cycles were plotted along the abscissa. It is understood from the graph that a target nucleic acid can be accurately quantified by conducting real-time PCR while using the nucleic acid probe set according to the present invention. It is to be noted that the average of maximum values of fluorescence quenching efficiency with respect to the above-described six kinds of PCR samples was about 25%.

Comparative Example 3

A real-time PCR experiment was performed in a similar manner as in Example 5 except that as a binding probe, an oligonucleotide (SEQ ID NO:18) having, on the side of a 3' -end thereof, a target nucleic acid binding region and, on the side of a 5' -end thereof, a fluorescent probe binding region was used, a fluorescent probe (SEQ ID NO:19) labeled at a 5'-terminal nucleotide unit thereof with BODIPY-FL (Invitrogen Corp.) was used, and as PCR reaction solutions, seven kinds of samples containing 10, $10^2$, $10^3$, $10^4$, $10^5$, $10^6$, $10^7$ and $10^8$ copies, respectively, of a human genomic DNA sample were used. It is to be noted that the binding probe had a phosphate group at the 3'-end thereof.
SEQ ID NO:18 cgatgcagcgagtggcggcgatgggtgtctgtttgaggttgctagtgaac
SEQ ID NO:19 BODIPY-FL-catcgccgccactcgctgcatcg

FIG. 18 shows a graph obtained by plotting the thus-determined fluorescence quenching efficiencies on a graph sheet on which PCR cycles were plotted along the abscissa. The average of maximum values of fluorescence quenching efficiency with respect to the six kinds of PCR samples was about 15%, and, compared with Example 5 which used the fluorescent probe according to the present invention having, on the side of the 5' -end thereof, the binding probe binding region and, on the side of the 3' -end thereof, the fluorescent probe binding region, the fluorescence quenching efficiency was about 60% or so. In other words, the fluorescence quenching efficiency was improved by about 1.7 times by changing the binding probe binding region from the side of the 5' -end to the side of the 3' -end.

<Second Aspect of the Present Invention>

Example 6

An oligonucleotide probe and an oligonucleotide (complementary strand) were synthesized. The oligonucleotide probe had a base sequence (SEQ ID NO : 20), was labeled at a 3' -end thereof with BODIPY-FL and was formed of LNA units only, while the oligonucleotide (complementary stand) had a base sequence (SEQ ID NO:21) and was formed of DNA units only. The complementary strand was designed such that upon hybridization with the probe, guanine is located near the fluorescent dye. Synthesis of the probe was relied upon Gene Design Inc. (Ibaraki, Osaka, Japan), and the labeling of the probe with BODIPY-FL and purification by HPLC were relied upon Tsukuba Oligo Service Co., Ltd. (Tsukuba, Ibaraki, Japan).
SEQ ID NO:20 CCCCCTCCCCCTT-BODIPY-FL
SEQ ID NO:21 gggaagggggaggggg

A reaction solution of the composition shown in Table 1 was prepared, and was subjected to a melting curve analysis by a real-time PCR system (LightCycler®, Roche Diagnostics K.K.). As a blank, a similar measurement was also conducted with respect to a sample similar to the reaction solution of Table 1 except that the complementary strand was not added. The results are shown in FIG. 19.
As a result, in the example in which the complementary strand was added, a lower fluorescence intensity was exhibited compared with the blank in which the complementary strand was not added, because by the formation of a double stand of the probe and complementary strand, the fluorescent dye with which the probe was labeled interacted with the guanine in the complementary strand and its fluorescence was thus quenched. From the foregoing, it has become evident that the oligonucleotide probe according to the second aspect of the present invention is suitably usable as a fluorescence quenching probe.

As no dissociation peak was observed on the melting curve, it has become evident that, once the above-described probe hybridizes with the complementary strand, they do not dissociate even at 100°C and always form a stable complex in a water system under normal pressure.
It is to be noted that the fluorescence intensity (-) in FIG. 19 can be of any unit.

Table 1

| Composition of Reaction Solution (total volume: 20 μL) | |
|---|---|
| Complementary strand | 400 nM |
| Oligonucleotide probe | 50 nM |
| KCl | 50 nM |
| Tris-HCl | 10 nM |
| $MgCl_2$ | 1.5 nM |
| pH | 8.7 |

Example 7

A melting curve analysis was performed in a similar manner as in Example 6 except that the LNA units in the oligonucleotide probe (SEQ ID NO: 20) were changed to PNA units. The results are shown in FIG. 19. Synthesis of the probe was relied upon Fasmac Co., Ltd. (Atsugi, Kanagawa, Japan).

Example 8

A melting curve analysis was performed in a similar manner as in Example 6 except that the LNA units in the oligonucleotide probe (SEQ ID NO: 20) were changed to ENA units. The results are shown in FIG. 19. Synthesis of the probe was relied upon Sigma-Genosys Japan K.K. (Ishikari, Hokkaido, Japan).

Example 9

A melting curve analysis was performed in a similar manner as in Example 6 except that the LNA units in the oligonucleotide probe (SEQ ID NO: 20) were changed to 2', 4'-$BNA^{NC}$ units. The results are shown in FIG. 19. Synthesis of the probe was relied upon BNA Inc. (Ibaraki, Osaka, Japan).

Example 10

A melting curve analysis was performed in a similar manner as in Example 6 except that the LNA units in the oligonucleotide probe (SEQ ID NO: 20) were changed to 2', 4' -$BNA^{coc}$ units. The results are shown in FIG. 19. Synthesis of the fluorescent probe was relied upon BNA Inc. (Ibaraki, Osaka, Japan).

From the results shown in FIG.19, it has become evident that the artificial nucleotides used in Examples 6-10 can be suitably employed in the oligonucleotide probe in the second aspect of the present invention. It has also become evident that these probes can also be suitably employed as the fluorescent probe in the nucleic acid probe set according to the first aspect of the present invention.

As no dissociation peak was observed on any of the melting curves, it has become evident that, once the probes used in Examples 6-10 hybridize with their corresponding complementary strands, respectively, they do not dissociate even at 100°C and always form stable complexes in a water system under normal pressure.

<First and Second Aspects of the Present Invention>

Example 11

Using the oligonucleotide probe, which was employed in Example 6, as the fluorescent probe (A) in the nucleic acid probe set according to the first aspect of the present invention, a real-time PCR experiment was performed, and with respect to the oligonucleotide probe according to the second aspect of the present invention, its effectiveness as the fluorescent probe (A) was evaluated.

Described specifically, a PCR reaction was conducted under similar conditions as in Example 1 except that a nucleic acid probe set consisting of the oligonucleotide probe, which was employed in Example 6 and was formed of the LNA units only, and a oligonucleotide (SEQ ID NO:22), which was formed of DNA units only, was used and the target nucleic acid (the human genomic DNA sample) was contained as many as 100 copies.

In the oligonucleotide (SEQ ID NO:22), the 13 bases on the side of the 3' -end were a fluorescent probe binding region (b1) while the side of the 5'-end was a target nucleic acid binding region (b2) that contained a portion of the human β-globin gene as a target nucleic acid sequence.

SEQ ID NO:22 ggtgtctgtttgaggttgctagtgaactatgaggaagggggaggggg

With respect to the fluorescence intensity in the final cycle ($50^{th}$ cycle), the fluorescence quenching efficiency was calculated in a similar manner as in Example 1. As a blank, a similar measurement was also conducted with respect to a system in which the target nucleic acid was not added. The results are shown in Table 2.

Examples 12 to 15

In a similar manner as in Example 11 except for the separate use of the fluorescent probes employed in Examples 7 to 10, the fluorescence quenching efficiencies were calculated. The results are shown in Table 2.

Table 2

| Fluorescence Quenching Efficiencies in 50th Cycle upon Use of Fluorescent Probes Formed of Artificial Nucleotide Units | | | |
|---|---|---|---|
| Ex. | Fluorescence quenching efficiency (%) | Fluorescence quenching efficiency of blank (%) | Nucleic acid making up fluorescent probe |
| 11 | 39 | 0 | LNA |
| 12 | 35 | 0 | PNA |
| 13 | 38 | 0 | ENA |
| 14 | 36 | 0 | 2', 4' -$BNA^{NC}$ |
| 15 | 41 | 0 | 2', 4' -$BNA^{coc}$ |

From the above results, fluorescence quenching was not confirmed with the blank in which the target nucleic acid did not exist, no matter whichever artificial nucleotide was employed in the nucleic probe. Where 100 copies of the target nucleic acid were contained, on the other hand, substantially the same level of fluorescence quenching efficiency was obtained no matter whichever artificial nucleotide was employed in the fluorescent probe.
From these results, it has become evident that the artificial nucleotides employed in Examples 11 to 15 can all be suitably employed in the nucleic acid probe set according to the first aspect of the present invention and also in gene analysis methods that use the probe set.

<Screening of Fluorescent Substance Usable in the Present Invention>

Example 16

Screening was performed for a fluorescent substance that can be used in the nucleic acid probe set according to the present invention. Described specifically, a PCR reaction was conducted under similar conditions as in Example 11 except that the oligonucleotide (SEQ ID NO:20) formed of the LNA units only was labeled at the 3' -end thereof with Pacific Blue (Invitrogen Corp.).
Synthesis of the oligonucleotide was relied upon Gene Design Inc. (Ibaraki, Osaka, Japan), and the labeling of the oligonucleotide with the fluorescent substance was relied upon Tsukuba Oligo Service Co., Ltd. (Tsukuba, Ibaraki, Japan).
Subsequent to the PCR reaction, the resulting reaction solution was diluted ten-fold with a PCR buffer (1X). Subsequently, the fluorescence intensity at 95°C and the fluorescence intensity at 55°C were measured by a fluorophotometer equipped with a constant-temperature system (LS50B, manufactured by PerkinElmer Co., Ltd.). The fluorescence intensity at 95°C was a fluorescence intensity upon dissociation, while the fluorescence intensity at 55°C was a fluorescence intensity upon hybridization.
The measured fluorescence intensities were introduced into the below-described equation (3) to determine the fluorescence quenching efficiencies. The results are shown in FIG. 4.
The excitation wavelength and fluorescence measurement wavelength for each dye were set at the corresponding values shown in Table 3. Further, the slit width was set at 5 nm for both excitation and fluorescence measurement.
$$\text{Fluorescence quenching efficiency} = [(G_{B,55}/G_{B,95})-(G_{100,55}/G_{100,95})]/(G_{B,55}/G_{B,95}) \quad (3)$$

where,

- $G_{B,55}$: Fluorescence intensity at 55°C from the blank (added amount of target nucleic acid: 0 copy)
- $G_{B,95}$: Fluorescence intensity at 95°C from the blank (added amount of target nucleic acid: 0 copy)
- $G_{100,55}$: Fluorescence intensity at 55°C at added amount of target nucleic acid: 100 copies
- $G_{100,95}$: Fluorescence intensity at 95°C at added amount of target nucleic acid: 100 copies

Table 3

| Excitation Wavelengths and Fluorescence Measurement Wavelengths for Respective Dyes | | |
|---|---|---|
| | Excitation wavelength (nm) | Fluorescence measurement wavelength (nm) |
| Pacific Blue | 400 | 460 |
| Alexa Fluor 488 | 480 | 520 |
| BODIPY-FL | 480 | 520 |
| Fluorescein | 480 | 520 |
| 6-JOE | 510 | 540 |
| Carboxyrhodamine 6G | 510 | 540 |
| Tetramethylrhodamine | 540 | 590 |
| Cy5 | 630 | 690 |

Examples 17 to 23

Fluorescence quenching efficiencies were measured in a similar manner as in Example 16 except for the use of Alexa Fluor 488 (Invitrogen Corp.), BODIPY-FL (Invitrogen Corp.), fluorescein, 6-JOE, carboxyrhodamine 6G (CR6G), tetramethylrhodamine (TMR) and Cy5 (GE Healthcare Bioscience) as fluorescent substances. The results are shown in Table 4.

The measured fluorescence quenching efficiencies are shown below in Table 4. Except for Alexa Fluor 488 and Cy5, fluorescence quenching efficiencies of 15% and higher were confirmed. Especially with Pacific Blue, BODIPY-FL and carboxyrhodamine 6G (CR6G), significant fluorescence quenching as high as about 40% was confirmed. From the foregoing results, it has been indicated that Pacific Blue, BODIPY-FL, carboxyrhodamine 6G (CR6G), fluorescein, 6-JOE and tetramethylrhodamine (TMR) are particularly useful as fluorescent substances for use in the present invention.

Table 4

| Maximum Fluorescence Quenching Efficiencies of Respective Fluorescence Substances | | |
|---|---|---|
| Example | Fluorescent substance | Fluorescence quenching efficiency (%) |
| 16 | Pacific Blue | 40 |
| 17 | Alexa Fluor 488 | 3 |
| 18 | BODIPY-FL | 37 |
| 19 | Fluorescein | 20 |
| 20 | 6-JOE | 15 |
| 21 | Carboxyrhodamine 6G (CR6G) | 36 |
| 22 | Tetramethylrhodamine (TMR) | 21 |
| 23 | Cy5 | 1 |

<Application Examples Making Use of the Present Invention>

The present invention can be applied to various gene analysis methods. A description will hereinafter be made based on examples.

Application Example 1

As an application example of the present invention, a nucleic acid probe set according to the present invention as shown in FIG. 20 can be mentioned. The nucleic acid probe set is consisted of a binding probe, a fluorescent probe (A) and an oligonucleotide (F) . The binding probe has a target nucleic acid binding region (b2), which is formed of a chimeric oligonucleotide of DNA/RNA units, and fluorescent probe binding regions 1, 2 (b1-1,b1-2), which have different sequences and are arranged at opposite ends of the target nucleic acid binding region (b2), respectively. The fluorescent probe (A) is labeled at an end thereof with a fluorescent substance, and can hybridize to the fluorescent probe binding region 1. The oligonucleotide (F) is labeled at an end thereof with a quencher substance, and can hybridize to the fluorescent probe binding region 2.

By introducing, into each of the fluorescent probe (A) and oligonucleotide (F), one or more artificial nucleotide units having the function to raise the dissociation temperature from the binding probe, the fluorescent probe (A) and oligonucleotide (F) are firmly bound to the binding probe so that these three molecules always move as a unitary complex. As the fluorescent substance and quencher substance are located very close to each other in this state, FRET (or direct energy transfer) occurs, and therefore, the fluorescence to be emitted from the fluorescent substance is reduced.

When the nucleic probe set and target nucleic acid have hybridized with each other, RNaseH which can recognize an RNA-DNA duplex and can cleave a strand at its RNA part is caused to act, whereby the RNaseH cleaves the binding probe at an RNA region (f) in the target nucleic acid binding region.

As a result, the FRET (or direct energy transfer) between the fluorescent substance and the quencher substance is eliminated so that the fluorescent substance emits fluorescence. By monitoring this change, the existence of the target nucleic acid can be detected.

Application Example 2

A description will be made about a method for detecting a target nucleic acid by using a nucleic acid probe set according to the present invention. As shown in FIG. 21, the nucleic acid probe set is consisted of a fluorescent probe (A) and a binding probe (B). The fluorescent probe (A) includes at least one artificial nucleotide, has a stem region (s1) of several bases at an end thereof, cytosine is contained as at least the outermost base of the stem region (s1), and the cytosine is labeled with a fluorescent substance. The binding probe (B) has, at an end thereof, a stem region (s2) complementary to the stem region (s1) of the fluorescent probe and, at an opposite end thereof, a fluorescent probe binding region (b1), and also has a target nucleic acid binding region (b2) between the stem region (s2) and the fluorescent probe binding region (b1).

Since the stem region (s1) of the fluorescent probe and the stem region (s2) of the binding probe have complementary base sequences as mentioned above, hybridization of the fluorescent probe and binding probe results in a secondary structure as shown in FIG. 22 and a stem structure is formed. At this time, a guanine base is located near the fluorescent substance so that the guanine and the fluorescent substance interact with each other, and the fluorescence is quenched accordingly.

When the target nucleic acid is added to a system in which the nucleic acid probe set is contained, the nucleic acid probe set and the target nucleic acid hybridize to each other, and the secondary structure of the nucleic acid probe set changes as shown in FIG. 23. As a result, the distance between the guanine and the fluorescent substance is widened, so that the fluorescence quenching is prevented to result in an increased fluorescence intensity. By monitoring this change, the existence of the target nucleic acid can be detected.

Application Example 3

This application example uses such a fluorescent probe (A), oligonucleotide (F) and binding probe (B) as shown in FIG. 24. The fluorescent probe (A) has one or more artificial nucleotide units. The oligonucleotide (F) has one or more artificial nucleotide units, and is labeled at an end thereof with a quencher substance. The binding probe (B) has, at ends thereof, base sequence regions (b1-1,b1-2) complementary to the fluorescent probe and oligonucleotide, respectively; base sequence regions located adjacent to the base sequence regions (b1-1,b1-2) to form a stem-loop structure; and also a target nucleic acid binding region (b2) between the latter base sequence regions.

When the fluorescent probe (A), oligonucleotide (F) and binding probe (B) are mixed together, these three molecules form such a complex as shown in FIG. 25. The double-stranded structures formed between the two probes, each of which contains the one or more artificial nucleotide units, and the base sequence regions (b1-1,b1-2), which are complementary to the probes, respectively, are very high in thermal stability, and therefore, these three molecules always exist as a unitary complex. As the fluorescent substance and quencher substance are very close to each other in this state, the fluorescence to be emitted from the fluorescent substance is reduced via FRET (or direct energy transfer).

When a target gene exists, the target nucleic acid binding region (b2) of the binding probe and a target nucleic acid hybridize with each other as shown in FIG. 26. At this time, the stem-loop structure of the nucleic acid probe complex is eliminated, and therefore, the distance between the fluorescent substance and the quencher substance is widened. As a result, it becomes possible for the fluorescent substance to emit fluorescence. By monitoring this change, the existence of the target nucleic acid can be detected.

Application Example 4

This application example uses such a fluorescent probe (A), oligonucleotide (F), first binding probe (B1) and second binding probe (B2) as shown in FIG. 27. The fluorescent probe (A) has one or more artificial nucleotide units. The oligonucleotide (F) has one or more artificial nucleotide units, and is labeled at an end thereof with a quencher substance. The first binding probe (B1) has a fluorescent probe binding region (b1-1) and a target nucleic acid binding region (b2-1). The second binding probe (B2) has a binding region (b2-1), to which the oligonucleotide (F) can be bound, and a target nucleic acid binding region (b2-2).
The target nucleic acid binding regions (b2-1,b2-2) are designed such that they can hybridize to adjacent parts of a target nucleic acid, respectively.
When the fluorescent probe (A), oligonucleotide (F) and first and second binding probes are mixed together, two complexes such as those shown in FIG. 28 are formed. The double-stranded structures formed between the two probes, each of which contains the one or more artificial nucleotide units, and the base sequence regions (b1-1,b1-2), which are complementary to the probes, respectively, are very high in thermal stability, and therefore, these complexes always exist as complexes in a water system. When the target nucleic acid does not exist, the fluorescent substance and quencher substance do not interact with each other so that the fluorescent substance emits strong fluorescence.
When the target nucleic acid exists, on the other hand, the two complexes hybridize with the target nucleic acid as shown in FIG. 29. As a result, the fluorescent substance and quencher substance are located adjacent to each other, and therefore, fluorescence quenching occurs via FRET (or direct energy transfer). By monitoring this change, the existence of the target nucleic acid can be detected.

Application Example 5

This application example uses a nucleic acid probe set consisted of a fluorescent probe (A) and a binding probe (B) as shown in FIG. 30. The fluorescent probe (A) is formed of one or more artificial nucleotide units and one or more DNA units, and is labeled at an end thereof with a fluorescent substance (d) and at an opposite end thereof with a quencher substance (q). The binding probe (B) has a fluorescent probe binding region (b1) and a target nucleic acid binding region (b2).
The fluorescent probe (A) has, at an end thereof, a target nucleic acid binding region (e), which can be brought adjacent to the target nucleic acid binding region (b2) of the binding probe to hybridize to the target nucleic acid.
When the fluorescent probe (A) and binding probe (B) are mixed together, such a nucleic acid probe complex as shown in FIG. 31 is formed. As the complex of the fluorescent probe, which has the one or more artificial nucleotide units, and the fluorescent probe binding region is very high in thermal stability, the complex always exists as a complex in a water system. When the target nucleic acid does not exist, the fluorescent substance and quencher substance, both of which are labeled on the fluorescent probe, come close to each other so that fluorescence quenching occurs via FRET (or direct energy transfer).
When the target nucleic acid exists, the complex hybridizes to the target nucleic acid. When simply hybridized together, however, the distance between the fluorescent substance and the quencher substance does not change much so that the fluorescence intensity does not change. When, in a state that the target nucleic acid exists, DNApolymerase (P) having 5' →3' exonuclease activity is added and a DNA synthesis reaction is then conducted, however, the fluorescent probe is hydrolyzed by the DNA polymerase, and as a result, the interaction between the fluorescent substance and the quencher substance is eliminated and the fluorescent substance emits fluorescence. By monitoring this change, the existence of the target nucleic acid can be detected.

<SNP Genotyping Experiment Making Use of Universal Nucleic Acid Probe Set>

Example 24

Using a nucleic acid probe set according to the present invention, an SNP genotyping experiment was performed on the β2-adrenergic receptor (ADRB2) gene (A/G) as a target.
Genomic DNA was extracted from buccal cells of a volunteer, and corresponding to the three genetic variants (homozygous A allele, homozygous G allele and heterozygous AG allele) of the ADRB2 gene, genomic DNAs were prepared, respectively.
With respect to the three genomic DNAs, PCR was conducted using a forward primer (SEQ ID NO:23) and a reverse primer (SEQ ID NO:24). From the respective genomic DNAs, the three genetic variants (homozygous A allele, homozygous G allele and heterozygous AG allele) of the ADRB2 gene were prepared.
SEQ ID NO:23 CATGTACGTTGCTATCCAGGC
SEQ ID NO:24 CTCCTTAATGTCACGCACGAT

The expected value of Tm of SEQ ID NO: 23 was 62.5°C, while the expected value of Tm of SEQ ID NO: 24 was 61.9°C.

With respect to the three genetic variants, samples containing the ADRB2 gene as much as $10^4$ copies were prepared, respectively. After those samples were separately subjected to 50-cycle PCR to fully amplify the three genetic variants, the samples were used in melting curve analyses. In the PCR, a forward primer (SEQ ID NO:25, Tm: 59.5°C) and a reverse primer (SEQ ID NO:26, Tm: 59.2°C) were used.

SEQ ID NO:25 CGCTGAATGAGGCTTCC

SEQ ID NO:26 CAGCACATTGCCAAACAC

To the amplified three genetic variants of the ADRB2 gene, a binding probe (SEQ ID NO:27) and a fluorescent probe (SEQ ID NO:28) were added, and the melting curve analyses were performed. The results are shown in FIG. 33.

In the figure, the homozygous (homozygous A allele) sample of the wild-type gene is indicated by "w", the homozygous (homozygous G allele) sample of the mutant-type gene is indicated by "m", and the heterozygous (heterozygous AG allele) sample of the wild-type and mutant-type gene is indicated by "w+m".

From these results, the wild-type homozygous (homozygous A allele), mutant-type homozygous (homozygous G allele) and heterozygous (heterozygous AG allele) SNP genotypes can be clearly discriminated from one another. Described specifically, the homozygote of the wild-type gene was 53.0°C in Tm, and therefore, was clearly distinguished from the homozygote of the mutant-type gene (Tm: 61.6°C). With respect to the heterozygote, on the other hand, two Tms were observed. From these results, it has become evident that the universal nucleic acid probe set according to the present invention can be also applied to SNP typing.

The binding probe (SEQ ID NO:27) employed in Example 24 was an oligonucleotide, which was formed of DNA units only and had, on the side of a 5' -end thereof, a target nucleic acid binding region having a sequence complementary to a portion of the ADRB2 gene and, on the side of a 3' -end thereof, a fluorescent probe binding region.

SEQ ID NO:27 CTTCCATTGGGTGCCAGCttgggggaggggg

In SEQ ID NO:27, the target nucleic acid binding region is shown in upper-case letters, while the fluorescent probe binding region is shown in lower-case letters. Cytosine, the 5th base as counted from the 5' -end, can form a complementary pair with an SNP site (the guanine in the homozygous G allele or heterozygous AG allele). The expected value of Tm of the target nucleic acid binding region was 63.0°C.

On the other hand, the fluorescent probe (SEQ ID NO: 28) was an oligonucleotide, which was formed of LNA units only and was labeled at the 3' -end thereof with BODIPY-FL. The expected value of Tm was 102°C.

SEQ ID NO:28 CCCCCTCCCCCAA-BODIPY-FL

The reaction solution for the melting curve analysis was 20 μL in total, and contained $10^4$ copies of the sample DNA, LC480 Genotyping Master (Roche Diagnostics K.K.), 0.25 mg/mL of BSA, 0.15 μM of the forward primer, 0.5 μM of the reverse primer, 0.15 μM of the fluorescent probe, 0.5 μM of the binding probe, and 0.1 unit of uracil DNA glycosylase (Roche Diagnostics K.K.). As a real-time PCR system, a LightCycler 480 (Roche Diagnostics K.K.) was used.

## Example 25

Using a nucleic acid probe set according to the present invention, an SNP genotyping experiment was performed on the β3-adrenergic receptor (ADRB3) gene (C/T) as a target.

Genomic DNA was extracted from buccal cells of a volunteer, and corresponding to the three genetic variants (homozygous C allele, homozygous T allele and heterozygous CT allele) of the ADRB3 gene, genomic DNAs were prepared, respectively.

With respect to the three genomic DNAs, PCR was conducted using a forward primer (SEQ ID NO:29) and a reverse primer (SEQ ID NO:30). From the respective genomic DNAs, the three genetic variants (homozygous C allele, homozygous T allele and heterozygous CT allele) of the ADRB3 gene were prepared.

SEQ ID NO:29 AGCTCTCTTGCCCCATG

SEQ ID NO:30 GCCAGCGAAGTCACGAA

The expected value of Tm of SEQ ID NO: 29 was 60.9°C, while the expected value of Tm of SEQ ID NO: 30 was 61 . 7°C.

With respect to each of the samples, the ADRB3 gene was amplified by a similar procedure as in Example 24 except for the use of a forward primer (SEQ ID NO:31, Tm: 60.5°C) and a reverse primer (SEQ ID NO:32, Tm: 60.7°C).

SEQ ID NO:31 TGGCCTCACGAGAACAG

SEQ ID NO:32 GAGTCCCATCACCAGGTC

Melting curve analyses were performed in a similar manner as in Example 24 except for the use of a binding probe (SEQ ID NO:33). The results are shown in FIG. 34.

In the figure, the homozygous (homozygous T allele) sample of the wild-type gene is indicated by "w", the homozygous (homozygous C allele) sample of the mutant-type gene is indicated by "m", and the heterozygous (heterozygous CT allele) sample of the wild-type and mutant-type gene is indicated by "w+m".

From these results, the homozygote (homozygous T allele) of the wild-type gene was 63.9°C in Tm, and therefore, was clearly distinguished from the homozygote (homozygous C allele) of the mutant-type gene (Tm: 70.2°C).

The binding probe (SEQ ID NO:33) employed in Example 25 was an oligonucleotide, which was formed of DNA units only and had, on the side of a 5' -end thereof, a target nucleic acid binding region having a sequence complementary to a portion of the ADRB3 gene and, on the side of a 3' -end thereof, a fluorescent probe binding region.
SEQ ID NO : 33 CCATCGCCCGGACTCCGAGACTCttgggggaggggg
In SEQ ID NO : 33, the target nucleic acid binding region is shown in upper-case letters, while the fluorescent probe binding region is shown in lower-case letters. Cytosine, the 9th base as counted from the 5' -end, can form a complementary pair with an SNP site (the cytosine in the homozygous C allele or heterozygous CT allele) in an antisense strand. The expected value of Tm of the target nucleic acid binding region was 71.8°C .

Example 26

Using a nucleic acid probe set according to the present invention, an SNP genotyping experiment was performed on the uncoupling protein (UCP1) gene (A/G) as a target.
Genomic DNA was extracted from buccal cells of a volunteer, and corresponding to the three genetic variants (homozygous A allele, homozygous G allele and heterozygous AG allele) of the UCP1 gene, genomic DNAs were prepared, respectively.
With respect to the three genomic DNAs, PCR was conducted using a forward primer (SEQ ID NO:34) and a reverse primer (SEQ ID NO:35). From the respective genomic DNAs, the three genetic variants (homozygous A allele, homozygous G allele and heterozygous AG allele) of the UCP1 gene were prepared.
SEQ ID NO:34 AGTGGTGGCTAATGAGAGAA
SEQ ID NO:35 AAGGAGTGGCAGCAAGT
The expected value of Tm of SEQ ID NO: 34 was 60.0°C, while the expected value of Tm of SEQ ID NO: 35 was 60. 7°C.
With respect to each of the samples, the UCP1 gene was amplified by a similar procedure as in Example 24 except for the use of a forward primer (SEQ ID NO:36, Tm: 60.8°C) and a reverse primer (SEQ ID NO:37, Tm: 58.6°C).
SEQ ID NO:36 TTCTTCTGTCATTTGCACATTTATCT
SEQ ID NO:37 AACTGACCCTTTATGACGTAG
Melting curve analyses were performed in a similar manner as in Example 24 except for the use of a binding probe (SEQ ID NO:38). The results are shown in FIG. 35.
In the figure, the homozygous (homozygous A allele) sample of the wild-type gene is indicated by "w", the homozygous (homozygous G allele) sample of the mutant-type gene is indicated by "m", and the heterozygous (heterozygous AG allele) sample of the wild-type and mutant-type gene is indicated by "w+m".
From these results, the homozygote (homozygous A allele) of the wild-type gene was 52.5°C in Tm, and therefore, was clearly distinguished from the homozygote (homozygous G allele) of the mutant-type gene (Tm: 60.2°C).
The binding probe (SEQ ID NO:38) employed in Example 26 was an oligonucleotide, which was formed of DNA units only and had, on the side of a 5' -end thereof, a target nucleic acid binding region having a sequence complementary to a portion of the UCP1 gene and, on the side of a 3' -end thereof, a fluorescent probe binding region.
SEQ ID NO:38 CACTCGATCAAACTGTGGTCttgggggaggggg
In SEQ ID NO : 38, the target nucleic acid binding region is shown in upper-case letters, while the fluorescent probe binding region is shown in lower-case letters. Cytosine, the 5th base as counted from the 5' -end, can form a complementary pair with an SNP site (the guanine in the homozygous G allele or heterozygous AG allele). The expected value of Tm of the target nucleic acid binding region was 59.9°C.
From the results of Examples 24 to 26, it has become evident that the universal nucleic acid probe set according to the present invention can be applied to SNP genotyping and can perform analyses at low cost and high speed.

## Industrial Applicability

According to the first aspect of the present invention, there is provided a nucleic acid probe set comprising a fluorescent probe and a binding probe and having an improved fluorescence quenching efficiency. The nucleic acid probe set according to the first aspect of the present invention exhibits a fluorescence quenching efficiency of a similar level as those of conventional, single-stranded nucleic acid probes. The nucleic acid probe set according to the first aspect of the present invention does not require preparing a fluorescently-labeled, costly nucleic acid probe specifically for every target nucleic acid to be analyzed, and therefore, has an advantage that a nucleic acid probe for a target nucleic acid can be prepared at low cost and in a short time compared with the conventional nucleic acid probes. The nucleic acid probe set according to the first aspect of the present invention can be applied to the detection, quantification and polymorphism analyses of nucleic acids, the detection of mutations, and the like in fields such as medical science, molecular biology and agricultural science.
According to the second aspect of the present invention, there can be provided an oligonucleotide capable of forming a stable complex that does not dissociate in a water system under normal pressure, and also a method for using the nucleic acid probe set.

According to the third aspect of the present invention, there is provided a nucleic acid probe set having an improved fluorescent quenching efficiency. The nucleic acid probe set according to the third aspect of the present invention does not require preparing a fluorescently-labeled, costly nucleic acid probe specifically for every target nucleic acid to be analyzed, and therefore, has an advantage that a nucleic acid probe for a target nucleic acid can be prepared at low cost and in a short time compared with the conventional nucleic acid probes. The nucleic acid probe set according to the third aspect of the present invention can be applied to the detection, quantification and polymorphism analyses of nucleic acids, the detection of mutations, and the like in fields such as medical science, molecular biology and agricultural science.

**Legend**

| | |
|---|---|
| A | Fluorescent probe |
| B | Binding probe |
| C | Target nucleic acid sequence |
| F | Oligonucleotide having one or more artificial nucleotide units and labeled at an end thereof with a quencher substance |
| P | DNA polymerase having 5' →3' exonuclease activity |
| T | Target nucleic acid |
| a | Nucleotide labeled with fluorescent substance |
| a' | Nucleotide labeled with labeling substance |
| b1 | Fluorescent probe binding region |
| b2 | Target nucleic acid binding region |
| d | Fluorescent substance |
| e | Target nucleic acid binding region |
| f | RNA region |
| h | Labeling substance |
| q | Quencher substance |
| s | Stem region |
| α | Nucleotide α |
| β | Nucleotide β |
| γ | Nucleotide γ |
| δ | Nucleotide δ |
| m | Melting curve of mutant-type homozygous allele |
| w | Melting curve of wild-type homozygous allele |
| w+m | Melting curve of wild-type and mutant-type heterozygous allele |

## Claims

1. A nucleic acid probe set comprising (A) a fluorescent probe, which is formed of an oligonucleotide including (a) a nucleotide unit labeled with (d) a fluorescent substance, and (B) a binding probe formed of an oligonucleotide having (b1) a fluorescent probe binding region, which can hybridize to the fluorescent probe (A), and (b2) a target nucleic acid binding region, which can hybridize to a target nucleic acid sequence (C), wherein the fluorescent substance (d) is a fluorescent substance which changes in fluorescent character upon interaction with guanine, and at least one of nucleotide units which constitute the fluorescent probe (A) is an artificial nucleotide unit having a function to raise a dissociation temperature between the probe (A) and the fluorescent probe binding region (b1).

2. The nucleic acid probe set according to claim 1, wherein the artificial nucleotide unit having the function to raise the dissociation temperature is at least one artificial nucleotide unit selected from the group consisting of LNA, PNA, ENA, 2', 4'-$BNA^{NC}$ and 2', 4' -$BNA^{COC}$ units.

3. The nucleic acid probe set according to claim 2, wherein at least one-third of the nucleotide units which constitute the fluorescent probe (A) are artificial nucleotide units.

4. The nucleic acid probe set according to claim 2, wherein at least 80% of the nucleotide units which constitute the fluorescent probe (A) are artificial nucleotide units.

5. The nucleic acid probe set according to claim 3, wherein the fluorescent substance (d) is any one selected from the group consisting of fluorescein, fluorescein-4-isothiocyanate, tetrachlorofluorescein, hexachlorofluorescein, tetrabromosulfonefluorescein, EDANS, 6-JOE, 3,6-diamino-9-[2,4-bis(lithiooxycarbonyl)phenyl]-4-(lithioxysulfonyl)-5-sulfonatoxanthylium/3,6-diamin o-9-[2,5-bis(lithiooxycarbonyl)phenyl]-4-(lithooxys ulfonyl)-5-sulfonatoxanthylium, [2,3,3,7,7,8-hexamethyl-5-[4-[5-(2,5-dioxo-3-pyrrol in-1-yl)pentylcarbamoyl]phenyl]-2,3,7,8-tetrahydro-9-azonia-1H-pyrano[3,2-f : 5,6-f'] diindole-10,12-disu lfonic acid 12-sodium] anion salt, 2-oxo-6,8-difluoro-7-hydroxy-2H-1-benzopyran-3-carb oxylic acid, rhodamine 6G, carboxyrhodamine 6G, tetramethylrhodamine, carboxytetramethylrhodamine and BODIPY-FL.

6. The nucleic acid probe set according to claim 3, wherein the target nucleic acid binding region (b2) is located on a side of a 5'-end of the binding probe (B), and the nucleotide unit (a) labeled with the fluorescent substance (d) is a 3'-terminal nucleotide unit of the fluorescent probe (A).

7. A method for detecting a target nucleic acid, which comprises the following steps (1) to (4):

   (1) hybridizing the nucleic acid probe set according to claim 3 and the target nucleic acid with each other,
   (2) then measuring the fluorescence intensity of a hybridized complex of the nucleic acid probe set and target nucleic acid,
   (3) conducting the steps (1) and (2) by changing a ratio of the nucleic acidprobe set to the target nucleic acid, and
   (4) comparing the fluorescence intensities obtained from the steps (2) and (3).

8. A method for analyzing a nucleic acid for a base sequence polymorphism, which comprises the following steps (1) to (4):

   (1) hybridizing the nucleic acid probe set according to claim 3 and a target nucleic acid with each other,
   (2) then measuring a temperature dependence of fluorescence intensity with respect to a hybridized complex of the nucleic acid probe set and target nucleic acid,
   (3) conducting the steps (1) and (2) by using another nucleic acid in place of the target nucleic acid, and
   (4) comparing the temperature dependences of fluorescence intensity as obtained from the steps (2) and (3).

9. A method, which comprises conducting a melting curve analysis on a complex of the nucleic acid probe set according to claim 3 and a target nucleic acid.

10. An oligonucleotide probe comprising nucleotide units including (a') a nucleotide unit labeled with (h) a labeling substance, a part or all of said nucleotide units being an artificial nucleotide unit or units having a function to raise a dissociation temperature of the oligonucleotide probe from a complementary strand, said dissociation temperature of the oligonucleotide probe from the complementary strand being 100°C or higher under normal pressure conditions.

**11.** The oligonucleotide probe according to claim 10, wherein the artificial nucleotide unit or units having the function to raise the dissociation temperature from the complementary strand is or are one or more artificial nucleotide units each selected from the group consisting of LNA, PNA, ENA, 2', 4'-$BNA^{NC}$ and 2', 4' -$BNA^{COC}$ units.

**12.** The oligonucleotide probe according to claim 10, wherein the labeling substance (h) is a fluorescent substance, quencher substance, protein or functional group.

**13.** A method, which comprises hybridizing the oligonucleotide probe according to claim 10 with (E) an oligonucleotide having a complementary base sequence to label the oligonucleotide (E) with the labeling substance (h).

**14.** The nucleic acid probe set according to claim 1, wherein the oligonucleotide probe according to claim 10, in which the labeling substance (h) is a fluorescent substance which changes in fluorescent character upon interaction with guanine, is used as a fluorescent probe (A).

**15.** A nucleic acid probe set comprising (A) one fluorescent probe, which is formed of an oligonucleotide including (a) a nucleotide unit labeled with (d) a fluorescent substance, and (B) one binding probe formed of an oligonucleotide having (b1) one fluorescent probe binding region, which can hybridize to the fluorescent probe (A), and (b2) one target nucleic acid binding region, which can hybridize to a target nucleic acid sequence (C), wherein the fluorescent substance (d) is a fluorescent substance which changes in fluorescent character upon interaction with guanine, the nucleotide unit (a) is a 3'-terminal nucleotide unit of the fluorescent probe (A), and the target nucleic acid binding region (b2) is located on a side of a 5' -end of the binding probe (B).

Fig. 1

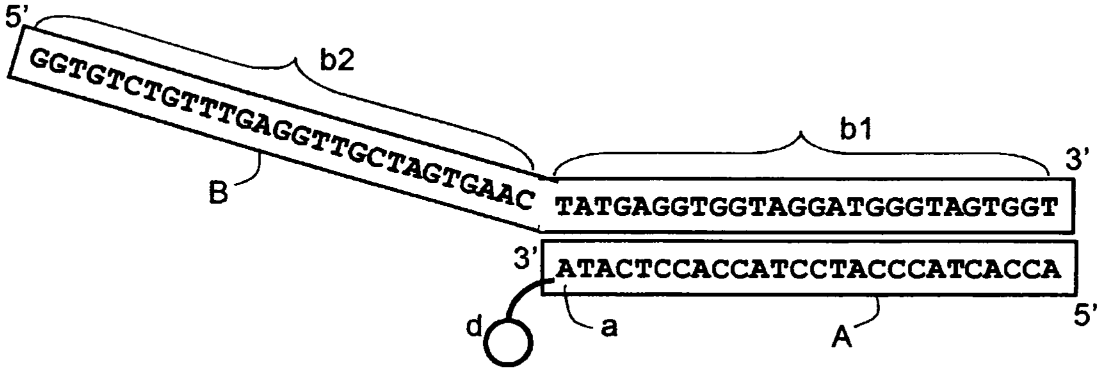
5'
GGTGTCTGTTTGAGGTTGCTAGTGAAC
b2
b1
3'
B
TATGAGGTGGTAGGATGGGTAGTGGT
3'
ATACTCCACCATCCTACCCATCACCA
d
a
A
5'

Fig. 2

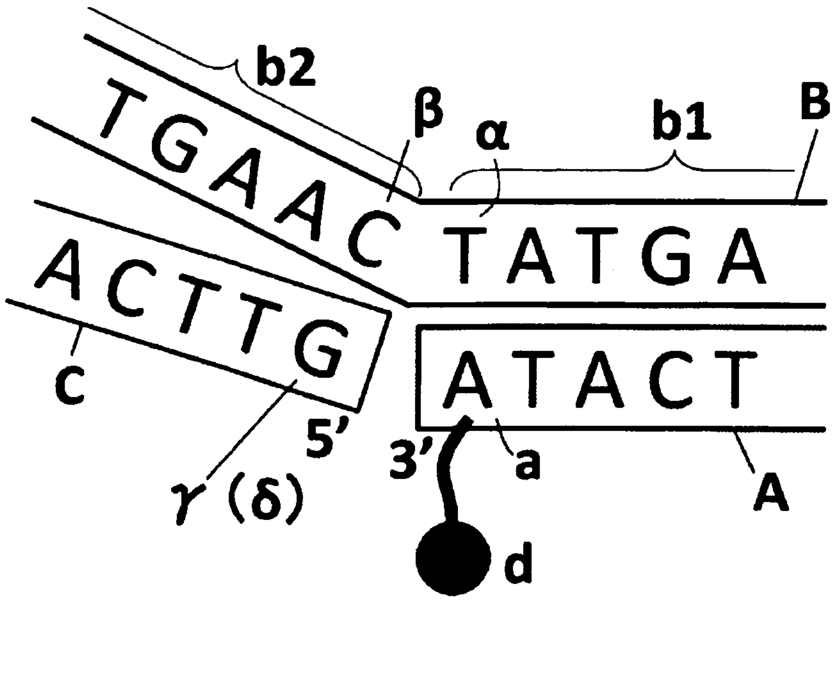
b2
β
α
b1
B
TGAAC
TATGA
ACTTG
ATACT
C
5'
3'
a
A
γ (δ)
d

Fig. 3

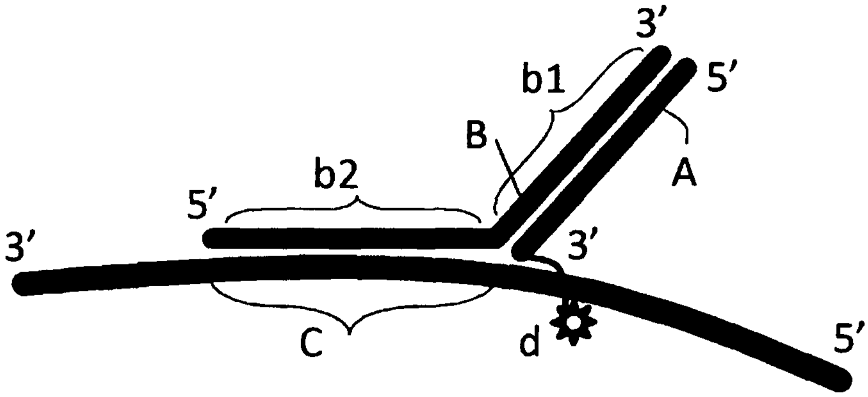


Fig. 4

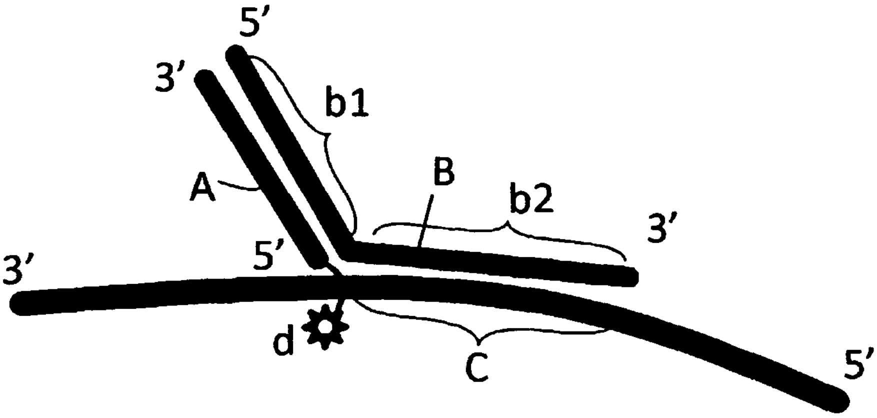

Fig. 5

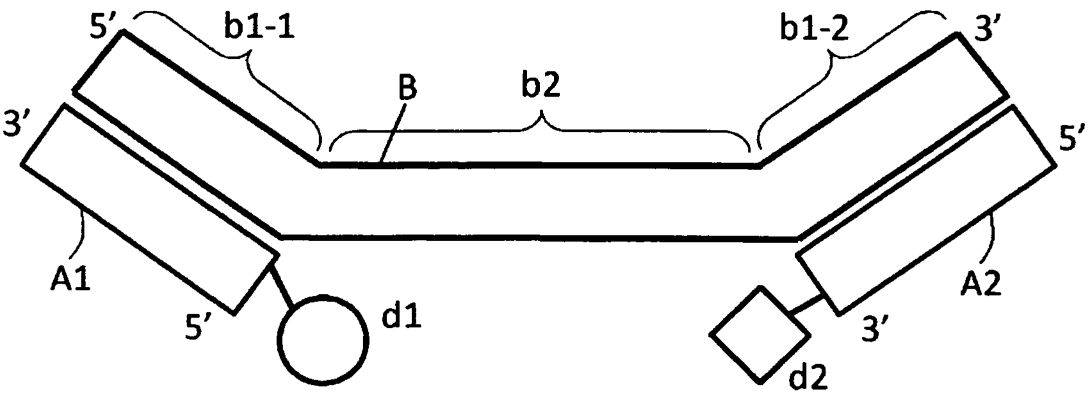
5'
b1-1
B
b2
b1-2
3'
3'
5'
A1
5'
d1
d2
3'
A2

Fig. 6

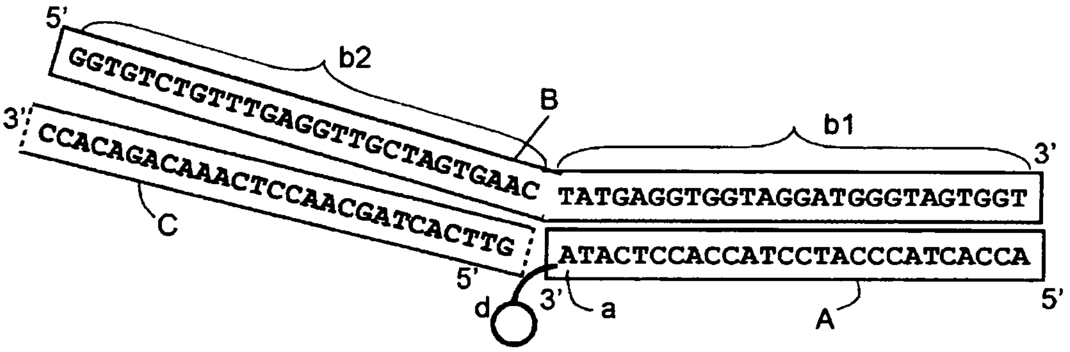
5'
GGTGTCTGTTTGAGGTTGCTAGTGAAC
b2
B
b1
3'
3'
CCACAGACAAACTCCAACGATCACTTG
TATGAGGTGGTAGGATGGGTAGTGGT
ATACTCCACCATCCTACCCATCACCA
C
5'
d
3'
a
A
5'

Fig. 7

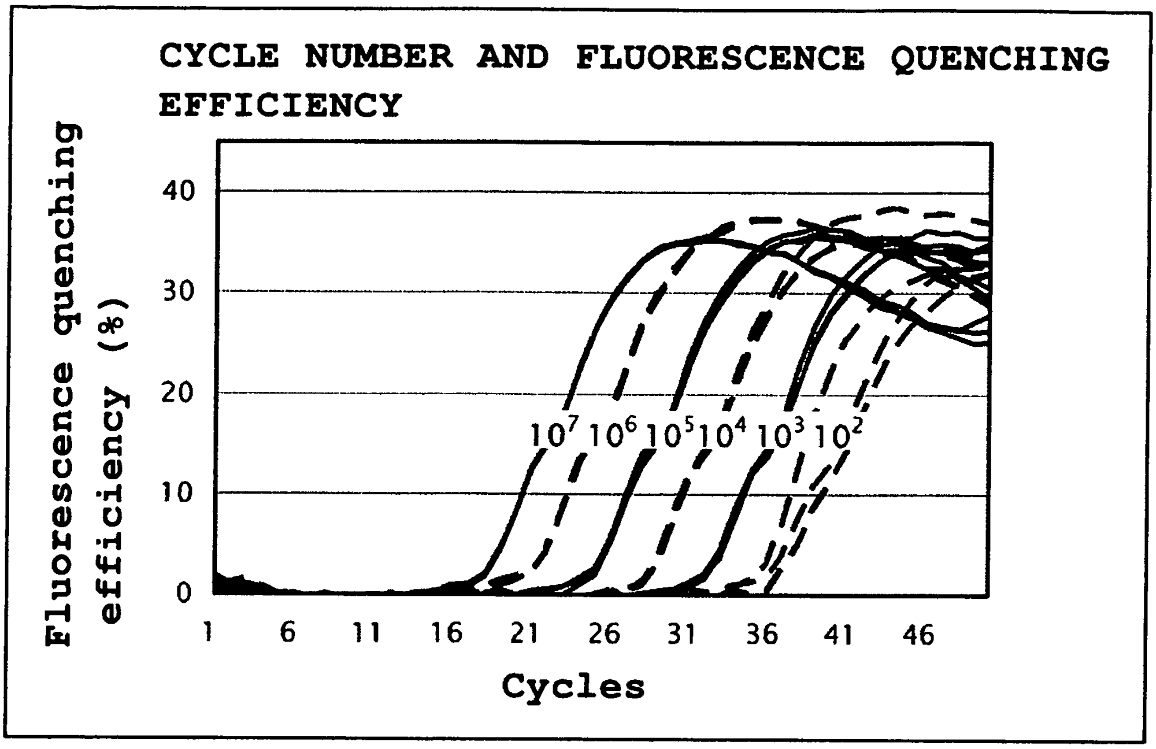
CYCLE NUMBER AND FLUORESCENCE QUENCHING EFFICIENCY
Fluorescence quenching efficiency (%)
40
30
20
10
0
10^7 10^6 10^5 10^4 10^3 10^2
1 6 11 16 21 26 31 36 41 46
Cycles

Fig. 8

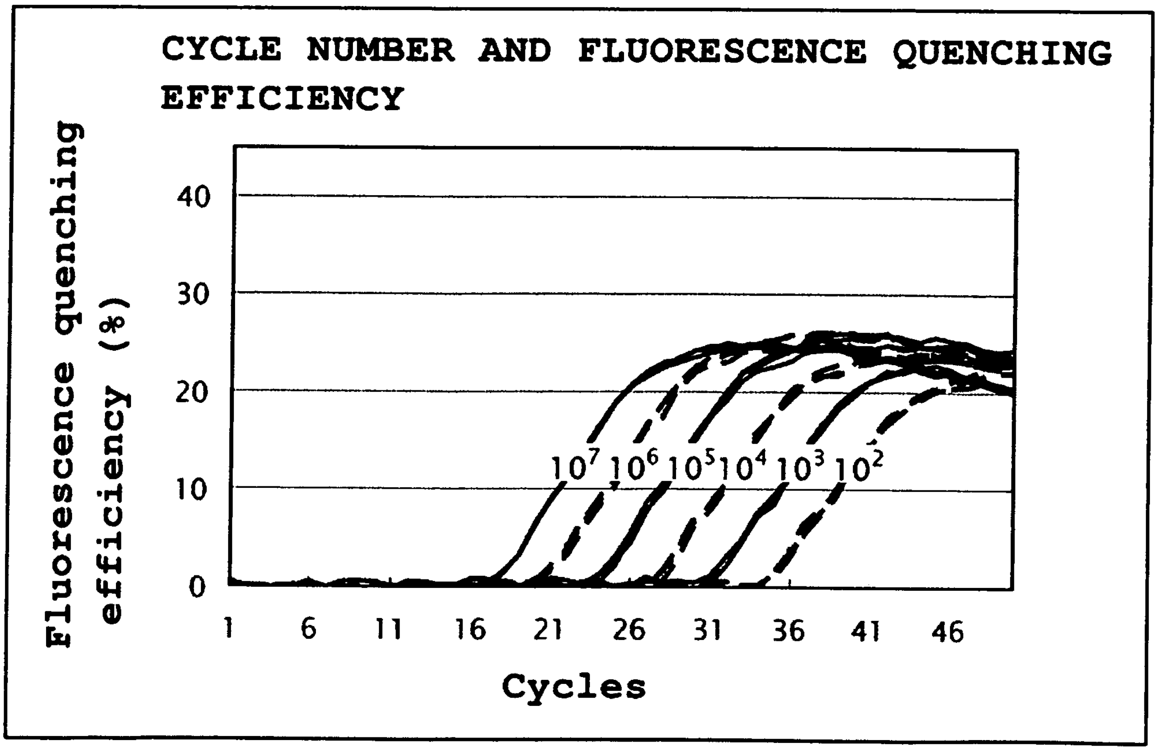
CYCLE NUMBER AND FLUORESCENCE QUENCHING EFFICIENCY
Fluorescence quenching efficiency (%)
40
30
20
10
0
10^7 10^6 10^5 10^4 10^3 10^2
1 6 11 16 21 26 31 36 41 46
Cycles

Fig. 9

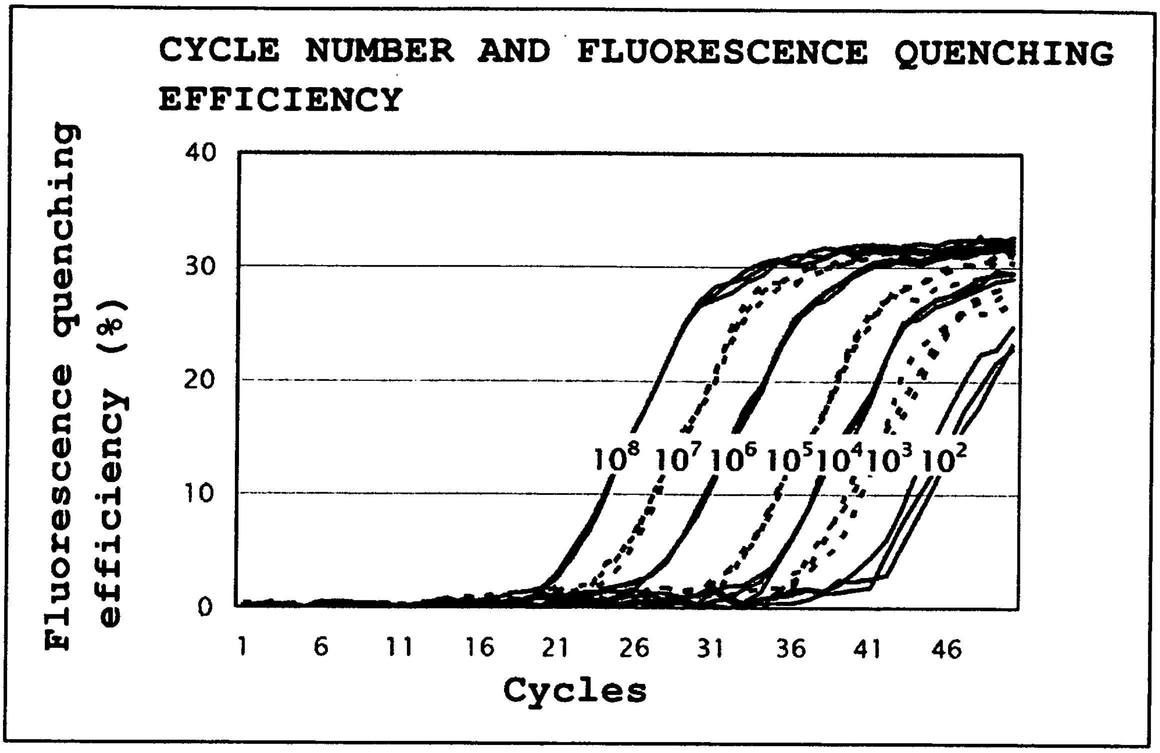
CYCLE NUMBER AND FLUORESCENCE QUENCHING EFFICIENCY
Fluorescence quenching efficiency (%)
40
30
20
10
0
$10^8$ $10^7$ $10^6$ $10^5$ $10^4$ $10^3$ $10^2$
1 6 11 16 21 26 31 36 41 46
Cycles

Fig. 10

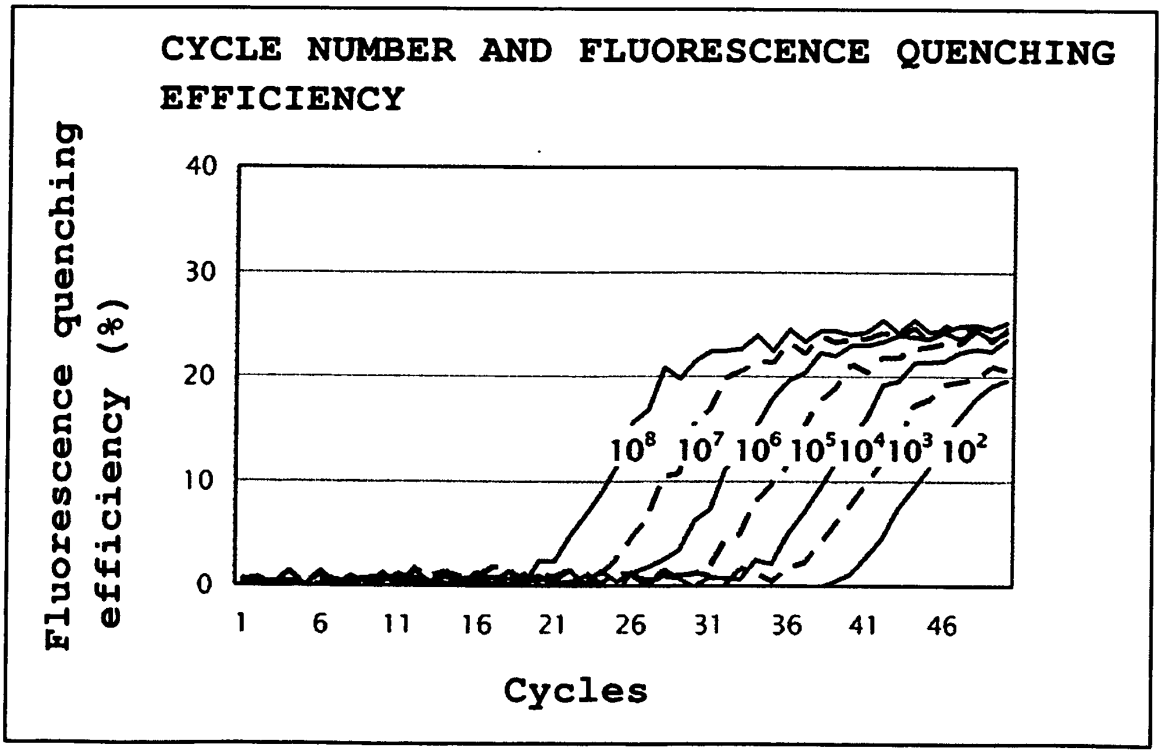
CYCLE NUMBER AND FLUORESCENCE QUENCHING EFFICIENCY
Fluorescence quenching efficiency (%)
40
30
20
10
0
$10^8$ $10^7$ $10^6$ $10^5$ $10^4$ $10^3$ $10^2$
1 6 11 16 21 26 31 36 41 46
Cycles

Fig. 11

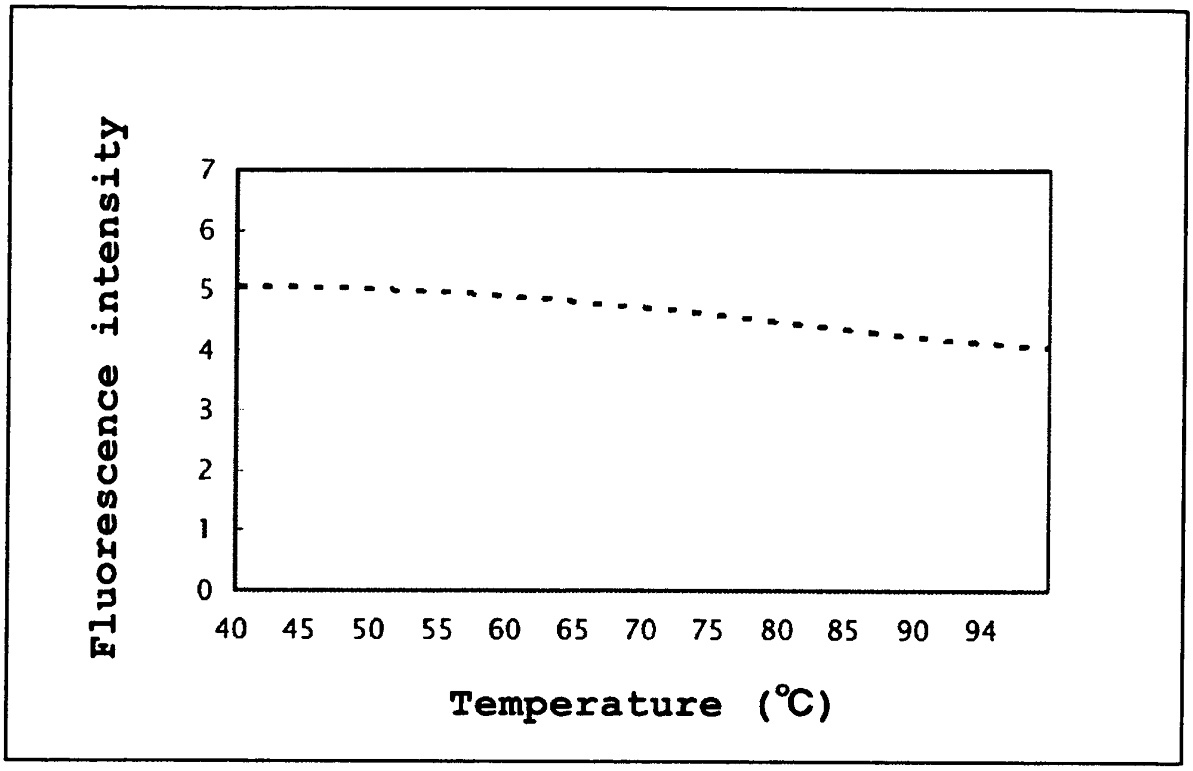
Fluorescence intensity
7
6
5
4
3
2
1
0
40 45 50 55 60 65 70 75 80 85 90 94
Temperature (℃)

Fig. 12

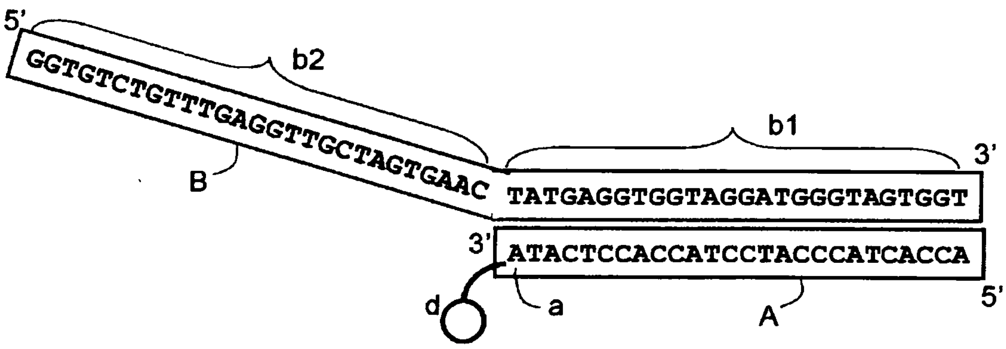
5'
b2
GGTGTCTGTTTGAGGTTGCTAGTGAAC
B
b1
3'
TATGAGGTGGTAGGATGGGTAGTGGT
3'
ATACTCCACCATCCTACCCATCACCA
5'
d
a
A

Fig. 13

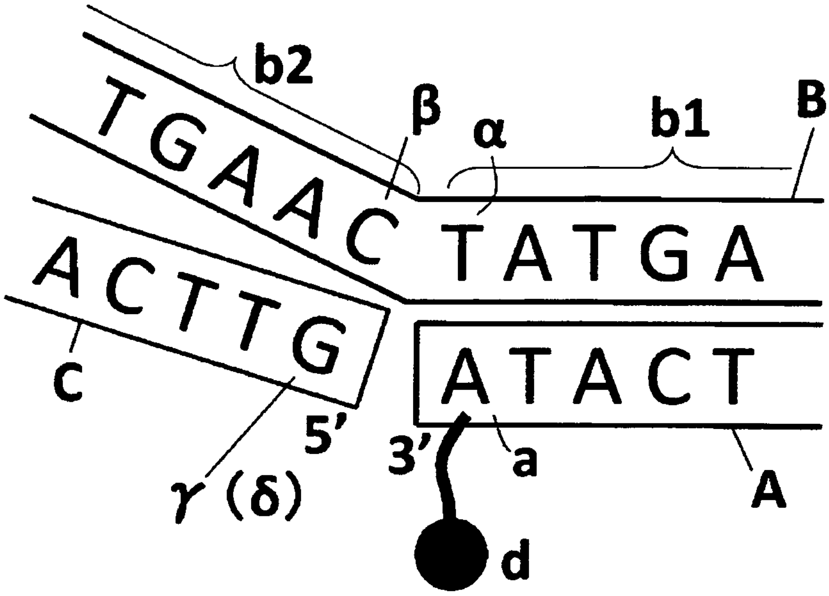


Fig. 14

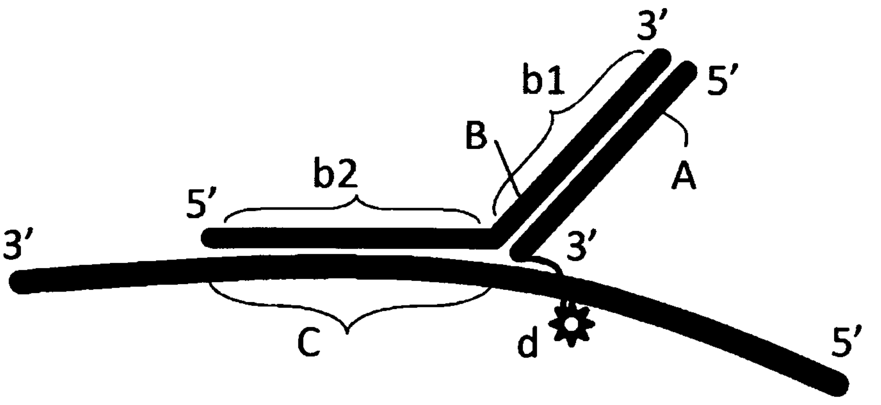

Fig. 15

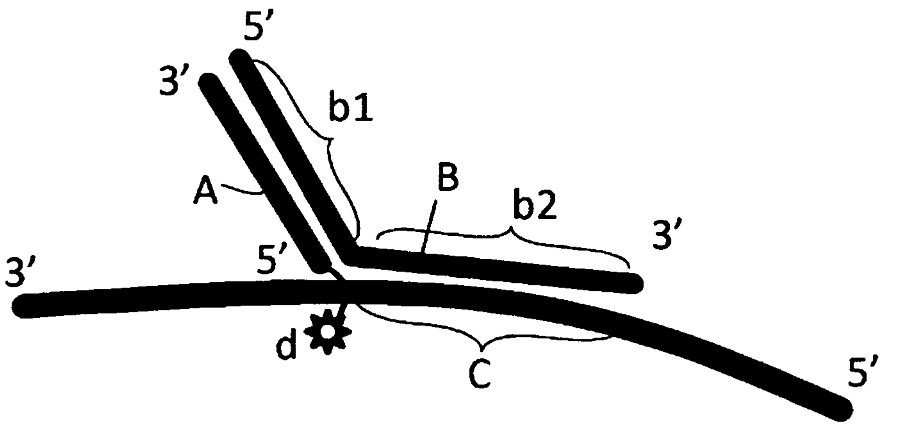
5'
3'
b1
A
B
b2
3'
3'
5'
d
C
5'

Fig. 16

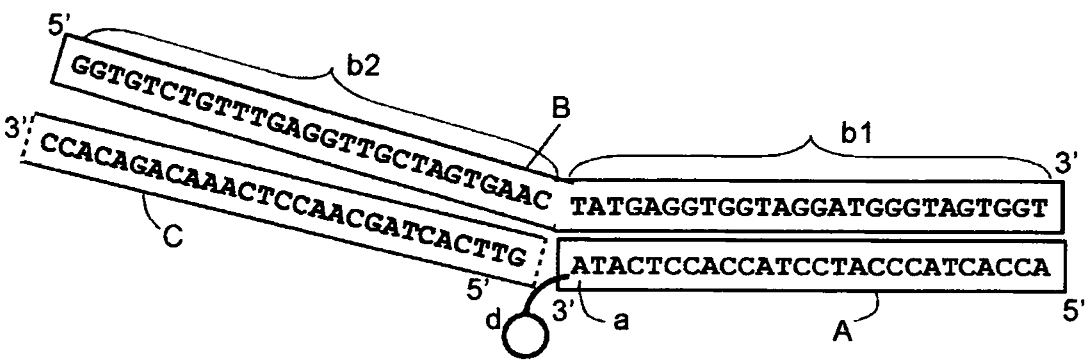
5'
b2
B
b1
3'
3'
GGTGTCTGTTTGAGGTTGCTAGTGAAC
TATGAGGTGGTAGGATGGGTAGTGGT
CCACAGACAAACTCCAACGATCACTTG
ATACTCCACCATCCTACCCATCACCA
C
5'
d
3'
a
A
5'

Fig. 17

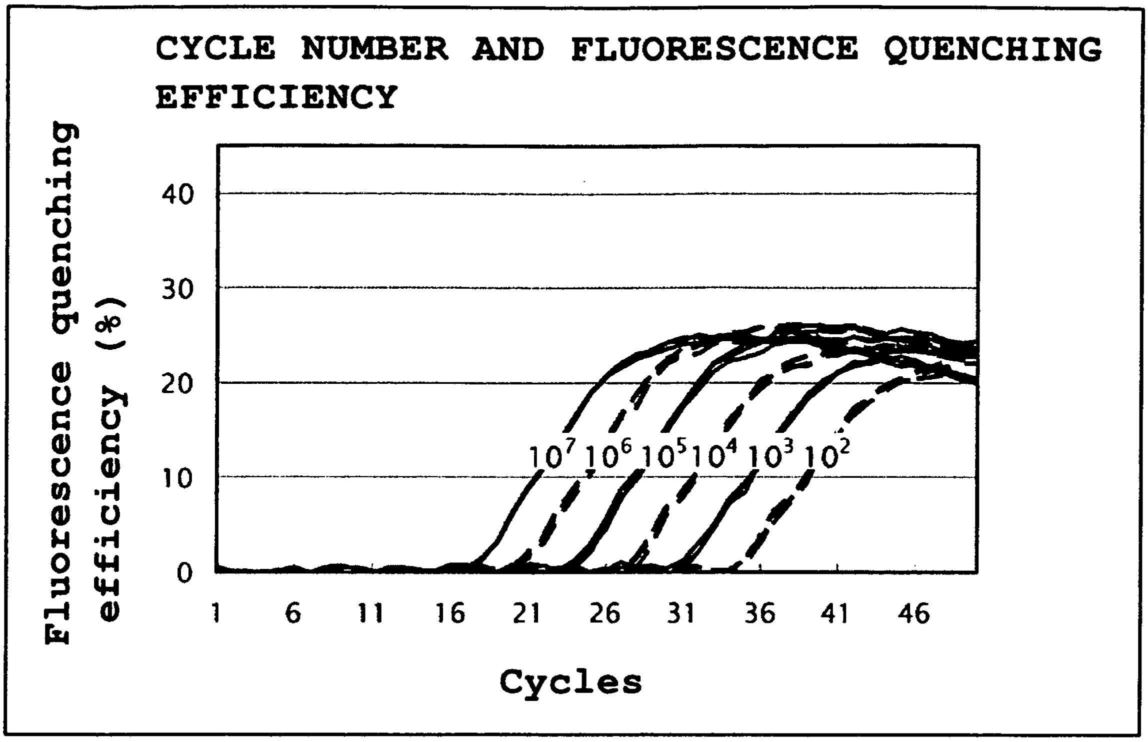
CYCLE NUMBER AND FLUORESCENCE QUENCHING EFFICIENCY
Fluorescence quenching efficiency (%)
40
30
20
10
0
$10^7$ $10^6$ $10^5$ $10^4$ $10^3$ $10^2$
1 6 11 16 21 26 31 36 41 46
Cycles

Fig. 18

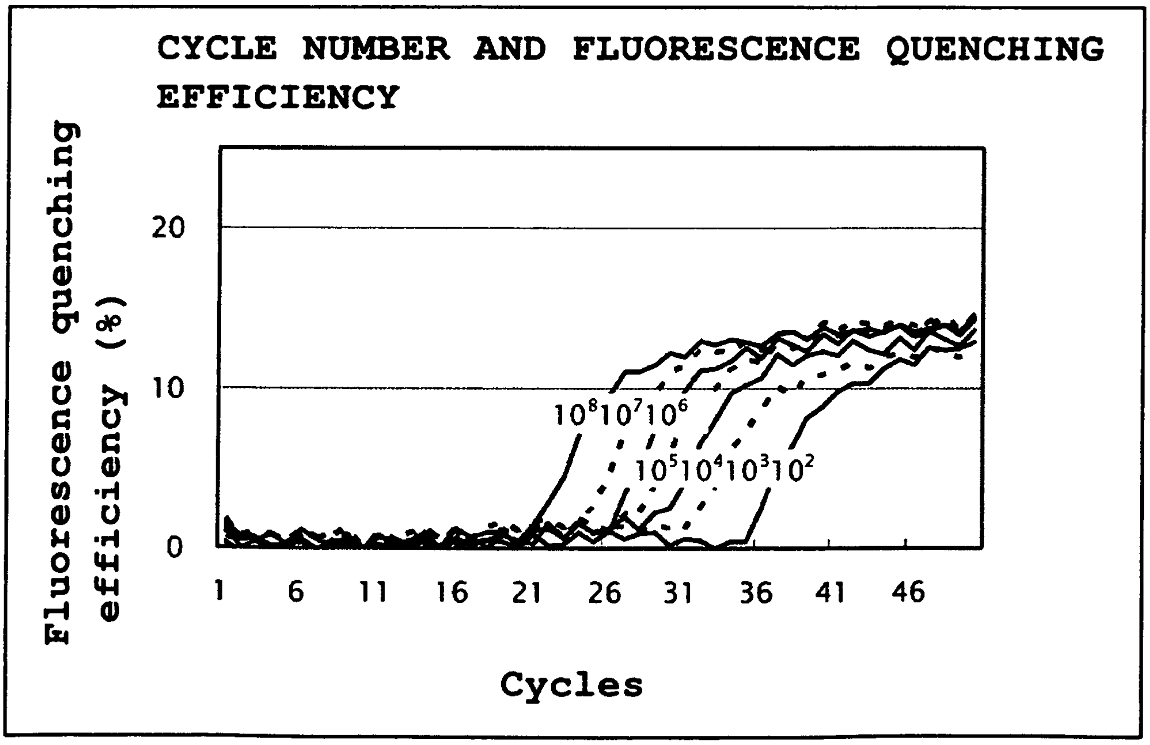
CYCLE NUMBER AND FLUORESCENCE QUENCHING EFFICIENCY
Fluorescence quenching efficiency (%)
20
10
0
$10^8$ $10^7$ $10^6$
$10^5$ $10^4$ $10^3$ $10^2$
1 6 11 16 21 26 31 36 41 46
Cycles

Fig. 19

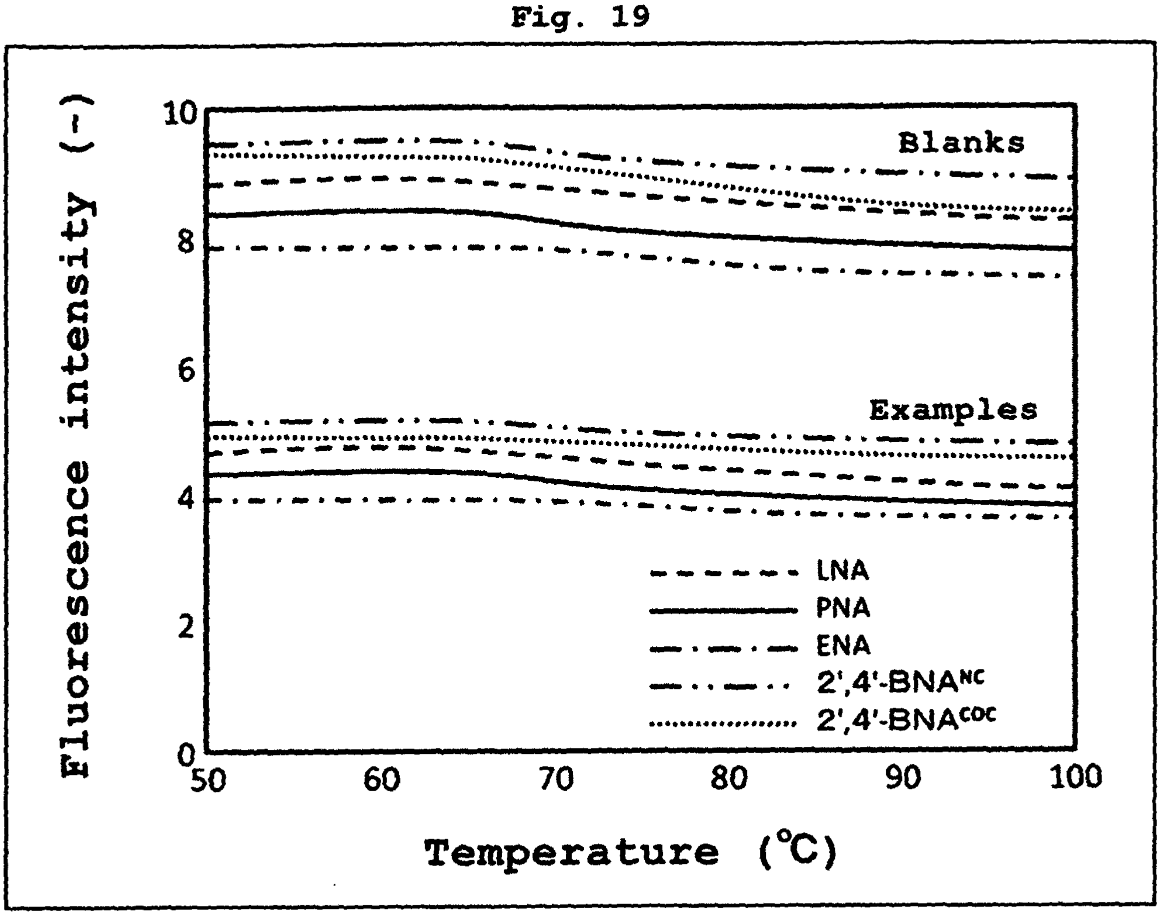
Fluorescence intensity (-)
10
8
6
4
2
0
50
60
70
80
90
100
Temperature (℃)
Blanks
Examples
LNA
PNA
ENA
2',4'-BNANC
2',4'-BNACOC

Fig. 20

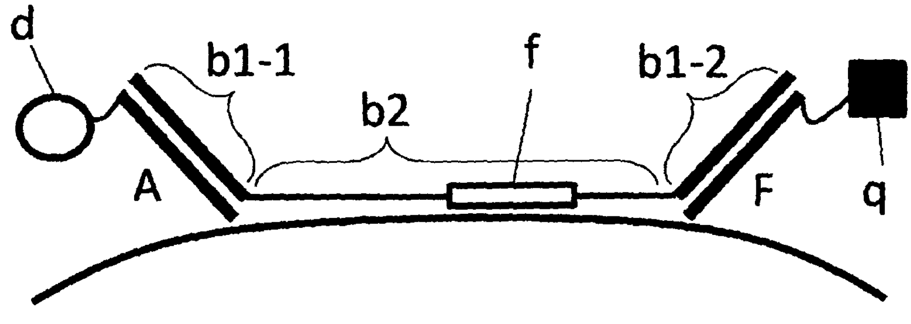
d
b1-1
f
b1-2
b2
A
F
q

Fig. 21

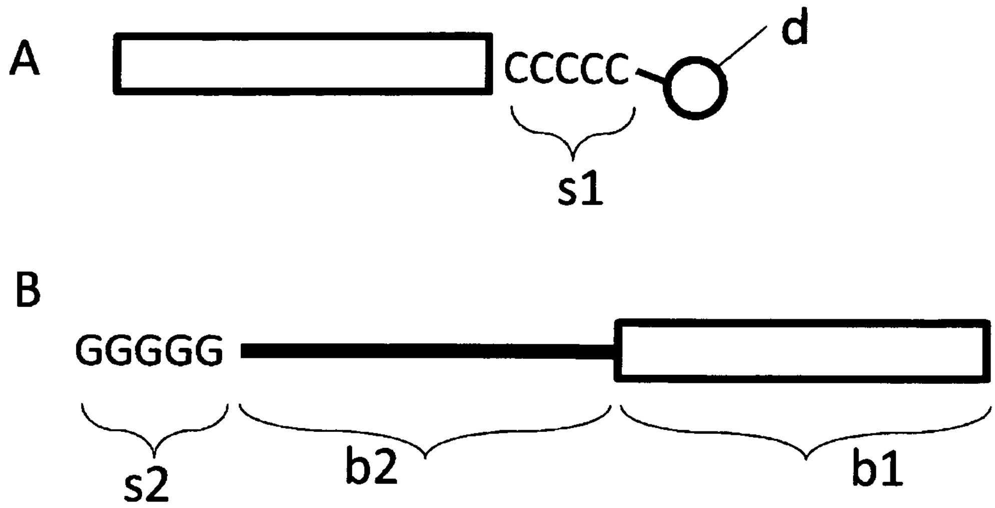


Fig. 22

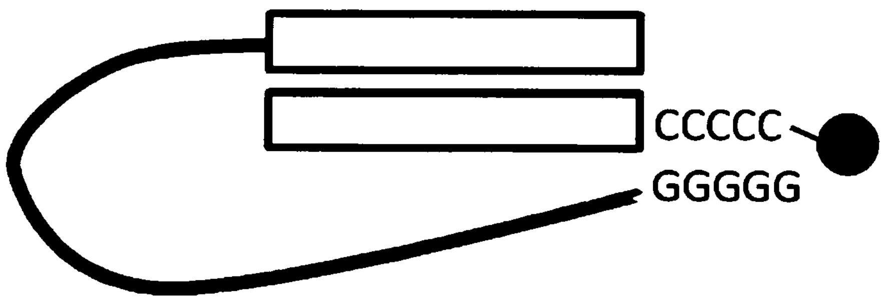

Fig. 23

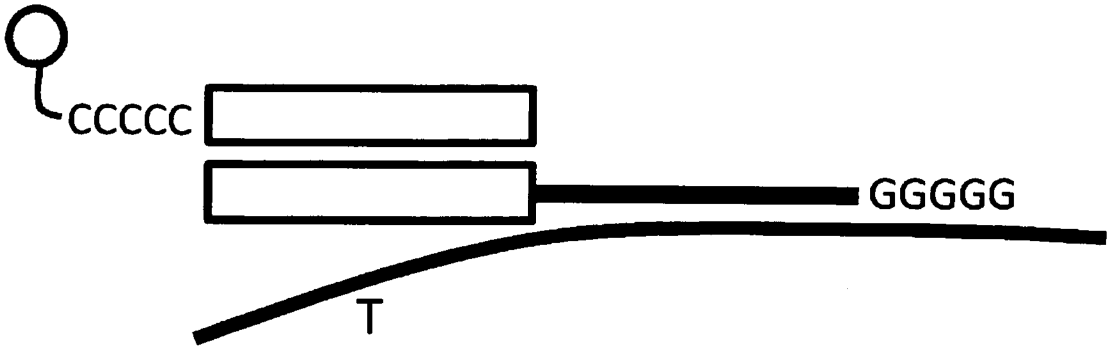


Fig. 24

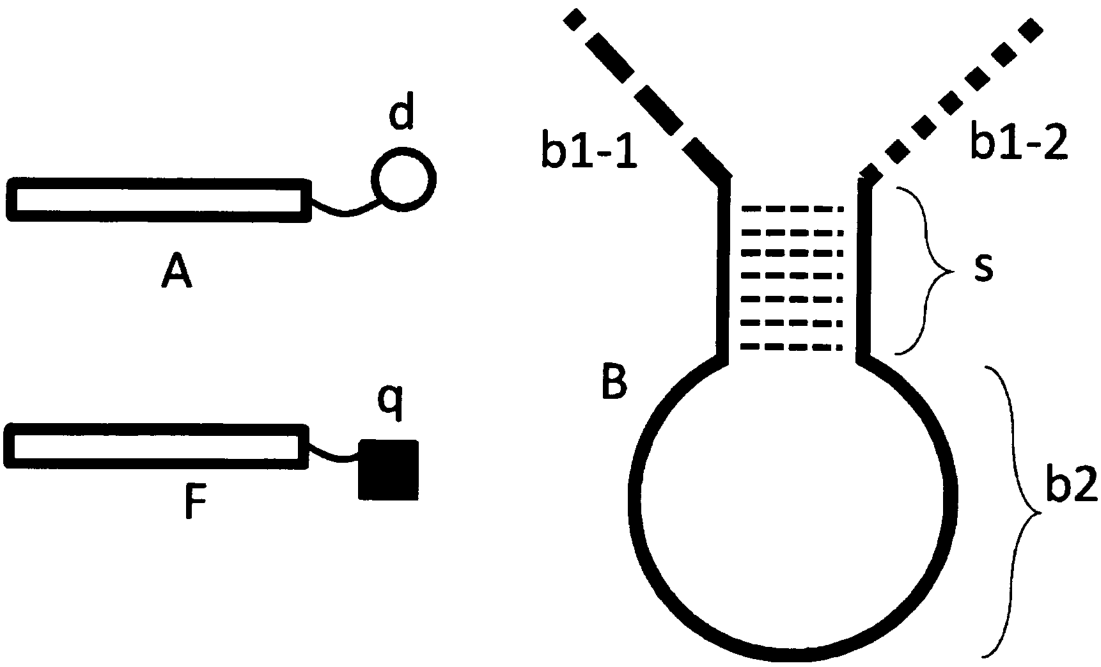

Fig. 25

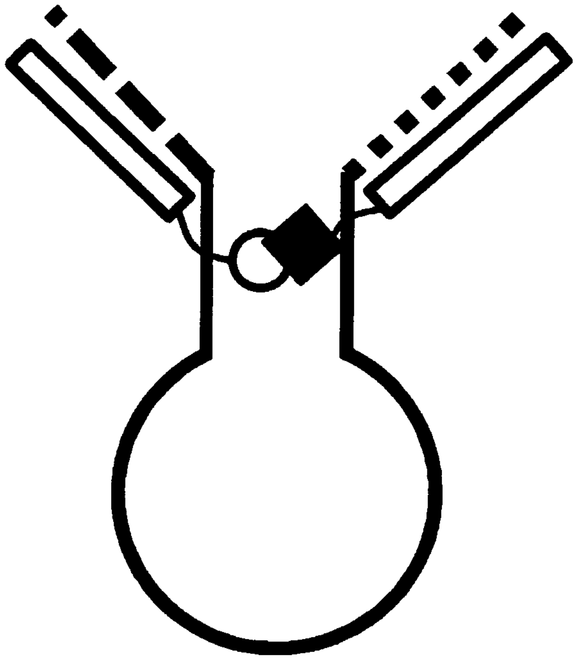

Fig. 26

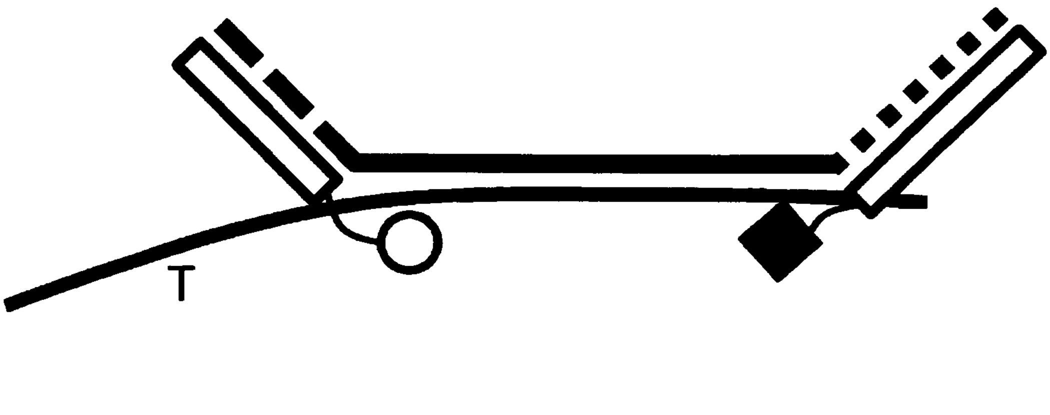

Fig. 27

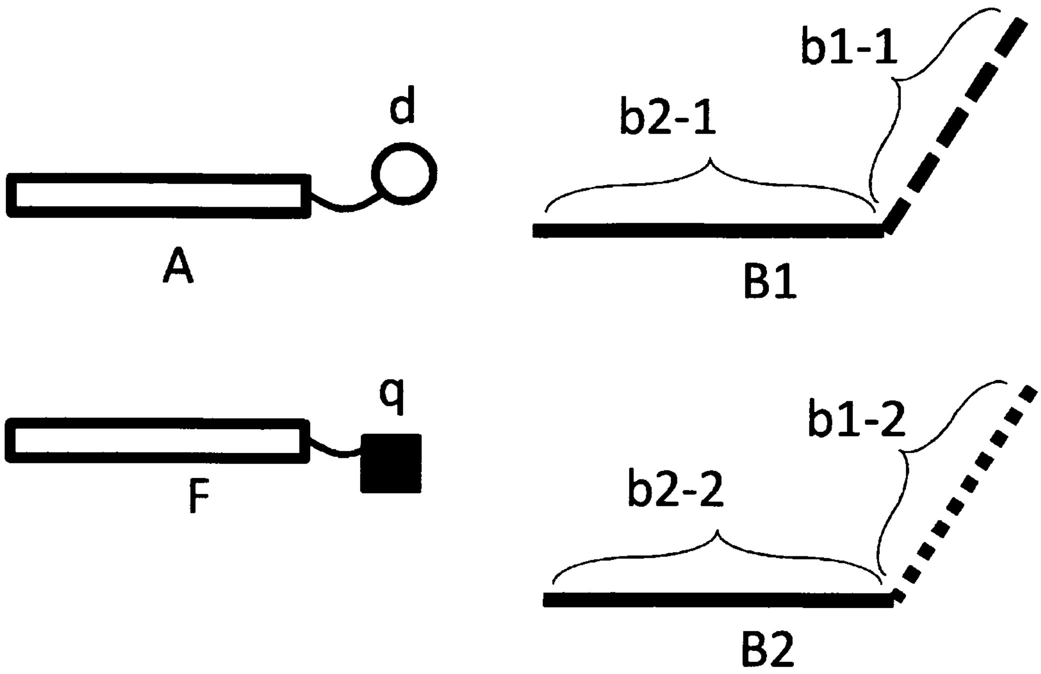


Fig. 28

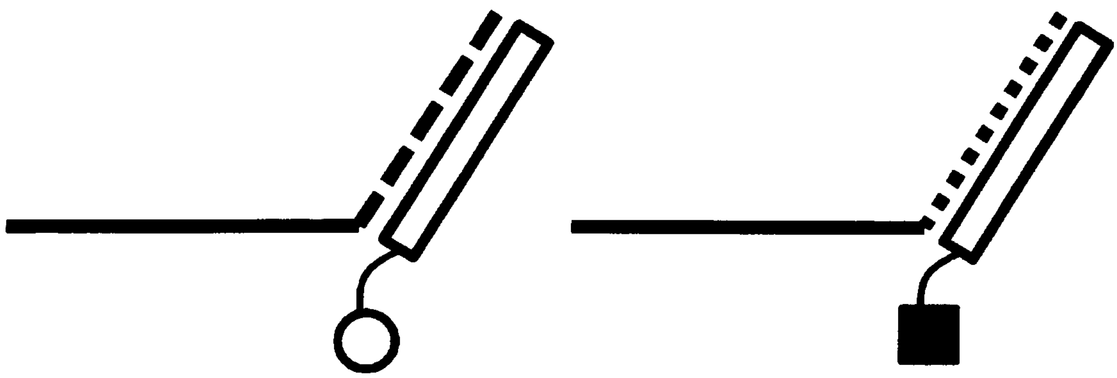

Fig. 29

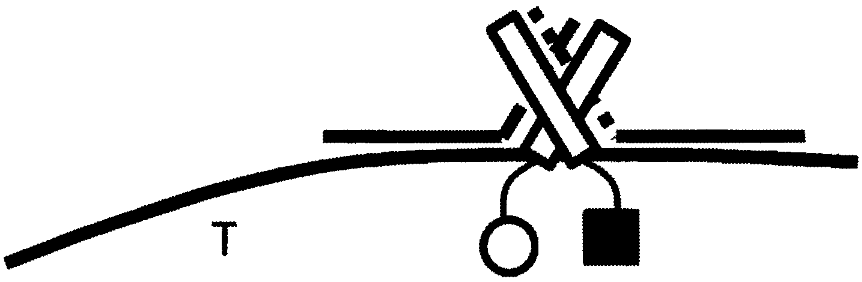


Fig. 30

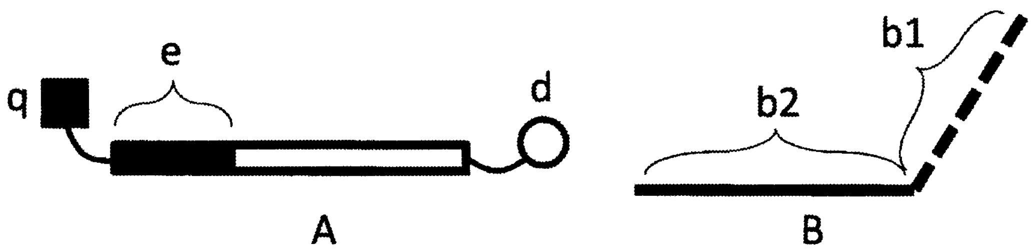


Fig. 31

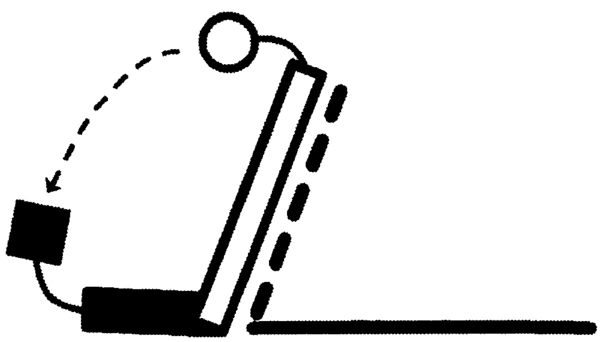

Fig. 32

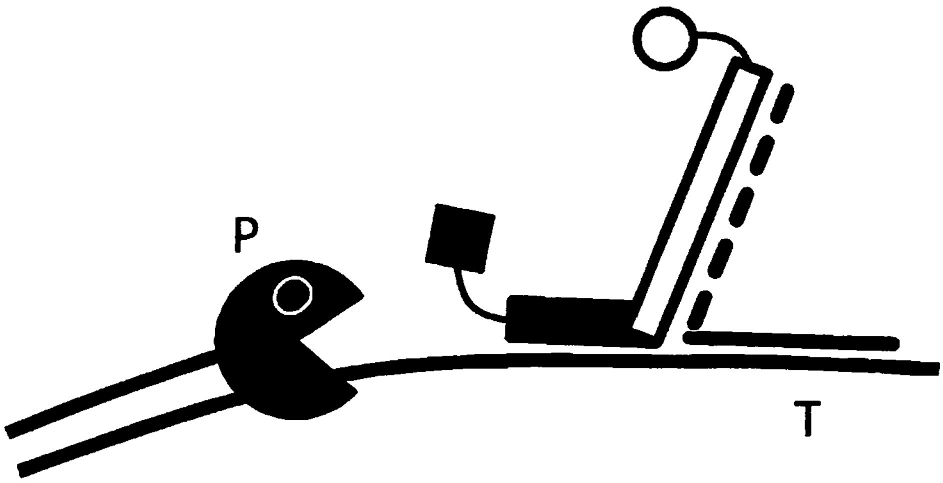
P
T

Fig. 33

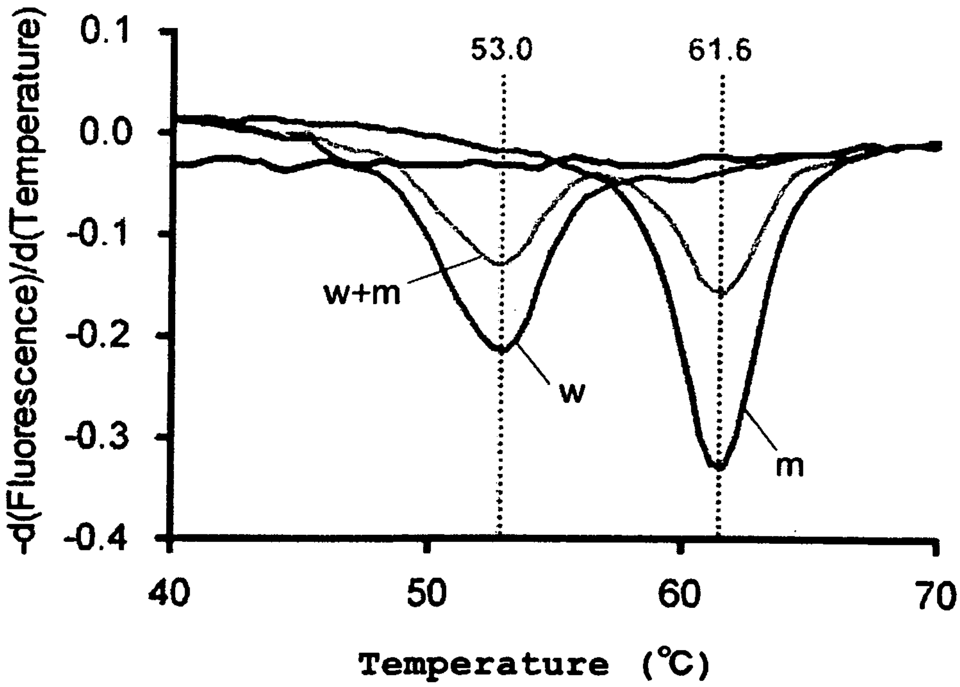
-d(Fluorescence)/d(Temperature)
0.1
0.0
-0.1
-0.2
-0.3
-0.4
53.0
61.6
w+m
w
m
40
50
60
70
Temperature (℃)

Fig. 34

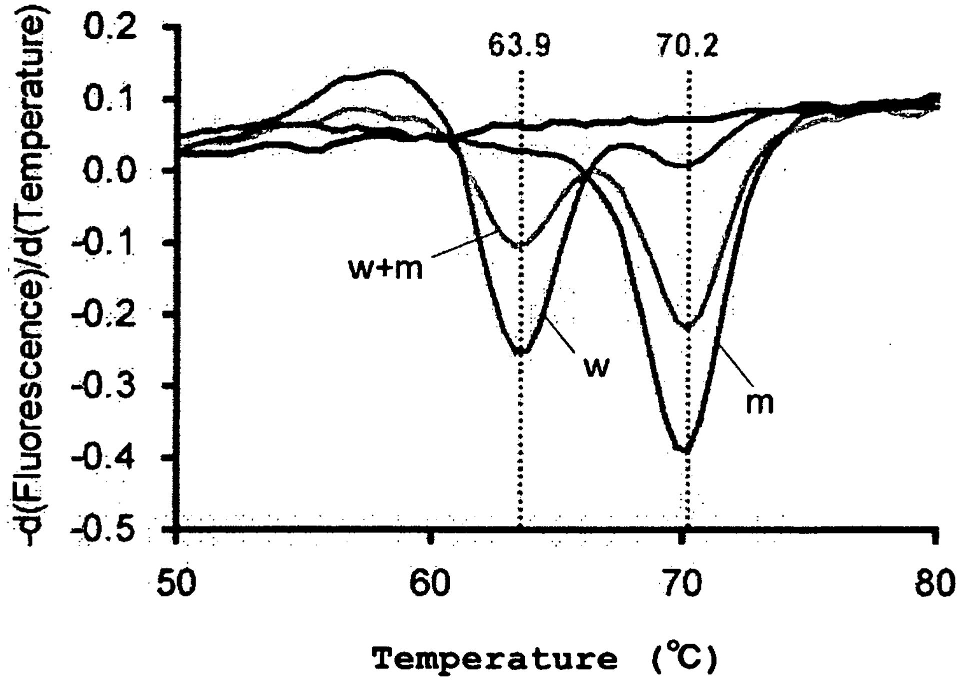
0.2
0.1
0.0
-0.1
-0.2
-0.3
-0.4
-0.5
-d(Fluorescence)/d(Temperature)
63.9
70.2
w+m
w
m
50
60
70
80
Temperature (℃)

Fig. 35

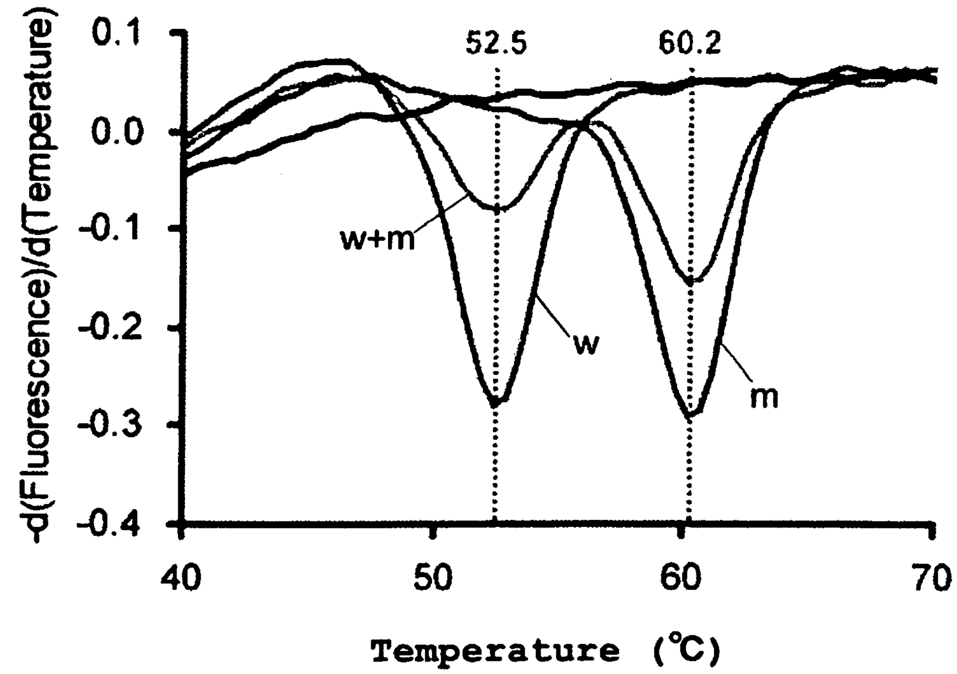
0.1
0.0
-0.1
-0.2
-0.3
-0.4
-d(Fluorescence)/d(Temperature)
52.5
60.2
w+m
w
m
40
50
60
70
Temperature (℃)

**INTERNATIONAL SEARCH REPORT**

International application No.
PCT/JP2009/063562

A. CLASSIFICATION OF SUBJECT MATTER
C12N15/09(2006.01)i, C12Q1/68(2006.01)i

According to International Patent Classification (IPC) or to both national classification and IPC

B. FIELDS SEARCHED

Minimum documentation searched (classification system followed by classification symbols)
C12N15/09, C12Q1/68

Documentation searched other than minimum documentation to the extent that such documents are included in the fields searched

Electronic data base consulted during the international search (name of data base and, where practicable, search terms used)
CAPLUS/BIOSIS/MEDLINE/WPIDS(STN), PubMed, JSTPlus(JDreamII)

C. DOCUMENTS CONSIDERED TO BE RELEVANT

| Category* | Citation of document, with indication, where appropriate, of the relevant passages | Relevant to claim No. |
|---|---|---|
| P,X | TANI H., et al., Universal quenching probe system: flexible, specific, and cost-effective real-time polymerase chain reaction method., Anal.Chem., 2009.07.15, Vol. 81, No. 14, p. 5678-5685 | 1-15 |
| P,X | JP 2008-182974 A (J-Bio 21 Corp.), 14 August, 2008 (14.08.08), Claims; Figs. 1 to 3 (Family: none) | 15 |
| X/Y | YANG C.J., et al., Synthesis and investigation of deoxyribonucleic acid/locked nucleic acid chimeric molecular beacons., Nucleic Acids Res., 2007, Vol. 35, No. 12, p. 4030-4041 | 10-13/14 |

☒ Further documents are listed in the continuation of Box C.    ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 10 September, 2009 (10.09.09) | 29 September, 2009 (29.09.09) |
| Name and mailing address of the ISA/ Japanese Patent Office | Authorized officer |
| Facsimile No. | Telephone No. |

Form PCT/ISA/210 (second sheet) (April 2007)

**INTERNATIONAL SEARCH REPORT**

International application No.
PCT/JP2009/063562

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2008/021466 A2 (GENETAG TECHNOLOGY, INC.), 21 February, 2008 (21.02.08), Page 5, lines 5 to 12; page 13, lines 25 to 31; Fig. 4 & EP 2059614 A2 | 1-9,15 |
| Y | JP 3437816 B1 (KANKYO ENGINEERING CO., LTD.), 18 August, 2003 (18.08.03), Claims; Par. No. [0010]; Fig. 6 & JP 2001-286300 A | 1-9,14,15 |
| A | KURATA S., et al., Fluorescent quenching-based quantitative detection of specific DNA/RNA using a BODIPY((R)) FL-labeled probe or primer., Nucleic Acids Res., 2001, Vol. 29, No. 6, E34 | 1-15 |
| A | CROCKETT A.O., et al., Fluorescein-labeled oligonucleotides for real-time PCR: using the inherent quenching of deoxyguanosine nucleotides., Anal.Biochem., 2001, Vol. 290, No. 1, p. 89-97 | 1-15 |
| A | YANG L., et al., A novel universal real-time PCR system using the attached universal duplex probes for quantitative analysis of nucleic acids., BMC Mol.Biol., 2008.06.04, Vol. 9, 54 | 1-15 |
| A | LI X., et al., Universal molecular beacon-based tracer system for real-time polymerase chain reaction., Anal.Chem., 2006, Vol. 78, No. 22, p. 7886-7890 | 1-15 |
| A | ZHANG Y., et al., A novel real-time quantitative PCR method using attached universal template probe., Nucleic Acids Res., 2003, Vol. 31, No. 20, e123 | 1-15 |

Form PCT/ISA/210 (continuation of second sheet) (April 2007)

**INTERNATIONAL SEARCH REPORT**

International application No.
PCT/JP2009/063562

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet.

1. ☐ As all required additional search fees were timely paid by the applicant, this international search report covers all searchable claims.
2. ☒ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

INTERNATIONAL SEARCH REPORT

International application No.
PCT/JP2009/063562

Continuation of Box No.III of continuation of first sheet(2)

The “special technical feature” of the inventions of claims 1-14 relates to a labeled nucleotide probe which has an increased melting temperature from a complementary strand thereof due to at least one artificial nucleotide contained therein. The “special technical feature” of the invention of claim 15 relates to a nucleic acid probe set comprising a fluorescent probe and a binding probe having a fluorescent probe-binding region and a target nucleic acid-binding region. There is not a technical relation which involves one or more of the same or corresponding special technical features between these groups of inventions. Therefore, it cannot be considered that these inventions are so linked as to form a single general inventive concept.

Further, although the “special technical feature” of the inventions of claims 1-14 relates to a labeled nucleotide probe which has an increased melting temperature from a complementary strand thereof due to at least one artificial nucleotide contained therein, a labeled nucleotide probe which has an increased melting temperature from a complementary strand thereof due to an artificial nucleotide contained therein is publicly known, as disclosed in Nucleic Acids Res., 2007, Vol.35, No.12, p.4030-4041. Therefore, it is obvious that the above-stated “special technical feature” does not make any contribution over the prior art of the invention. Consequently, it cannot be considered that the inventions of claims 1-9 and the inventions of claims 10-14 are so linked as to form a single general inventive concept unless there is any other same or corresponding special technical feature between these groups of inventions.

Form PCT/ISA/210 (extra sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 3437816 B
- JP 3963422 B

### Non-patent literature cited in the description

- *Nature Biotechnology,* 1996, vol. 14, 303-308
- *Applied and Environmental Microbiology,* 1997, vol. 63, 1143-1147
- *Nucleic Acids Research,* 1996, vol. 24, 4532-4535
- *ANALYTICAL BIOCHEMISTRY,* 1998, vol. 225, 32-38
- **Tani et al.** Alternately Binding Probe Competitive PCR. *Analytical Chemistry*